(19) Europäisches Patentamt
European Patent Office
Office européen des brevets

(11) EP 3 801 553 B1

(12) EUROPEAN PATENT SPECIFICATION

(45) Date of publication and mention of the grant of the patent:
**25.03.2026 Bulletin 2026/13**

(21) Application number: **19808360.2**

(22) Date of filing: **24.05.2019**

(51) International Patent Classification (IPC):
***C07J 31/00*** (2006.01) ***C07J 41/00*** (2006.01)
***C07J 43/00*** (2006.01) ***C07J 51/00*** (2006.01)
***A61K 31/565*** (2006.01) ***A61P 35/00*** (2006.01)

(52) Cooperative Patent Classification (CPC):
**C07J 51/00; C07J 31/006; C07J 41/0072; C07J 41/0088; C07J 43/003**

(86) International application number:
**PCT/IB2019/054315**

(87) International publication number:
**WO 2019/224790 (28.11.2019 Gazette 2019/48)**

# (54) PRODRUGS OF FULVESTRANT

PRODRUGS VON FULVESTRANT

PROMÉDICAMENTS DE FULVESTRANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.05.2018 IN 201821019542**
**18.12.2018 IN 201821047939**

(43) Date of publication of application:
**14.04.2021 Bulletin 2021/15**

(73) Proprietor: **Kashiv Biosciences, LLC**
**Piscataway, NJ 08854 (US)**

(72) Inventors:
- **PUROHIT, Parva Yogeshchandra**
**Gujarat Ahmedabad 38248 (IN)**
- **BRAHMKSHATRIYA, Pathik Subhashchandra**
**Gujarat Ahmedabad 380015 (IN)**
- **GOSWAMI, Vishalgiri Gunvantgiri**
**Gujarat Ahmedabad 382481 (IN)**

(74) Representative: **Haseltine Lake Kempner LLP**
**Bürkleinstrasse 10**
**80538 München (DE)**

(56) References cited:
**CN-A- 103 421 069** **US-A1- 2011 183 949**
**US-A1- 2015 105 357** **US-A1- 2016 060 288**

- **HALASA A F ET AL: "POLYLITHIATION OF FERROCENE*", JOURNAL OF ORGANOMETALLIC CHEMISTRY, vol. 24, no. 3, 1 June 1970 (1970-06-01), AMSTERDAM, NL, pages 769 - 773, XP093314413, ISSN: 0022-328X**
- **DATABASE PUBCHEM [online] 12 June 2009 (2009-06-12), Database accession no. 76487450**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 801 553 B1

## Description

FIELD OF INVENTION

The present invention relates to fulvestrant prodrugs of formula II and process for the preparation thereof. The present disclosure also relates to pharmaceutical composition of fulvestrant prodrugs and method of treatment using the same.

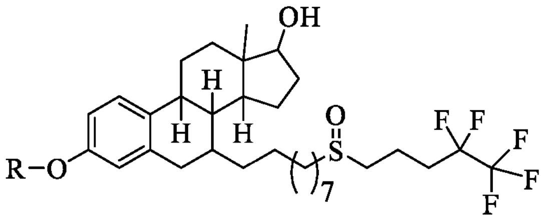

(II)

BACKGROUND OF THE INVENTION

Fulvestrant is an estrogen receptor antagonist marketed as Faslodex™ for the treatment of hormone receptor positive metastatic breast cancer in postmenopausal women with disease progression following anti-estrogen therapy. Fulvestrant has clinical therapeutic effect in patients failed in treatment with tamoxifen. Therefore, among many drugs for treating breast cancer, fulvestrant is the only anti-estrogen agent that may be widely used in clinical treatment after the failure of tamoxifen, which has initiated a new way of treating hormone-sensitive breast cancer. Due to the poor solubility and oral bioavailability of fulvestrant, the drug is currently administered via intramuscular injection of an oil-based fulvestrant formulation. The Faslodex™ product is approved for administration by intramuscular injection on days 1, 15, 29 and once monthly thereafter. This injection contains upto 10% of benzyl alcohol which might act as anaesthetic level while castor oil is used as release rate modifier which can be viscous that can be painful at the time of injection and the solution might be appear yellowish colour too. According to FDA drug approval summaries, injection site reaction and hot flashes were observed.

Fulvestrant is a highly lipophilic molecule which is practically insoluble in water. This restricts their bioavailability. A drug with poor solubility will often exhibit poor bioavailability and require administration of high dosages to attain therapeutically effective blood levels of the drug.

US 6,774,122 B2 describes that intra-muscular injections of fulvestrant in the form of an aqueous suspension were not suitable for use. Those suspensions resulted in extensive local tissue irritation at the injection site as well as a poor release profile due to the presence of fulvestrant in the form of solid particles.

The use of prodrugs allows the artisan to modify one or more properties of a biologically active compound. Prodrugs include chemical derivatives of a biologically active parent compound which, upon administration, will eventually liberate the active parent compound in vivo. The rate of release of the active drug is influenced by several factors including the rate of hydrolysis of the linker which joins the parent biologically active compound to the prodrug carrier.

WO2016/004166 A1 discloses boron based fulvestrant prodrugs for the treatment of breast cancer. This PCT application also discloses the need of improved bioavailability of fulvestrant to make it more effective therapeutic regimen for tamoxifen-resistant breast cancer. Despite clinical efficacy of fulvestrant, the utility of fulvestrant has been limited by the amount of drug that can be administered in a single injection and by reduced bioavailability.

J. Med. Chem., 2016, 59 (17), pp. 8134-8140 discloses that fulvestrant undergoes rapid and extensive O-glucoronidation and O-sulfation to form polar phase II metabolites that are inactivate and water soluble.

CN103421069A discloses fulvestrant phosphate derivatives which has better thermal stability than fulvestrant. It also discloses the amount of fulvestrant release from these derivatives using cytochrome P 450 enzyme CYP3A4 and alkaline phosphatase.

Accordingly, an object of the present invention is to provide a compound or a salt thereof that has good water solubility and is metabolized rapidly to produce fulvestrant in the body.

The present inventors have surprisingly found that an introduction of a substituent at least at the phenolic hydroxyl group of fulvestrant markedly alters the physical properties of the compound and improves water solubility.

BRIEF DESCRIPTION OF THE DRAWINGS

Figure 1. Time-concentration profile in mice (compound (I-t) and fulvestrant blood levels)

SUMMARY OF THE INVENTION

In one aspect, the present invention relates to prodrugs of fulvestrant of formula I-A and enantiomers, diastereomers, racemates, pharmaceutically acceptable salts and solvates thereof,

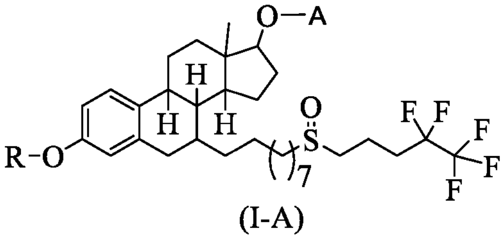

(I-A)

wherein

A is selected from hydrogen,

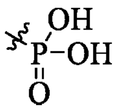

,

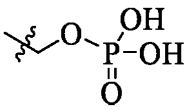

,

and

R is selected from group comprising of:

a)

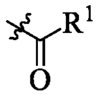

wherein $R^1$ is selected from group comprising of substituted aryl; optionally substituted alkyl, alkenyl, alkynyl, cycloalkyl, heteroaryl and $CR^2R^3$. $R^2$ and $R^3$ are taken together along with the carbon to which they are attached to form a three to seven membered saturated, partially unsaturated or unsaturated heterocyclic ring in which up to 4 carbon atoms are replaced by heteroatoms chosen from the group consisting of O, S or N and may optionally be substituted at a substitutable position with one or more $R^4$ radicals, wherein the $R^4$ radicals are independently selected at each occurrence from the group consisting of alkyl, cycloalkyl, heterocycloalkyl, aryl, heteroaryl, alkylcarbonyl, formyl, halo, alkylphosphate, phosphate, cyano, nitro, alkoxy, alkoxycarbonyl, alkenyl, alkynyl, alkylthio and arylcarbonyl;

b)

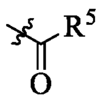

wherein $R^5$ is

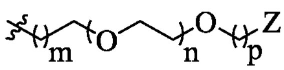

;

wherein m and p are independently selected from 0 to 5, n refers to degree of polymerization and is selected from 1 to 250 and Z is optionally substituted alkyl or cycloalkyl;

c)

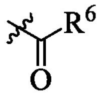

wherein $R^6$ is selected from $NH_2$, $NHR^7$ and $NR^8R^9$. $R^7$ is selected from optionally substituted alkyl, alkenyl, alkynyl, cycloalkyl, heterocycloalkyl, aryl and heteroaryl. $R^8$ and $R^9$ are taken together along with the nitrogen to which they are attached to form a three to seven membered saturated, partially unsaturated or unsaturated heterocyclic ring and may optionally be substituted at a substitutable position with one or more $R^{10}$ radicals, wherein the $R^{10}$ radicals are independently selected at each occurrence from the group consisting of alkyl, alkylcarbonyl, formyl, halo, haloalkyl, alkylphosphate, phosphate, oxo, cyano, nitro, amino, alkoxy, alkoxycarbonyl, carboxyalkyl, hydroxyalkyl, alkenyl, alkynyl, alkylthio, cycloalkyl, aryl, aralkyl, heterocycloalkyl, alkylthioalkyl, arylcarbonyl, aralkylcarbonyl and alkoxyalkyl;

d)

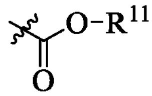

wherein $R^{11}$ is selected from group comprising of optionally substituted alkyl, alkenyl, alkynyl, cycloalkyl, heterocycloalkyl, aryl and heteroaryl;

e)

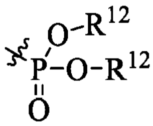

wherein each $R^{12}$ is selected independently from group comprising of hydrogen; optionally substituted alkyl, aryl, acyl and heteroaryl;
with a proviso that when A is hydrogen and one of $R^{12}$ substitution is selected from alkyl and aryl, then another $R^{12}$ substitution is hydrogen;

f)

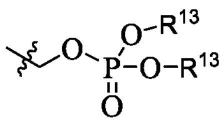

wherein each $R^{13}$ is selected independently from group comprising of hydrogen; optionally substituted alkyl, cycloalkyl, heterocycloalkyl, aryl and heteroaryl;

g)

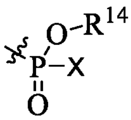

wherein $R^{14}$ is selected from group comprising of hydrogen; optionally substituted alkyl, aryl, acyl, heteroaryl; and X is optionally substituted heterocycloalkyl; and

h) hydrogen with the proviso that when R is hydrogen A is not hydrogen.

In another aspect, the present invention relates to prodrugs of fulvestrant of formula II and enantiomers, diastereomers, racemates, pharmaceutically acceptable salts and solvates thereof,

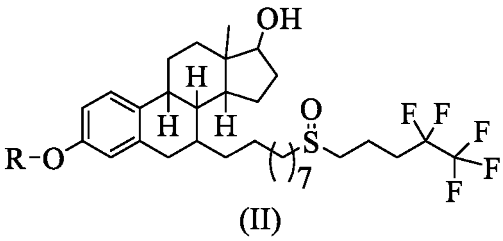

(II)

wherein R is selected from group comprising of:

a)

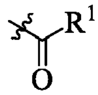

wherein $R^1$ is selected from group comprising of substituted aryl; optionally substituted alkyl, alkenyl, alkynyl, cycloalkyl, heteroaryl and $CR^2R^3$. $R^2$ and $R^3$ are taken together along with the carbon to which they are attached to form a three to seven membered saturated, partially unsaturated or unsaturated heterocyclic ring in which up to 4 carbon atoms are replaced by heteroatoms chosen from the group consisting of O, S or N and may optionally be substituted at a substitutable position with one or more $R^4$ radicals, wherein the $R^4$ radicals are independently selected at each occurrence from the group consisting of alkyl, cycloalkyl, heterocycloalkyl, aryl, heteroaryl, alkylcarbonyl, formyl, halo, alkylphosphate, phosphate, cyano, nitro, alkoxy, alkoxycarbonyl, alkenyl, alkynyl, alkylthio and arylcarbonyl;

b)

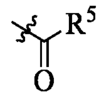

wherein $R^5$ is

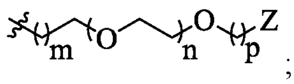

wherein m and p are independently selected from 0 to 5, n refers to degree of polymerization and is selected from 1 to 250 and Z is optionally substituted alkyl or cycloalkyl;

c)

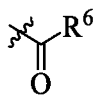

wherein $R^6$ is selected from $NH_2$, $NHR^7$ and $NR^8R^9$. $R^7$ is selected from optionally substituted alkyl, alkenyl, alkynyl, cycloalkyl, heterocycloalkyl, aryl and heteroaryl. $R^8$ and $R^9$ are taken together along with the nitrogen to which they are attached to form a three to seven membered saturated, partially unsaturated or unsaturated heterocyclic ring and may optionally be substituted at a substitutable position with one or more $R^{10}$ radicals, wherein the $R^{10}$ radicals are independently selected at each occurrence from the group consisting of alkyl, alkylcarbonyl, formyl, halo, haloalkyl, alkylphosphate, phosphate, oxo, cyano, nitro, amino, alkoxy, alkoxycarbonyl, carboxyalkyl, hydroxyalkyl, alkenyl, alkynyl, alkylthio, cycloalkyl, aryl, aralkyl, heterocycloalkyl, alkylthioalkyl, arylcarbonyl, aralkylcarbonyl and alkoxyalkyl;

d)

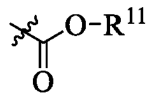

wherein $R^{11}$ is selected from group comprising of optionally substituted alkyl, alkenyl, alkynyl, cycloalkyl, heterocycloalkyl, aryl and heteroaryl.

e)

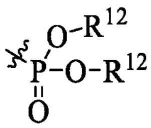

wherein each $R^{12}$ is selected independently from group comprising of hydrogen; optionally substituted alkyl, aryl, acyl and heteroaryl;
with a proviso that when one of $R^{12}$ is selected from alkyl and aryl, then another $R^{12}$ substitution is hydrogen;

f)

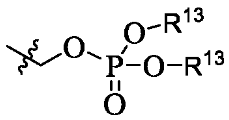

wherein each $R^{13}$ is selected independently from group comprising of hydrogen; optionally substituted alkyl, cycloalkyl, heterocycloalkyl, aryl and heteroaryl; and

g)

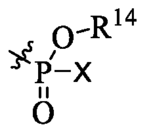

wherein $R^{14}$ is selected from group comprising of hydrogen; optionally substituted alkyl, aryl, acyl, heteroaryl; and X is optionally substituted heterocycloalkyl.

In one embodiment, the present invention relates to prodrugs of fulvestrant of formula III and enantiomers, diastereomers, racemates, pharmaceutically acceptable salts and solvates thereof,

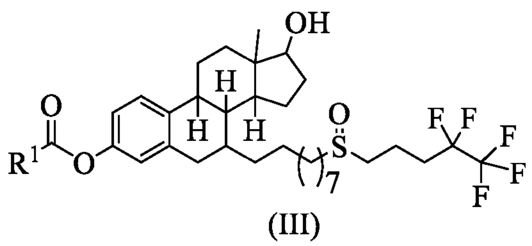

(III)

wherein $R^1$ is selected from group comprising of substituted aryl; optionally substituted alkyl, alkenyl, alkynyl, cycloalkyl, heteroaryl and $CR^2R^3$. $R^2$ and $R^3$ are taken together along with the carbon to which they are attached to form a three to seven membered saturated, partially unsaturated or unsaturated heterocyclic ring in which up to 4 carbon atoms are replaced by heteroatoms chosen from the group consisting of O, S or N and may optionally be substituted at a substitutable position with one or more $R^4$ radicals, wherein the $R^4$ radicals are independently selected at each occurrence from the group consisting of alkyl, cycloalkyl, heterocycloalkyl, aryl, heteroaryl, alkylcarbonyl, formyl, halo, alkylphosphate, phosphate, cyano, nitro, alkoxy, alkoxycarbonyl, alkenyl, alkynyl, alkylthio and arylcarbonyl.
In another embodiment, the present invention relates to prodrugs of fulvestrant of formula IV and enantiomers,

diastereomers, racemates, pharmaceutically acceptable salts and solvates thereof,

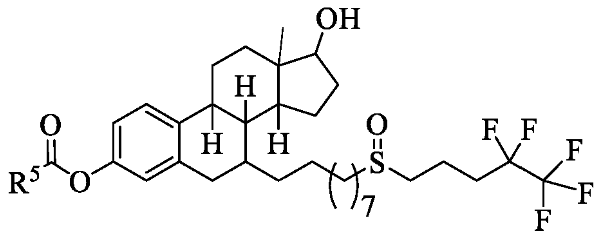

(IV)

wherein $R^5$ is

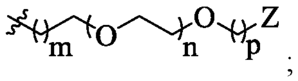

wherein m and p are independently selected from 0 to 5, n refers to degree of polymerization and is selected from 1 to 250 and Z is optionally substituted alkyl or cycloalkyl.

In yet another embodiment, the present invention relates to prodrugs of fulvestrant of formula V and enantiomers, diastereomers, racemates, pharmaceutically acceptable salts and solvates thereof,

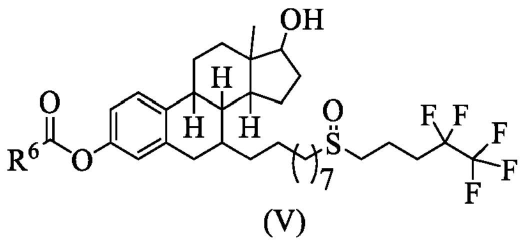

(V)

wherein $R^6$ is selected from $NH_2$, $NHR^7$ and $NR^8R^9$. $R^7$ is selected from optionally substituted alkyl, alkenyl, alkynyl, cycloalkyl, heterocycloalkyl, aryl and heteroaryl. $R^8$ and $R^9$ are taken together along with the nitrogen to which they are attached to form a three to seven membered saturated, partially unsaturated or unsaturated heterocyclic ring and may optionally be substituted at a substitutable position with one or more $R^{10}$ radicals, wherein the $R^{10}$ radicals are independently selected at each occurrence from the group consisting of alkyl, alkylcarbonyl, formyl, halo, haloalkyl, alkylphosphate, phosphate, oxo, cyano, nitro, amino, alkoxy, alkoxycarbonyl, carboxyalkyl, hydroxyalkyl, alkenyl, alkynyl, alkylthio, cycloalkyl, aryl, aralkyl, heterocycloalkyl, alkylthioalkyl, arylcarbonyl, aralkylcarbonyl and alkoxyalkyl.

In yet another embodiment, the present invention relates to prodrugs of fulvestrant of formula VI and enantiomers, diastereomers, racemates, pharmaceutically acceptable salts and solvates thereof,

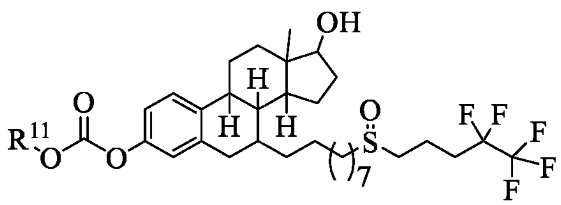

(VI)

wherein $R^{11}$ is selected from group comprising of optionally substituted alkyl, alkenyl, alkynyl, cycloalkyl, heterocycloalkyl, aryl and heteroaryl.

In yet another embodiment, the present invention relates to prodrugs of fulvestrant of formula VII and enantiomers, diastereomers, racemates, pharmaceutically acceptable salts and solvates thereof,

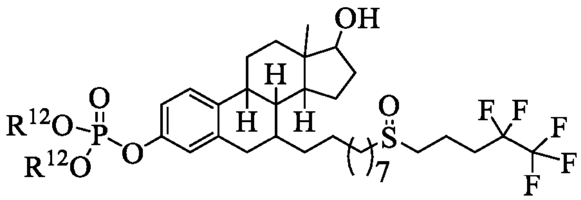

(VII)

wherein each $R^{12}$ is selected independently from group comprising of hydrogen; optionally substituted alkyl, aryl, acyl and heteroaryl;

with a proviso that when one of $R^{12}$ is selected from alkyl and aryl, then another $R^{12}$ substitution is hydrogen.

In yet another embodiment, the present invention relates to prodrugs of fulvestrant of formula VIII and enantiomers, diastereomers, racemates, pharmaceutically acceptable salts and solvates thereof,

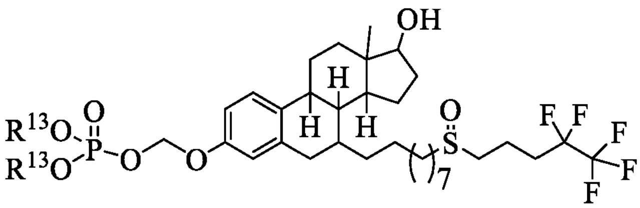

(VIII)

wherein $R^{13}$ is selected from group comprising of hydrogen; optionally substituted alkyl, cycloalkyl, heterocycloalkyl, aryl and heteroaryl.

In yet another embodiment, the present invention relates to prodrugs of fulvestrant of formula XV and enantiomers, diastereomers, racemates, pharmaceutically acceptable salts and solvates thereof,

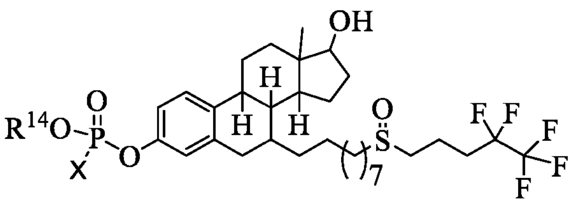

(XV)

wherein $R^{14}$ is selected from group comprising of hydrogen; optionally substituted alkyl, aryl, acyl, heteroaryl; and X is optionally substituted heterocycloalkyl.

In another aspect, the present invention relates to process for the preparation of prodrugs of fulvestrant of formula III comprising reacting fulvestrant of formula I with compound of formula IX,

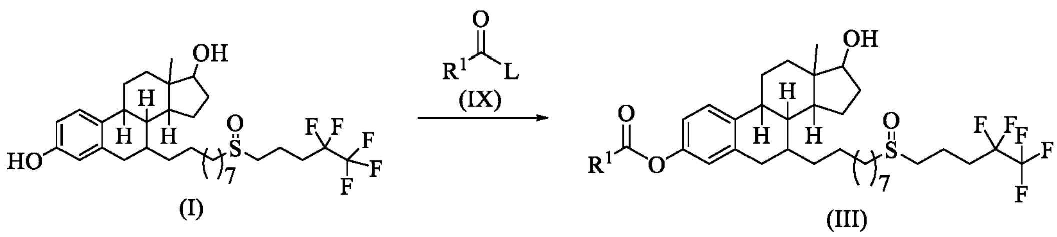

wherein $R^1$ has same meaning as defined above and L is a leaving group.

In yet another aspect, the present invention relates to process for the preparation of prodrugs of fulvestrant of formula IV comprising reacting fulvestrant of formula I with compound of formula X,

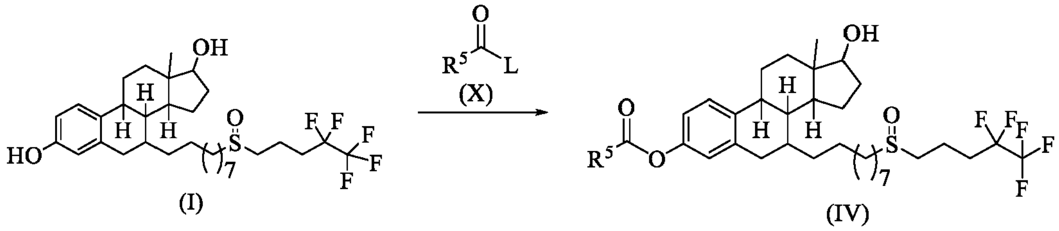

(I) (IV)

wherein $R^5$ has same meaning as defined above and L is a leaving group.

In yet another aspect, the present invention relates to process for the preparation of prodrugs of fulvestrant of formula V comprising reacting fulvestrant of formula I with compound of formula XI,

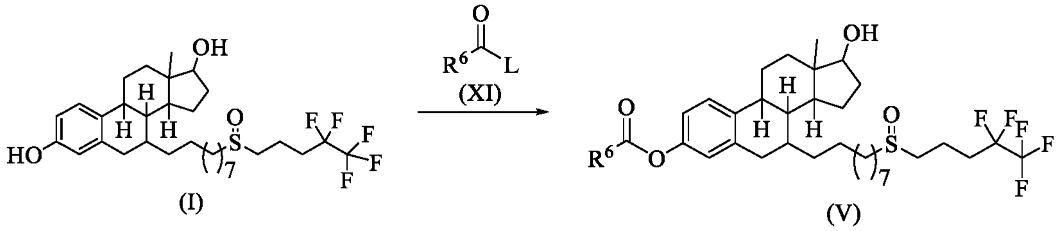

(I) (V)

wherein $R^6$ has same meaning as defined above and L is a leaving group.

In yet another aspect, the present invention relates to process for the preparation of prodrugs of fulvestrant of formula VI comprising reacting fulvestrant of formula I with compound of formula XII,

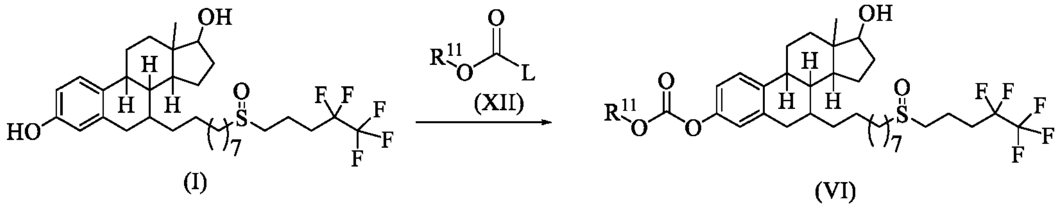

(I) (VI)

wherein $R^{11}$ has same meaning as defined above and L is a leaving group.

In yet another aspect, the present invention relates to process for the preparation of prodrugs of fulvestrant of formula VII comprising reacting fulvestrant of formula I with compound of formula XIII,

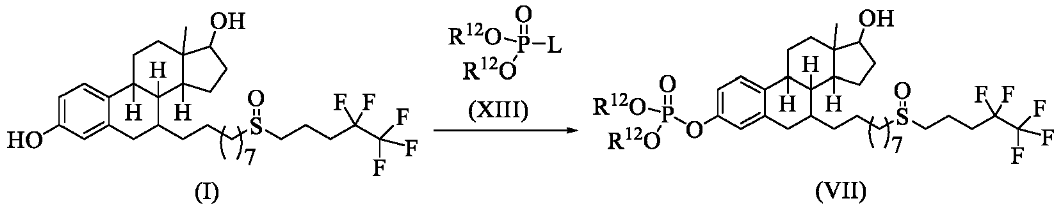

(I) (VII)

wherein $R^{12}$ has same meaning as defined above and L is a leaving group.

In yet another aspect, the present invention relates to process for the preparation of prodrugs of fulvestrant of formula VIII comprising reacting fulvestrant of formula I with compound of formula XIV,

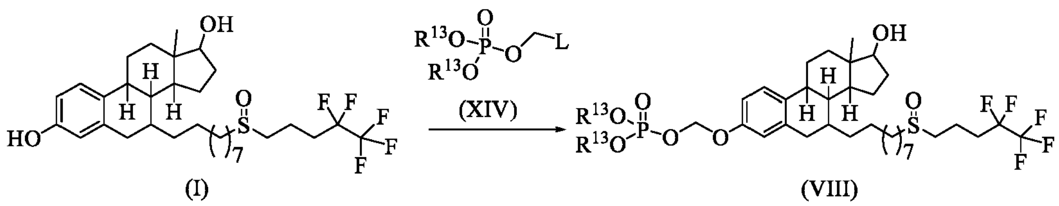

(I) (VIII)

wherein $R^{13}$ has same meaning as defined above and L is a leaving group.

In yet another aspect, the present invention relates to process for the preparation of prodrugs of fulvestrant of

formula XV comprising reacting compound of formula XVII with compound of formula XVI,

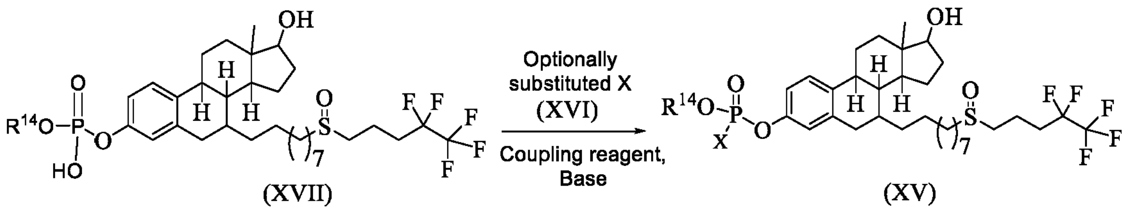


(XVII) (XV)

wherein $R^{14}$ and X have same meaning as defined above.

In yet another aspect, the present invention relates to pharmaceutical composition comprising prodrugs of fulvestrant of formula II and enantiomers, diastereomers, racemates, pharmaceutically acceptable salts and solvates thereof and pharmaceutically acceptable excipients.

In yet another aspect, the present invention relates to use of prodrugs of fulvestrant of formula II and enantiomers, diastereomers, racemates, pharmaceutically acceptable salts and solvates thereof in treatment of cancer.

In yet another aspect, fulvestrant prodrug offers an oral administration formulation that has the potential to improve efficacy over existing fulvestrant therapy.

## DETAILED DESCRIPTION OF THE INVENTION

As used herein, the term "prodrug" refers to a precursor compound that, following administration, releases a biologically active compound in vivo via a chemical or physiological process. A prodrug itself may either lack or possess the desired biological activity.

The terms "a" and "an" do not denote a limitation of quantity, but rather denote the presence of at least one of the referenced item.

The term "about" as used herein, when referring to a measurable value is meant to encompass variations of ±10%, preferably ±5%, more preferably ±1% and still more preferably ±0.1% from the specified value.

The term "cancer" refers to conditions including solid cancers, lymphomas and leukemias. Examples of different types of cancer include, but are not limited to, breast cancer, lung cancer, ovarian cancer, prostate cancer, colorectal cancer, liver cancer, renal cancer, bladder cancer, thyroid cancer, pleural cancer, pancreatic cancer, uterine cancer, cervical cancer, testicular cancer, anal cancer, bile duct cancer, gastrointestinal carcinoid tumors, esophageal cancer, gall bladder cancer, appendix cancer, small intestine cancer, stomach cancer, cancer of the central nervous system, skin cancer, choriocarcinoma, head and neck cancer, blood cancer, osteogenic sarcoma, fibrosarcoma, neuroblastoma, glioma, melanoma, B-cell lymphoma, non-Hodgkin's lymphoma, Burkitt's lymphoma, small cell lymphoma, large cell lymphoma, monocytic leukemia, myelogenous leukemia, acute lymphocytic leukemia, acute myelocytic leukemia, and multiple myeloma.

As used herein, the term "alkyl" refers to a straight or branched, saturated, aliphatic radical having from 1 to about 10 carbon atoms., for example, methyl, ethyl, propyl, isopropyl, n-butyl, i-butyl, t-butyl and the like.

The term "alkenyl" refers to a straight chain or branched hydrocarbon having at least 2 carbon atoms and at least one carbon-carbon double bond. Alkenyl groups can have any suitable number of double bonds, including, but not limited to 1, 2, 3, 4, 5 or more. Preferable alkenyl groups include ethenyl ($-CH=CH_2$), 2-propenyl (allyl, $-CH2-CH=CH_2$) and the like.

The term "alkynyl" denotes an alkynyl groups having from 2 to 10 carbon atoms and having at least 1-2 sites of alkynyl unsaturation, preferred alkynyl groups include ethynyl ($-C\equiv CH$), propargyl ($-CH_2-C\equiv CH$), 1-butenyl, 2-butenyl, isobutenyl, butadienyl and the like.

The term "cycloalkyl" denotes a saturated carbocyclic group of from 3 to 10 carbon atoms having a single ring (e.g., cyclohexyl) or multiple condensed rings (e.g., norbornyl). Preferred cycloalkyl include cyclopropyl, cyclobutyl, cyclpentyl, cyclohexyl, cycloheptyl, norbornyl and the like.

The term "heterocycloalkyl" denotes a C3-C10 cycloalkyl group according to the definition above, in which up to 4 carbon atoms are replaced by heteroatoms chosen from the group consisting of O, S or N. Preferred heterocycloalkyl include pyrrolidine, piperidine, piperazine, morpholine, tetrahydrofuran, tetrahydrothiophenyl, and the like.

The term "aryl" denotes a cyclic aromatic hydrocarbon radical consisting of one or more fused rings containing 6-14 carbon atoms in which at least one ring is aromatic in nature, for example phenyl, naphthyl, 1,2,3,4-tetrahydronaphthalenyl, indanyl and the like.

The term "heteroaryl" denotes a cyclic aromatic hydrocarbon radical consisting of one or more fused rings containing 5-14 ring atoms, preferably containing 5-10 ring atoms, in which at least one ring is aromatic in nature, and which contains at least one heteroatom, selected from N, O or S, for example pyridyl, pyrrolyl, furyl, thienyl, imidazolyl,

oxazolyl, isoxazolyl, thiazolyl, isothiazolyl, pyrazolyl, 1,2,3-triazolyl, 1,2,4-triazolyl, benzothienyl, benzotriazolyl, isobenzothienyl, indolyl, isoindolyl, benzimidazolyl, imidazo[1,2-a]pyridyl, benzothiazolyl, benzoxazolyl, quinolizinyl, quinazolinyl, benzoquinolyl and the like.

The term "halogen" denotes chlorine, iodine, fluorine and bromine.

As used herein, the term "leaving group" or "L" or "$L_1$"can be defined as part of a substrate that cleaved by the action of a nucleophile. Examples of leaving groups include, but are not limited to: halogen (F, Cl, Br, and I), tosylate, mesylate, triflate, acetate, hydroxyl, camphorsulfonate, aryloxide, and aryloxide and the like.

The tem "alkoxy" refers to the group -O-alkyl.

The tem "alkoxyalkyloxy" refers to the group alkyl-O-alky-O-

The tem "alkoxycarbonyloxy" refers to the group alkyl-O-CO-O-.

The term "optionally" means the subsequently described event or circumstance can or cannot occur, and that the description includes instances where the event or circumstance occurs and instances where it does not.

The term "pharmaceutically acceptable carrier" refers to a non-toxic carrier that may be administered to a patient, together with a compound of this invention, and which does not destroy the pharmacological activity thereof.

The term "pharmaceutically acceptable excipient" as used herein includes vehicles, adjuvants, or diluents or other auxiliary substances, such as those conventional in the art, which are readily available to the public. For example, pharmaceutically acceptable excipients include pH adjusting and buffering agents, tonicity adjusting agents, stabilizers, wetting agents and the like.

As used herein, the term "salt" refers to an acid or base salt of a compound of the invention. Salts of basic compounds are salts formed with mineral acids, organic carboxylic acids, organic sulfonic acids, and the like. Examples of suitable acids include hydrochloric, hydrobromic, sulfuric, nitric, perchloric, fumaric, maleic, phosphoric, glycollic, lactic, salicylic, succinic, toluene-p-Sulfonic, tartaric, acetic, citric, methanesulfonic, formic, benzoic, malonic, naphthalene-2-sulfonic and benzenesulfonic acids. Salts of acidic compounds are formed with bases, namely cationic species such as alkali and alkaline earth metal cations e.g., sodium, lithium, potassium, calcium, and magnesium ions, as well as ammonium cations e.g., ammonium, trimethylammonium and diethylammonium.

The compounds of this invention contain one or more asymmetric carbon atoms and thus occur as racemate and racemic mixtures, single enantiomers, diastereomeric mixtures and individual diastereomers. All such isomeric forms of these compounds are expressly included in the present invention. Each stereogenic carbon may be of the R or S configuration.

As used herein, the term "deprotecting agents" includes, but are not limited to, hydrogen and palladium on carbon ($H_2$, Pd/C), ammonium formate and palladium on carbon ($HCOONH_4$, Pd/C), hydrogen and palladium hydroxide on carbon ($H_2$, $Pd(OH)_2$/C), combination of Pd/C and $Pd(OH)_2$/C and acid such as hydrochloride acid, hydrobromic acid and the like.

For purposes of the present invention, the term "substituted" shall be understood to include adding or replacing one or more atoms contained within a functional group or compound with one or more different atoms.

Unless otherwise constrained by the definition of the individual substituent, the above set out groups, like "alkyl", "alkenyl", "alkynyl", "cycloalkyl", "heterocycloalkyl", "aryl" and "heteroaryl" etc. groups can optionally be substituted with from 1 to 5 substituents selected from the group consisting of "C1-C8-alkyl", "C2-C8-alkenyl", "C2-C8-alkynyl", "cycloalkyl", "heterocycloalkyl", "aryl", "heteroaryl", "amino", "alkylamino", "acyl", "acyloxy", "acylamino", "aminocarbonyl", "alkoxycarbonyl", "alkoxyalkyloxy", "alkoxycarbonyloxy", "carbamate," "sulfinyl", "sulfonyl", "alkoxy", "sulfanyl", "halogen", "carboxy", trihalomethyl, cyano, hydroxy, mercapto, nitro and the like.

The term "coupling reagent" as used herein includes but not limited to O-benzotriazole-N,N,N',N'-tetramethyl-uronium-hexafluoro-phosphate (HBTU), 2-(7-Aza-1H-benzotriazole-1-yl)-1,1,3,3-tetramethyluroniumhexafluorophosphate (HATU), acid halide, 1-hydroxybenzotriazole (HOBt), 1-Hydroxy-7-aza-1H-benzotriazole (HOAt), diisopropylcarbodiimide (DIC), dicyclohexylcarbodiimide (DCC), N-(3-Dimethylaminopropyl)-N'-ethylcarbodiimide hydrochloride (EDC), 2-(6-Chloro-1H-benzotriazol-1-yl)-N,N,N',N'-tetramethylaminium hexafluoro-phosphate (HCTU), 1-[1-(Cyano-2-ethoxy-2-oxoethylideneaminooxy) dimethyl-aminomorpholino]-uroniumhexa-fluorophosphate (COMU) and the like.

Base used in the present invention can be inorganic and organic base. The examples of organic base includes but not limiting to amines such as diisopropylethylamine, triethylamine, pyridine, 1,8-diazabicyclo[5.4.0]undec-7-ene (DBU), imidazole, N,N-dimethyl aniline, N,N-dimethyl amino pyridine (DMAP), 1,5-diazabicyclo[4.3.0]non-5-ene (DBN) and the like or mixtures thereof. The examples of inorganic base includes but not limiting to alkali or alkaline earth metal carbonate, bicarbonate, hydroxide or phosphate such as potassium carbonate, sodium carbonate, lithium carbonate, sodium bicarbonate, potassium bicarbonate, lithium bicarbonate, sodium hydroxide, potassium hydroxide, lithium hydroxide, potassium phosphate, sodium phosphate; hydride such as sodium hydride, lithium hydride or potassium hydride; alkoxide such as sodium or potassium methoxide or ethoxide, tertiary butoxide and the like or mixtures thereof.

As used herein, the term "solvent" refers to the solvents include, but are not limited to, nitriles such as acetonitrile, propionitrile, butyronitrile, isobutyronitrile and the like; ethers such as dioxane, diethyl ether, diisopropylether, tetrahydrofuran, dimethoxyethane and the like; hydrocarbon such as toluene, xylene, hexane, heptane, cyclohexane and the like; chlorinated hydrocarbon such as methylene chloride, ethylene dichloride, carbon tetra chloride, chloroform, chlorobenzene and the like; polar aprotic solvents such as N,N-dimethylformamide (DMF), dimethyl acetamide (DMAc), dimethyl sulfoxide (DMSO) and the like or mixtures thereof.

The novel compounds of the present invention can be used in conventional solid or liquid pharmaceutical forms, for example as uncoated or film coated tablets, capsules, powders, granules, solutions or sprays. The active substances can for this purpose be processed with conventional pharmaceutical aids such as tablet binders, bulking agents, preservatives, tablet disintegrants, flow regulators, plasticizers, wetting agents, dispersants, emulsifiers, solvents, release-slowing agents, antioxidants and/or propellant gases.

The prodrugs of the present invention are characterized by unexpectedly high aqueous solubility. This solubility facilitates administration of higher doses of the prodrug, resulting in a greater drug load per unit dosage. The prodrugs of the present invention are also characterized by facile hydrolytic cleavage to release the active fulvestrant in vivo. The high aqueous solubility and the facile in vivo metabolism result in a greater bioavailability of the drug.

When the prodrugs of this invention are administered in combination therapies with other agents, they may be administered sequentially or concurrently to the patient. Alternatively, pharmaceutical compositions according to this invention may be comprised of a combination of a prodrug of this invention and another therapeutic agent.

In one aspect, the present invention relates to prodrugs of fulvestrant of formula I-A and enantiomers, diastereomers, racemates, pharmaceutically acceptable salts and solvates thereof,

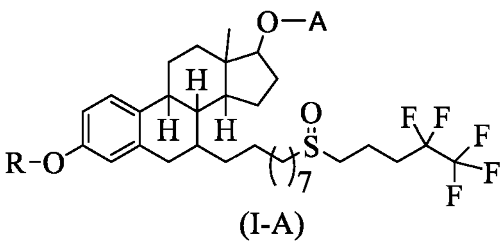

(I-A)

wherein

A is selected from hydrogen,

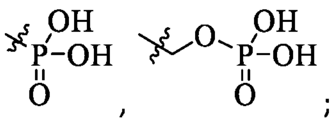

and

R is selected from group comprising of:

a)

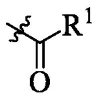

wherein $R^1$ is selected from group comprising of substituted aryl; optionally substituted alkyl, alkenyl, alkynyl, cycloalkyl, heteroaryl and $CR^2R^3$. $R^2$ and $R^3$ are taken together along with the carbon to which they are attached to form a three to seven membered saturated, partially unsaturated or unsaturated heterocyclic ring in which up to 4 carbon atoms are replaced by heteroatoms chosen from the group consisting of O, S or N and may optionally be substituted at a substitutable position with one or more $R^4$ radicals, wherein the $R^4$ radicals are independently selected at each occurrence from the group consisting of alkyl, cycloalkyl, heterocycloalkyl, aryl, heteroaryl, alkylcarbonyl, formyl, halo, alkylphosphate, phosphate, cyano, nitro, alkoxy, alkoxycarbonyl, alkenyl, alkynyl, alkylthio and arylcarbonyl;

b)

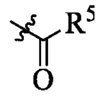

wherein $R^5$ is

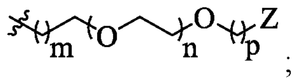

wherein m and p are independently selected from 0 to 5, n refers to degree of polymerization and is selected from 1 to 250 and Z is optionally substituted alkyl or cycloalkyl;

c)

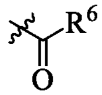

wherein $R^6$ is selected from $NH_2$, $NHR^7$ and $NR^8R^9$. $R^7$ is selected from optionally substituted alkyl, alkenyl, alkynyl, cycloalkyl, heterocycloalkyl, aryl and heteroaryl. $R^8$ and $R^9$ are taken together along with the nitrogen to which they are attached to form a three to seven membered saturated, partially unsaturated or unsaturated heterocyclic ring and may optionally be substituted at a substitutable position with one or more $R^{10}$ radicals, wherein the $R^{10}$ radicals are independently selected at each occurrence from the group consisting of alkyl, alkylcarbonyl, formyl, halo, haloalkyl, alkylphosphate, phosphate, oxo, cyano, nitro, amino, alkoxy, alkoxycarbonyl, carboxyalkyl, hydroxyalkyl, alkenyl, alkynyl, alkylthio, cycloalkyl, aryl, aralkyl, heterocycloalkyl, alkylthioalkyl, arylcarbonyl, aralkylcarbonyl and alkoxyalkyl;

d)

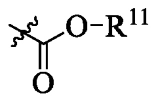

wherein $R^{11}$ is selected from group comprising of optionally substituted alkyl, alkenyl, alkynyl, cycloalkyl, heterocycloalkyl, aryl and heteroaryl;

e)

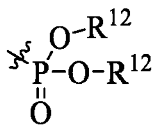

wherein each $R^{12}$ is selected independently from group comprising of hydrogen; optionally substituted alkyl, aryl, acyl and heteroaryl;
with a proviso that when A is hydrogen and one of $R^{12}$ substitution is selected from alkyl and aryl, then another $R^{12}$ substitution is hydrogen;

f)

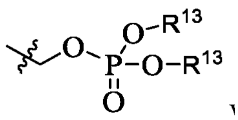

wherein each $R^{13}$ is selected independently from group comprising of hydrogen; optionally substituted alkyl, cycloalkyl, heterocycloalkyl, aryl and heteroaryl;

g)

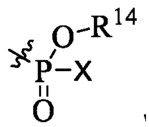

**wherein** $R^{14}$ is selected from group comprising of hydrogen; optionally substituted alkyl, aryl, acyl, heteroaryl; and X is optionally substituted heterocycloalkyl; and

h) hydrogen with the proviso that when R is hydrogen A is not hydrogen.

In one embodiment, the present invention relates to prodrugs of fulvestrant of formula III-A and enantiomers, diastereomers, racemates, pharmaceutically acceptable salts and solvates thereof,

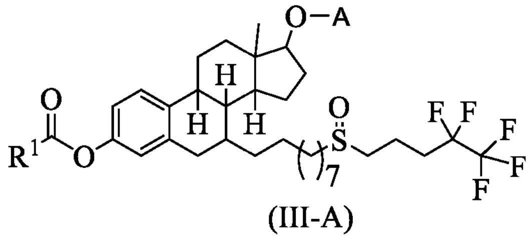

(III-A)

Wherein $R^1$ and A has same meaning as defined above.

In another embodiment, the present invention relates to prodrugs of fulvestrant of formula IV-A and enantiomers, diastereomers, racemates, pharmaceutically acceptable salts and solvates thereof,

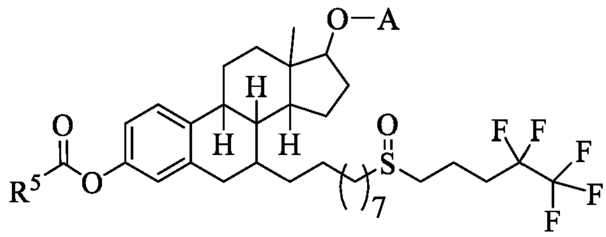

(IV-A)

wherein $R^5$ and A have same meaning as defined above.

In yet another embodiment, the present invention relates to prodrugs of fulvestrant of formula V-A and enantiomers, diastereomers, racemates, pharmaceutically acceptable salts and solvates thereof,

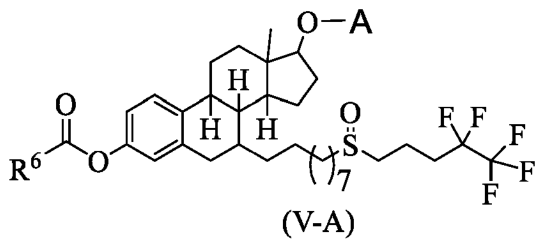

(V-A)

wherein $R^6$ and A have same meaning as defined above.

In yet another embodiment, the present invention relates to prodrugs of fulvestrant of formula VI-A and enantiomers, diastereomers, racemates, pharmaceutically acceptable salts and solvates thereof,

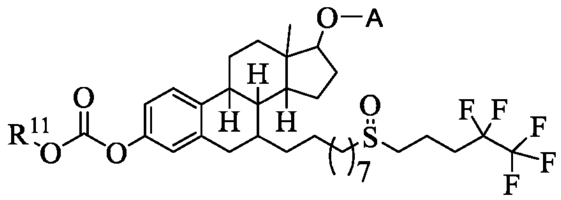

(VI-A)

wherein $R^{11}$ and A have same meaning as defined above.

In yet another embodiment, the present invention relates to prodrugs of fulvestrant of formula VII-A and enantiomers, diastereomers, racemates, pharmaceutically acceptable salts and solvates thereof,

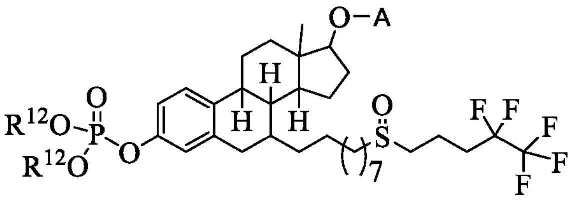

(VII-A)

wherein $R^{12}$ and A have same meaning as defined above.

In yet another embodiment, the present invention relates to prodrugs of fulvestrant of formula VIII-A and enantiomers, diastereomers, racemates, pharmaceutically acceptable salts and solvates thereof,

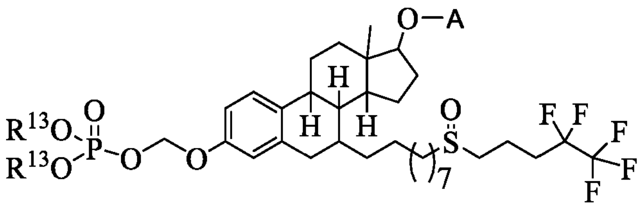

(VIII-A)

wherein $R^{13}$ and A have same meaning as defined above.

In yet another embodiment, the present invention relates to prodrugs of fulvestrant of formula XV-A and enantiomers, diastereomers, racemates, pharmaceutically acceptable salts and solvates thereof,

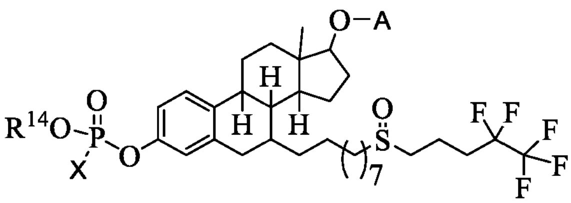

(XV-A)

wherein $R^{14}$, X and A have same meaning as defined above.

In another aspect, the present invention relates to prodrugs of fulvestrant of formula II and enantiomers, diastereomers, racemates, pharmaceutically acceptable salts and solvates thereof,

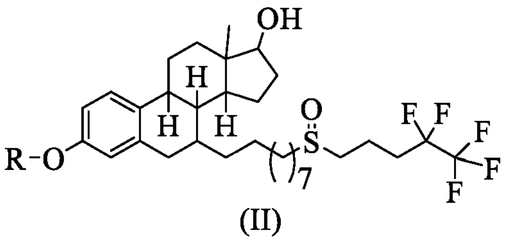

(II)

wherein R is selected from group comprising of:

a)

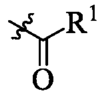

wherein $R^1$ is selected from group comprising of substituted aryl; optionally substituted alkyl, alkenyl, alkynyl, cycloalkyl, heteroaryl and $CR^2R^3$. $R^2$ and $R^3$ are taken together along with the carbon to which they are attached to form a three to seven membered saturated, partially unsaturated or unsaturated heterocyclic ring in which up to 4 carbon atoms are replaced by heteroatoms chosen from the group consisting of O, S or N and may optionally be substituted at a substitutable position with one or more $R^4$ radicals, wherein the $R^4$ radicals are independently selected at each occurrence from the group consisting of alkyl, cycloalkyl, heterocycloalkyl, aryl, heteroaryl, alkylcarbonyl, formyl, halo, alkylphosphate, phosphate, cyano, nitro, alkoxy, alkoxycarbonyl, alkenyl, alkynyl, alkylthio and arylcarbonyl;

b)

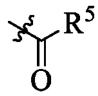

wherein $R^5$ is

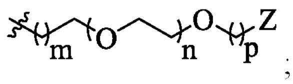

wherein m and p are independently selected from 0 to 5, n refers to degree of polymerization and selected from 1 to 250 and Z is optionally substituted alkyl or cycloalkyl;

c)

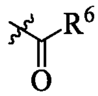

wherein $R^6$ is selected from $NH_2$, $NHR^7$ and $NR^8R^9$. $R^7$ is selected from optionally substituted alkyl, alkenyl, alkynyl, cycloalkyl, heterocycloalkyl, aryl and heteroaryl. $R^8$ and $R^9$ are taken together along with the nitrogen to which they are attached to form a three to seven membered saturated, partially saturated or unsaturated heterocyclic ring and may optionally be substituted at a substitutable position with one or more $R^{10}$ radicals, wherein the $R^{10}$ radicals are independently selected at each occurrence from the group consisting of alkyl, alkylcarbonyl, formyl, halo, haloalkyl, alkylphosphate, phosphate, oxo, cyano, nitro, amino, alkoxy, alkoxycarbonyl, carboxyalkyl, hydroxyalkyl, alkenyl, alkynyl, alkylthio, cycloalkyl, aryl, aralkyl, heterocycloalkyl, alkylthioalkyl, arylcarbonyl, aralkylcarbonyl and alkoxyalkyl;

d)

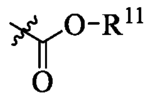

wherein $R^{11}$ is selected from group comprising of optionally substituted alkyl, alkenyl, alkynyl, cycloalkyl, heterocycloalkyl, aryl and heteroaryl;

e)

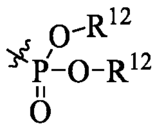

wherein each $R^{12}$ is selected independently from group comprising of hydrogen; optionally substituted alkyl, aryl, acyl and heteroaryl;
with a proviso that when one of $R^{12}$ is selected from alkyl and aryl, then another $R^{12}$ substitution is hydrogen;

f)

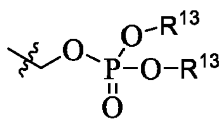

wherein each $R^{13}$ is selected from group comprising of hydrogen; optionally substituted alkyl, cycloalkyl, heterocycloalkyl, aryl and heteroaryl; and

g)

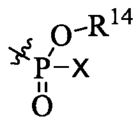

wherein $R^{14}$ is selected from group comprising of hydrogen; optionally substituted alkyl, aryl, acyl, heteroaryl; and X is optionally substituted heterocycloalkyl.

In one embodiment, the present invention relates to prodrugs of fulvestrant of formula III and enantiomers, diastereomers, racemates, pharmaceutically acceptable salts and solvates thereof,

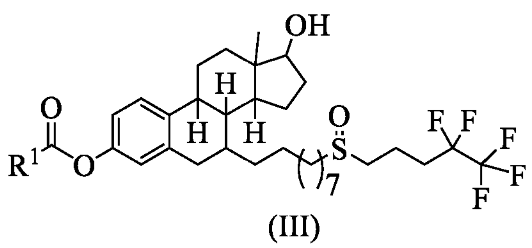

(III)

Wherein $R^1$ has same meaning as defined above.

In certain aspects, there is provided a compound of Formula III and enantiomers, diastereomers, racemates, pharmaceutically acceptable salts and solvates thereof,

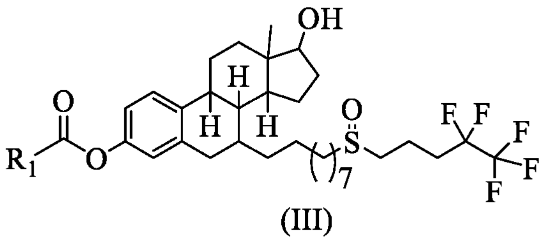

(III)

wherein $R^1$ is selected from:

| | | | |
|---|---|---|---|
| 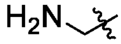 | 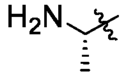 | 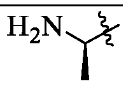 | 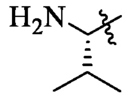 |
| 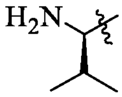 | 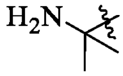 | 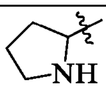 | 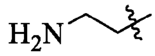 |
| 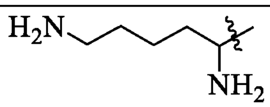 | 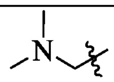 | 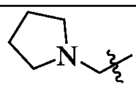 | 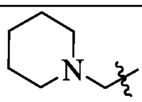 |
| 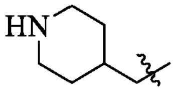 | 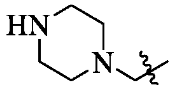 | 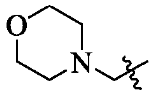 | 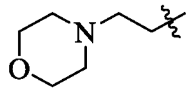 |
| 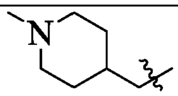 | 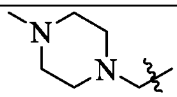 | 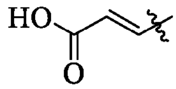 | 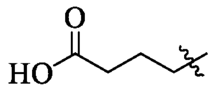 |
| 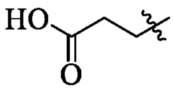 | 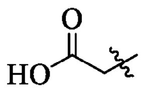 | 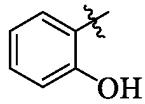 | 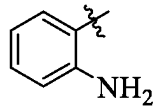 |
| 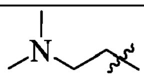 | | | |

In another embodiment, the present invention relates to prodrugs of fulvestrant of formula IV and enantiomers, diastereomers, racemates, pharmaceutically acceptable salts and solvates thereof,

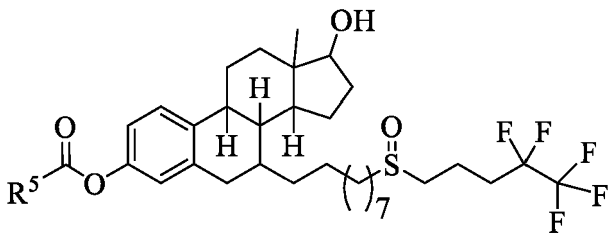

(IV)

wherein $R^5$ has same meaning as defined above.

In certain aspects, there is provided a compound of Formula IV and enantiomers, diastereomers, racemates, pharmaceutically acceptable salts and solvates thereof,

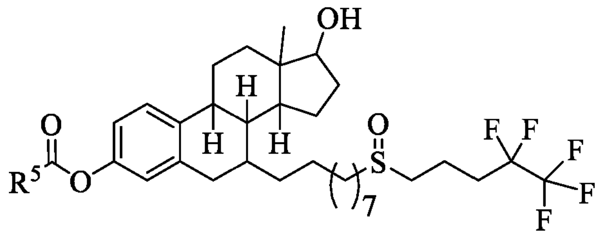

(IV)

wherein $R^5$ is selected from:

| | |
|---|---|
| 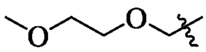 | 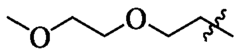 |
| 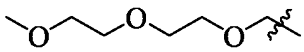 | 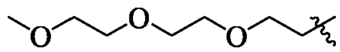 |
| 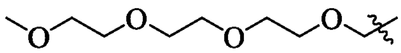 | 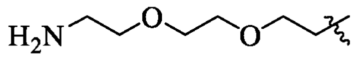 |
| 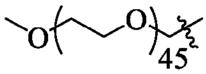 | 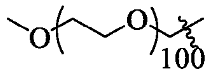 |
| 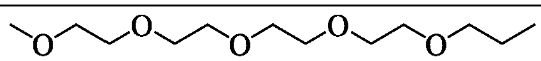 | 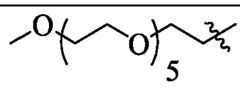 |
| 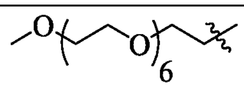 | |

In yet another embodiment, the present invention relates to prodrugs of fulvestrant of formula V and enantiomers, diastereomers, racemates, pharmaceutically acceptable salts and solvates thereof,

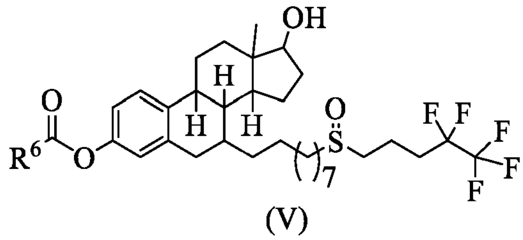

(V)

wherein $R^6$ has same meaning as defined above.

In certain aspects, there is provided a compound of Formula V and enantiomers, diastereomers, racemates, pharmaceutically acceptable salts and solvates thereof,

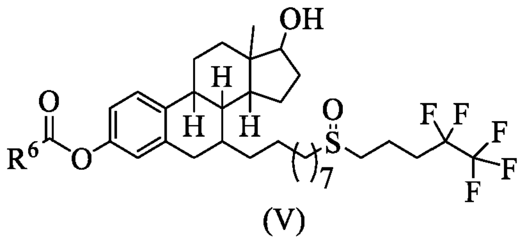

(V)

wherein $R^6$ is selected from:

| | | |
|---|---|---|
| 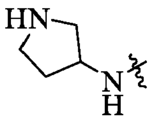 | 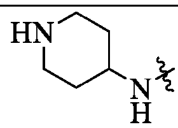 | 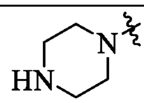 |
| 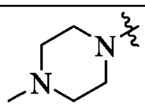 | 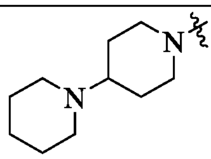 | 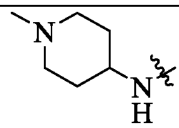 |
| 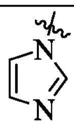 | 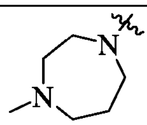 | 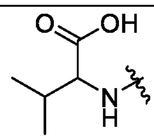 |
| 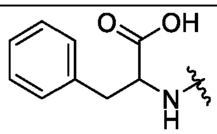 | 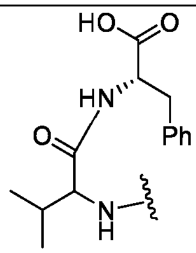 | |

In yet another embodiment, the present invention relates to prodrugs of fulvestrant of formula VI and enantiomers, diastereomers, racemates, pharmaceutically acceptable salts and solvates thereof,

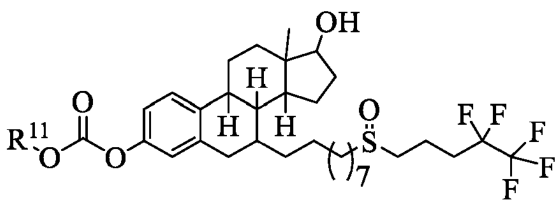

(VI)

wherein $R^{11}$ has same meaning as defined above.

In certain aspects, there is provided a compound of Formula VI and enantiomers, diastereomers, racemates, pharmaceutically acceptable salts and solvates thereof,

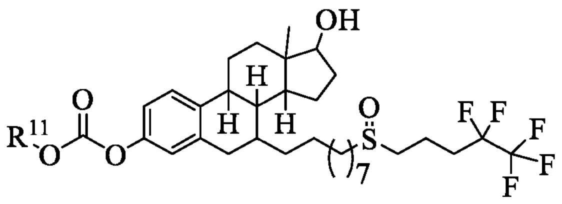

(VI)

wherein $R^{11}$ is selected from:

| | | |
|---|---|---|
| 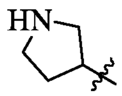 | 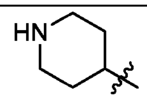 | 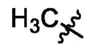 |
| 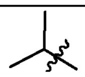 | 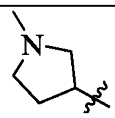 | 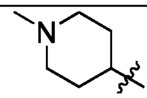 |

In yet another embodiment, the present invention relates to prodrugs of fulvestrant of formula VII and enantiomers, diastereomers, racemates, pharmaceutically acceptable salts and solvates thereof,

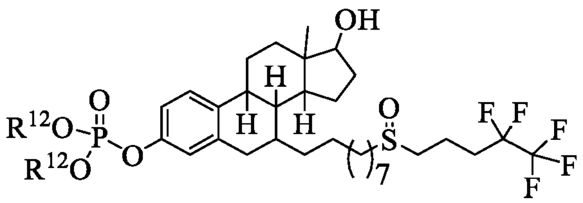

(VII)

wherein $R^{12}$ has same meaning as defined above.

In certain aspects, there is provided a compound of Formula VII and enantiomers, diastereomers, racemates, pharmaceutically acceptable salts and solvates thereof,

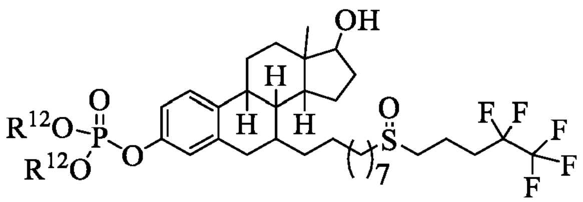

(VII)

wherein $R^{12}$ is independently selected from hydrogen, methyl, propyl, isopropyl, n-butyl,

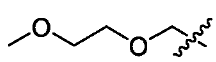 , 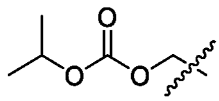 ;

with a proviso that when one of $R^{12}$ is selected from alkyl and aryl, another $R^{12}$ substitution is hydrogen.

In yet another embodiment, the present invention relates to prodrugs of fulvestrant of formula VIII and enantiomers, diastereomers, racemates, pharmaceutically acceptable salts and solvates thereof,

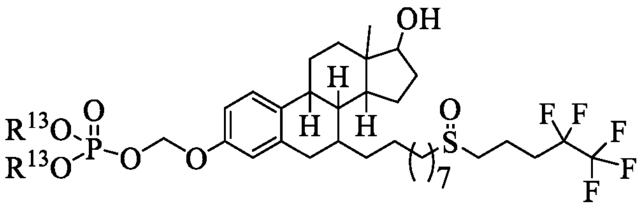

(VIII)

wherein $R^{13}$ has same meaning as defined above.

In certain aspects, there is provided a compound of Formula VIII and enantiomers, diastereomers, racemates, pharmaceutically acceptable salts and solvates thereof,

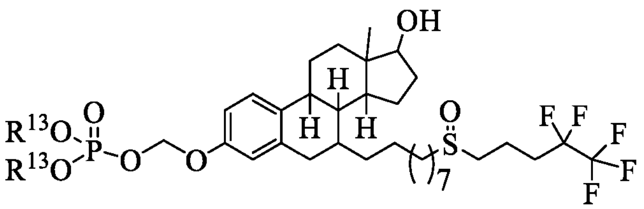

(VIII)

wherein $R^{13}$ is independently selected from hydrogen, methyl, ethyl, propyl, isopropyl, n-butyl, t-butyl, cyclopropyl, hexyl, phenyl, 3-pyridyl.

In yet another embodiment, the present invention relates to prodrugs of fulvestrant of formula XV and enantiomers, diastereomers, racemates, pharmaceutically acceptable salts and solvates thereof,

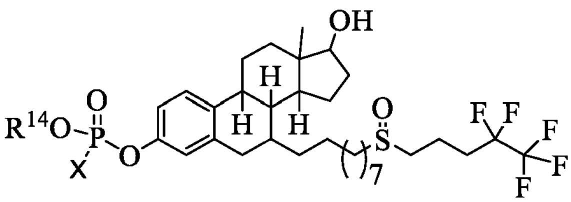

(XV)

wherein $R^{14}$ and X have same meaning as defined above.

In certain aspects, there is provided a compound of formula XV and enantiomers, diastereomers, racemates, pharmaceutically acceptable salts and solvates thereof,

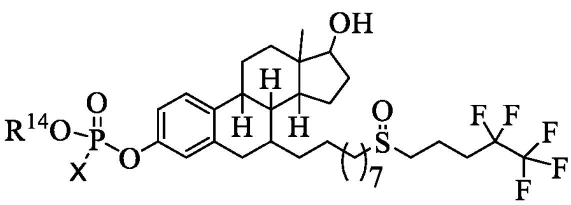

(XV)

wherein $R^{14}$ is selected from hydrogen, methyl, propyl, isopropyl, n-butyl and the like.and X is selected from optionally substituted aziridine, azetidine, pyrrolidine, piperidine, azepane, azocane.

In another aspect, the invention provides compound selected from the group comprising of:

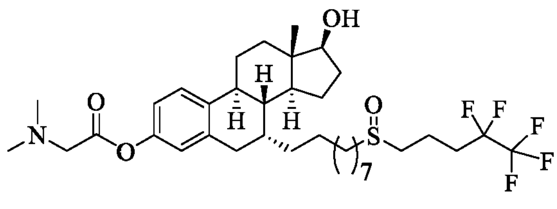

(I-a)

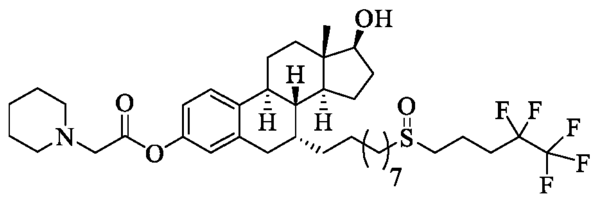

(I-b)

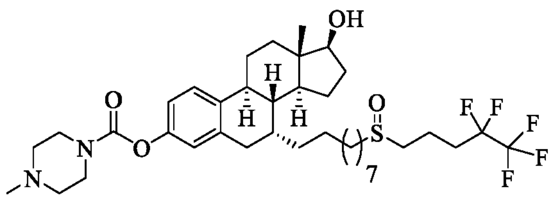

(I-c)

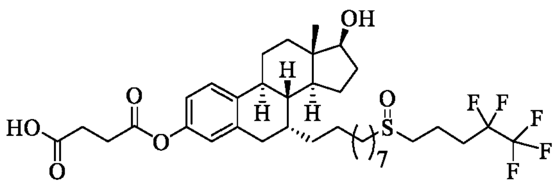

(I-d)

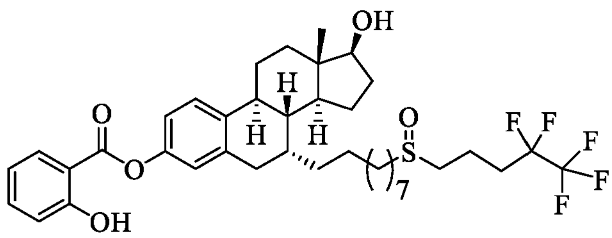

(I-e)

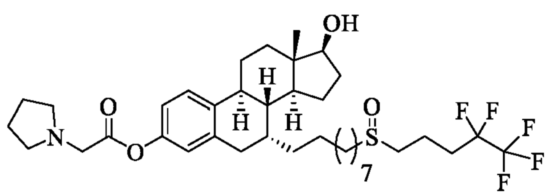

(I-g)

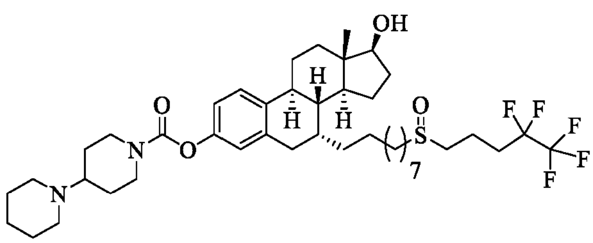

(I-h)

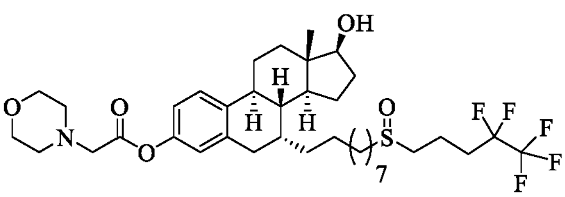

(I-i)

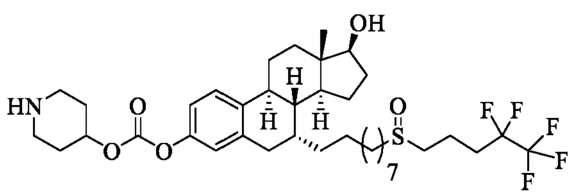

(I-j)

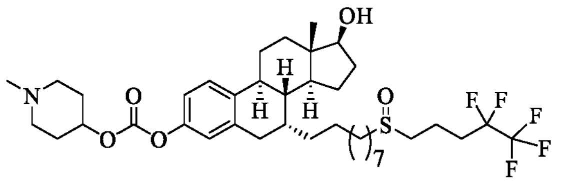

(I-k)

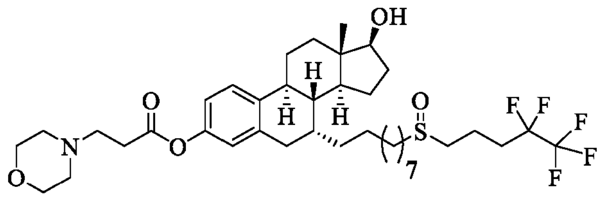

(I-l)

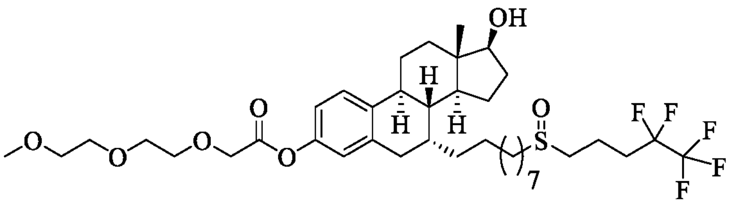

(I-m)

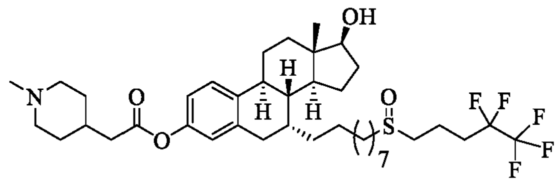

(I-n)

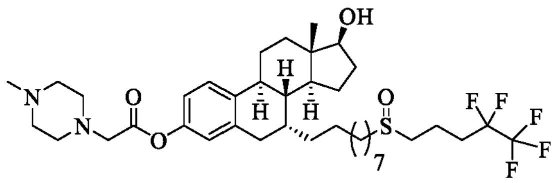

(I-o)

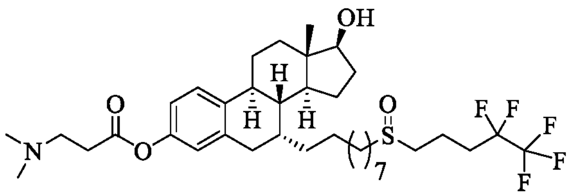

(I-p)

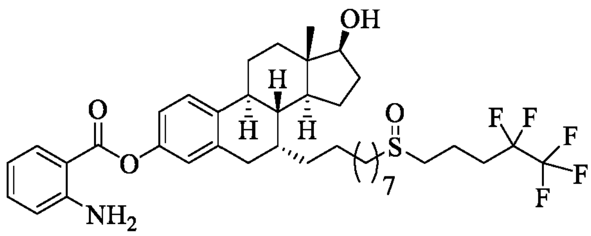

(I-q)

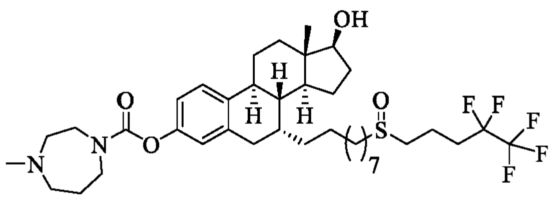

(I-r)

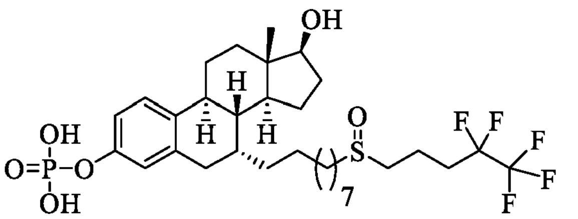

(I-s)

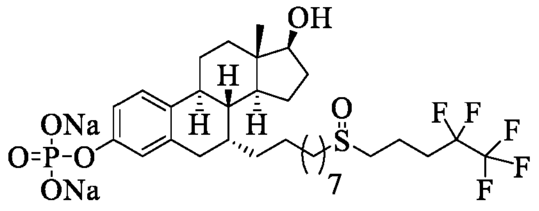

(I-t)

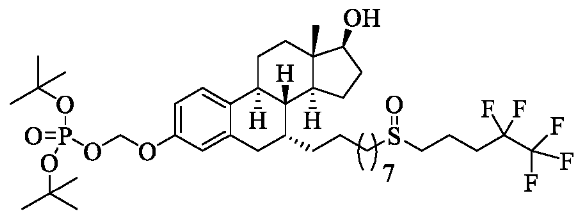

(I-u)

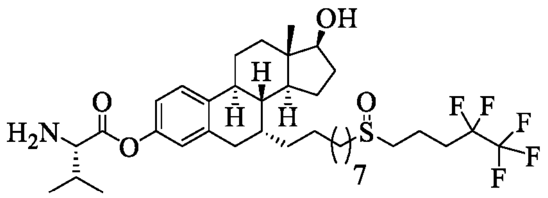

(I-v)

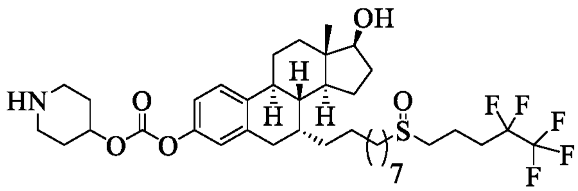

(I-w)

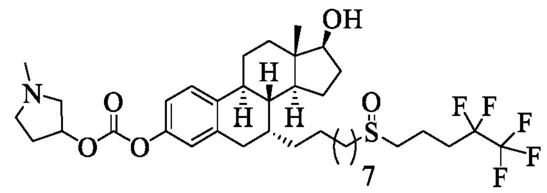

(I-x)

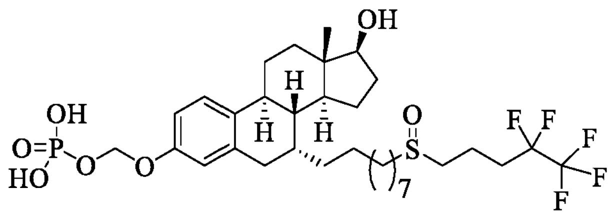

(I-y)

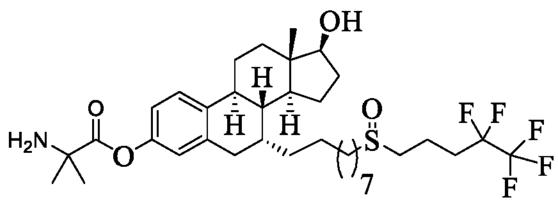

(I-z)

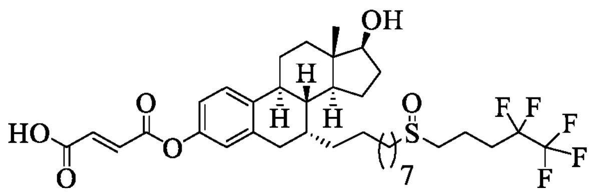

(I-aa)

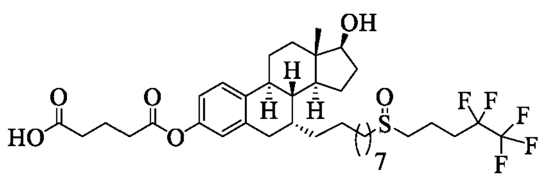

(I-ab)

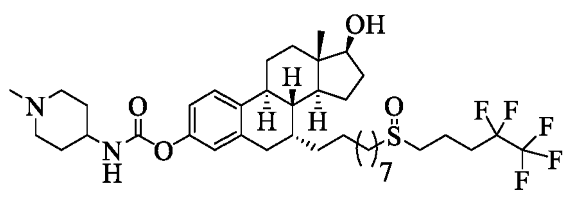

(I-ac)

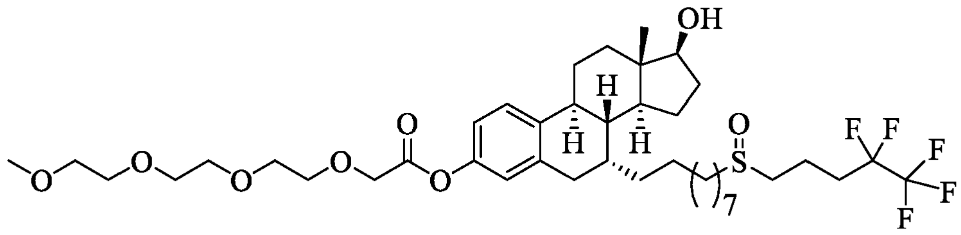

(I-ad)

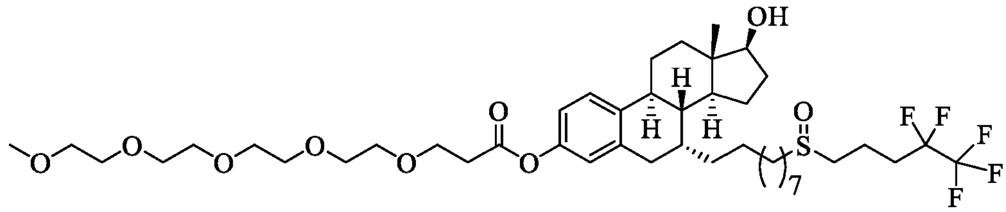

(I-ae)

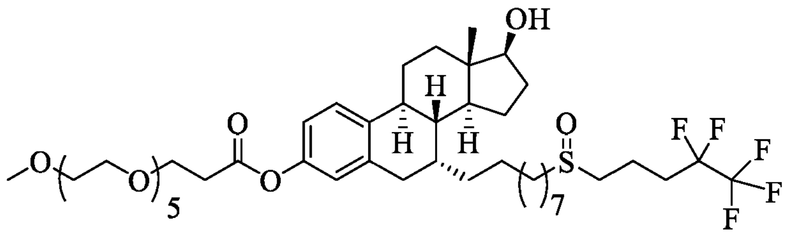

(I-af)

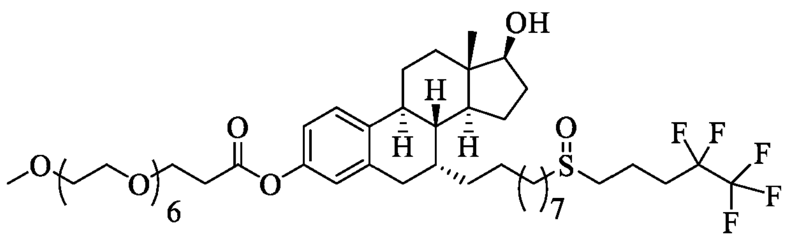

(I-ag)

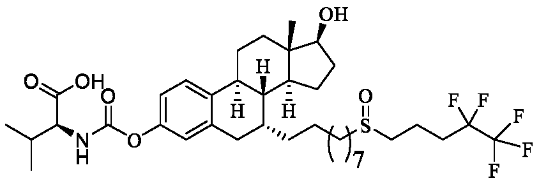

(I-ah)

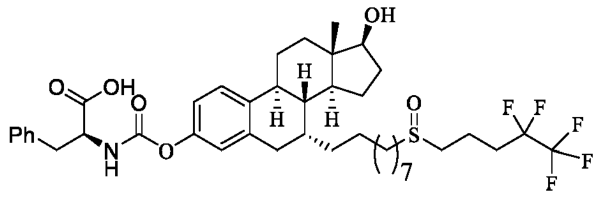

(I-ai)

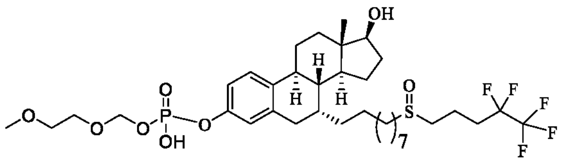

(I-aj)

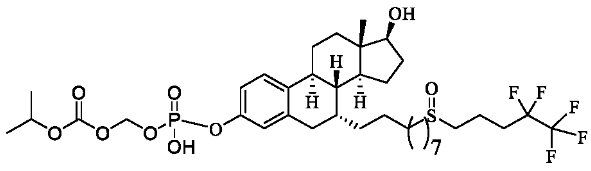

(I-ak)

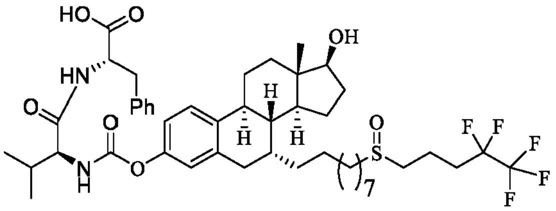

(I-al)

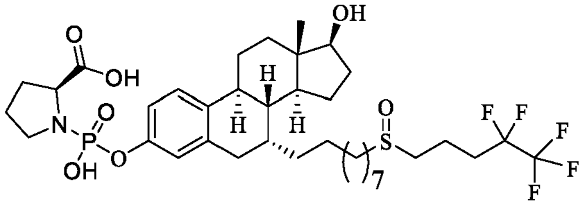

(I-am)

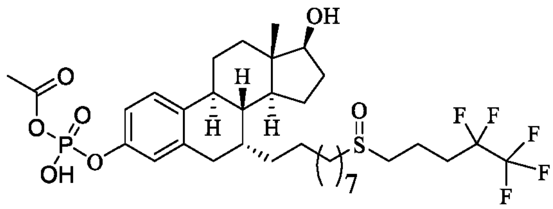

(I-an)

;

and enantiomers, diastereomers, racemates, pharmaceutically acceptable salts and solvates thereof.

In another aspect, the present invention relates to process for the preparation of prodrugs of fulvestrant of formula III comprising reacting fulvestrant of formula I with compound of formula IX,

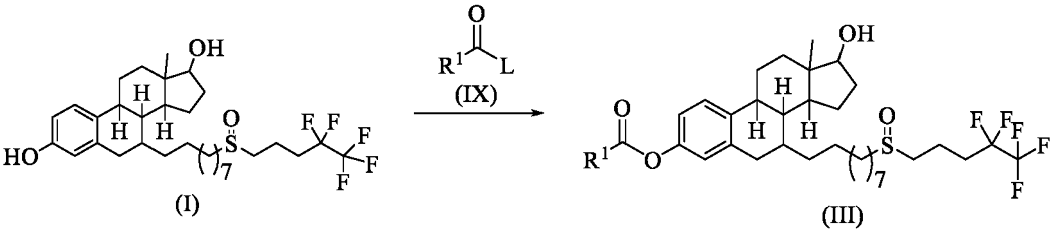

(IX)

(I)

(III)

wherein $R^1$ has same meaning as defined above and L is a leaving group.

In one embodiment, fulvestrant used for the preparation of pro-drugs can be prepared according to the process disclosed in prior-art and known to person having ordinary skills in the art.

An examples of compound of formula IX includes, but are not limited to dimethylglycine, 2-(piperidin-1-yl)acetic acid, salicylic acid, 3-morpholinopropanoic acid, 2-(pyrrolidin-1-yl)acetic acid, 2-morpholinoacetic acid, 3-morpholinopropanoic acid, 2-(1-methylpiperidin-4-yl)acetic acid, 2-(4-methylpiperazin-1-yl)acetic acid, 3-(dimethylamino)propanoic acid, anthranilic acid, (tert-butoxycarbonyl)-L-valine, 2-(tert-butoxycarbonylamino)-2-methylpropionic acid, maleic anhydride, succinic anhydride, glutaric anhydride and the like.

The reaction can be carried out at any temperature ranging from about -10° C to about 120° C.

In yet another aspect, the present invention relates to process for the preparation of prodrugs of fulvestrant of formula IV comprising reacting fulvestrant of formula I with compound of formula X,

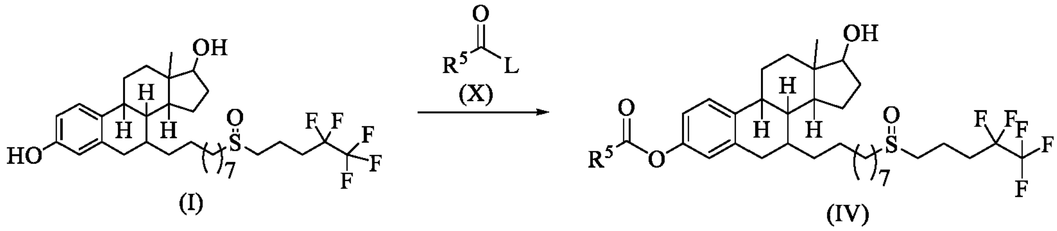


(I) (IV)

wherein $R^5$ has same meaning as defined above and L is a leaving group.

An examples of compound of formula X includes, but are not limited to 2-(2-methoxyethoxy)acetyl chloride, 3-(2-methoxyethoxy)propanoyl chloride, 2-(2-(2-methoxyethoxy)ethoxy)acetyl chloride, 3-(2-(2-methoxyethoxy)ethoxy)- propanoyl chloride, 2-(2-(2-methoxyethoxy)ethoxy)acetic acid, 2,5,8,11-tetraoxatridecan-13-oic acid, 2,5,8,11,14-pentaoxaheptadecan-17-oic acid, 2,5,8,11,14,17,20-heptaoxatricosan-23-oic acid, 2,5,8,11,14,17-hexaoxaicosan-20-oic acid and the like.

In yet another aspect, the present invention relates to process for the preparation of prodrugs of fulvestrant of formula V comprising reacting fulvestrant of formula I with compound of formula XI,

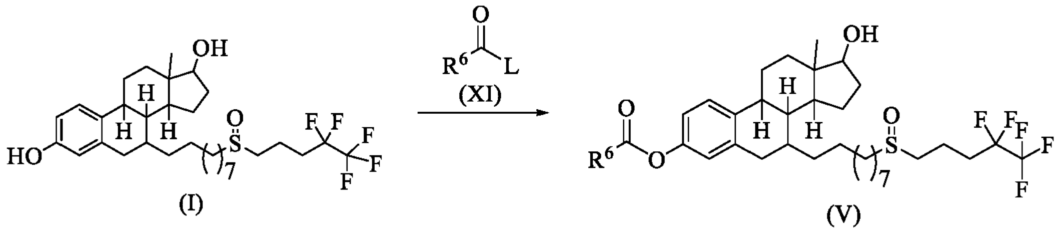


(I) (V)

wherein $R^6$ has same meaning as defined above and L is a leaving group.

An examples of compound of formula XI includes, but are not limited to 4-methylpiperazine-1-carbonyl chloride, [1,4'-bipiperidine]-1'-carbonyl chloride, morpholine-4-carbonyl chloride, piperidine-1-carbonyl chloride, 4-methyl-1,4-diazepane-1-carbonyl chloride, (1-methylpiperidin-4-yl)carbamic chloride and the like.

In yet another aspect, the present invention relates to process for the preparation of prodrugs of fulvestrant of formula VI comprising reacting fulvestrant of formula I with compound of formula XII;

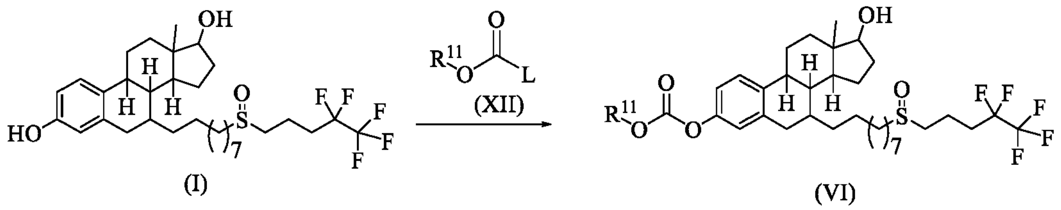


(I) (VI)

wherein $R^{11}$ has same meaning as defined above and L is a leaving group.

An examples of compound of formula XII includes, but are not limited to methyl chloroformate, ethyl chloroformate, isopropyl chloroformate, tert-butyl 4-((chlorocarbonyl)oxy)piperidine-1-carboxylate, 1-methylpiperidin-4-yl (4-nitrophenyl) carbonate, 1-methylpyrrolidin-3-yl carbonochloridate and the like.

In certain aspect, fulvestrant of formula I first reacted with protected compound of formula XII to provide protected compound of formula VI followed by reaction with deprotecting agents to provide compound of formula VI.

Optionally, deprotection can be carried out in the presence or absence of a solvent. The solvents that can be used in the said reaction is selected from, but is not limited to, nitriles such as acetonitrile; ketones such as acetone, methyl ethyl ketone; ethers such as tetrahydrofuran, diethyl ether; alcohols such as methanol, ethanol, isopropanol; amides such as dimethyl acetamide, acetamide, N,N-dimethylformamide; sulfoxides such as dimethyl sulfoxide; aromatic hydrocarbons such as toluene, xylene; halogenated hydrocarbons such as dichloromethane, dichloroethane, chloro benzene or mixtures thereof.

The molar ratio of deprotecting agent can be derived by a person skilled in the art. For example, the said mole ratio can be about 0.01, about 0.02, about 0.05, about 0.1, about 0.2, about 0.5, about 1.0, about 1.5, about 2, about 2.5, or about 3 mole per mole of the protected compound of formula VI, or any other suitable mole ratio.

The reaction time should be sufficient to complete the reaction which depends on scale and mixing procedures, as is commonly known to one skilled in the art. Typically, the reaction time can vary from about few minutes to several

hours. For example, the reaction time can be from about 10 min. to about 24 h, or any other suitable time period.

In yet another aspect, the present invention relates to process for the preparation of prodrugs of fulvestrant of formula VII comprising reacting fulvestrant of formula I with compound of formula XIII,

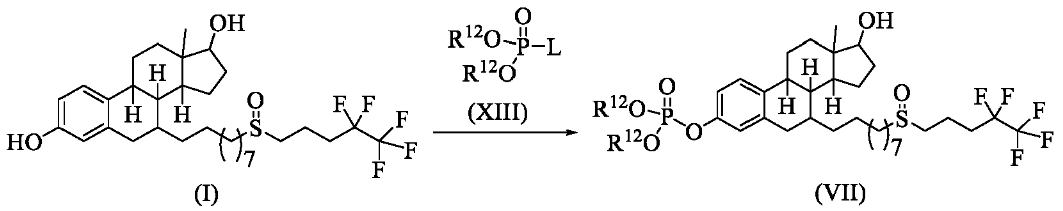

wherein $R^{12}$ has same meaning as defined above and L is a leaving group.

An examples of compound of formula XIII includes, but are not limited to dimethyl chlorophosphate, diisopropyl chlorophosphate, dibutyl chlorophosphate, chlorophosphonic acid, tetrabenzyl pyrophosphate, dibenzyl phosphorochloridate and the like.

In certain aspect, compound of formula VII, wherein $R^{12}$ is hydrogen, may be prepared from compound of formula VII, wherein $R^{12}$ is other than hydrogen, by reaction with suitable deprotecting reagents. Solvents used for this step include, but are not limited to, alcohols, esters, hydrocarbons, chlorinated hydrocarbons, polar solvents, polar aprotic solvents or mixtures thereof. The reaction can be carried out at any temperature ranging from about -10° C to about 160° C.

In yet another aspect, the present invention relates to process for the preparation of prodrugs of fulvestrant of formula VIII comprising reacting fulvestrant of formula I with compound of formula XIV,

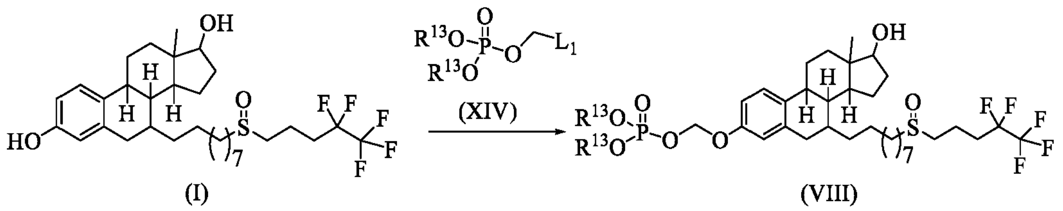

wherein $R^{13}$ has same meaning as defined above and $L_1$ is a leaving group.

Examples of compound of formula XIV includes, but are not limited to chloromethylphosphonic acid diethyl ester, bromomethylphosphonic acid diethyl ester, chloromethylphosphonic acid dimethyl ester, ((dimethoxyphosphoryl)oxy) methyl methanesulfonate, di-tert-butyl (chloromethyl) phosphate, dibenzyl (chloromethyl) phosphate and the like.

The above reactions can be carried out in presence of solvent and base. Solvent that can be used are selected from, but are not limited to, ethers, hydrocarbons, chlorinated hydrocarbons, polar aprotic solvents or mixtures thereof. Base that can be used are selected from, but are not limited to, DIPEA (diisopropylethyl amine), TEA (triethyl amine), pyridine, DBU, imidazole, N,N-dimethyl aniline, potassium carbonate, sodium carbonate, DMAP and the like or mixtures thereof.

In certain aspect, compound of formula VIII, wherein $R^{13}$ is hydrogen, may be prepared from compound of formula VIII, wherein $R^{13}$ is other than hydrogen, by reaction with suitable deprotecting reagents. Solvents used for this step include, but are not limited to, alcohols, esters, hydrocarbons, chlorinated hydrocarbons, polar solvents, polar aprotic solvents or mixtures thereof. The reaction can be carried out at any temperature ranging from about -10° C to about 160° C.

In yet another aspect, the present invention relates to process for the preparation of prodrugs of fulvestrant of formula XV comprising reacting compound of formula XVII with compound of formula XVI,

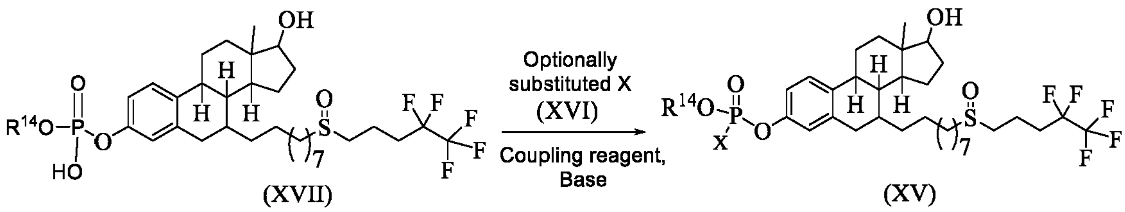


wherein $R^{14}$ and X have same meaning as defined above.

Examples of compound of formula (XVI) includes, but are not limited to substituted pyrrolidine.

The above reactions can be carried out in presence of solvent, base and coupling reagent. Solvent that can be used are selected from, but are not limited to, ethers, hydrocarbons, chlorinated hydrocarbons, polar aprotic solvents or

mixtures thereof. Base that can be used are selected from, but are not limited to, DIPEA (diisopropylethyl amine), TEA (triethyl amine), pyridine, DBU, imidazole, N,N-dimethyl aniline, potassium carbonate, sodium carbonate, DMAP and the like or mixtures thereof. Coupling reagent can be used is selected from, but are not limited to, O-benzotriazole-N,N,N',N'-tetramethyl-uronium-hexafluoro-phosphate (HBTU), 2-(7-Aza-1H-benzotriazole-1-yl)-1,1,3,3-tetramethyluroniumhexafluorophosphate (HATU), acid halide, 1-hydroxybenzotriazole (HOBt), 1-Hydroxy-7-aza-1H-benzotriazole (HOAt), diisopropylcarbodiimide (DIC), dicyclohexylcarbodiimide (DCC), N-(3-Dimethylaminopropyl)-N'-ethylcarbodiimide hydrochloride (EDC), 2-(6-Chloro-1H-benzotriazol-1-yl)-N,N,N',N'-tetramethylaminium hexafluoro-phosphate (HCTU), 1-[1-(Cyano-2-ethoxy-2-oxoethylideneaminooxy) dimethyl-aminomorpholino]-uroniumhexa-fluorophosphate (COMU) and the like.

In yet another aspect, the present invention relates to pharmaceutical composition comprising prodrugs of fulvestrant of formula I-A and enantiomers, diastereomers, racemates, pharmaceutically acceptable salts and solvates thereof and pharmaceutically acceptable excipients.

In yet another aspect, the present invention relates to pharmaceutical composition comprising prodrugs of fulvestrant of formula II and enantiomers, diastereomers, racemates, pharmaceutically acceptable salts and solvates thereof and pharmaceutically acceptable excipients.

In yet another aspect, the present invention relates to pharmaceutical composition comprising prodrugs of fulvestrant of formula I-A and enantiomers, diastereomers, racemates, pharmaceutically acceptable salts and solvates thereof

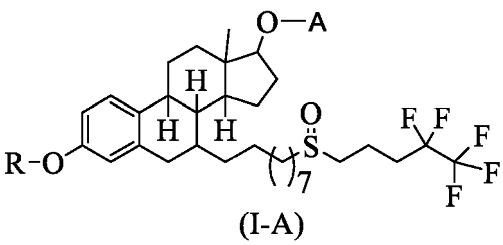

(I-A)

wherein

A is selected from hydrogen,

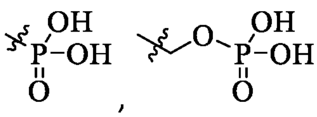

,

;

and

R is selected from group comprising of:

a)

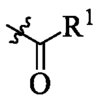

wherein $R^1$ is selected from group comprising of substituted aryl; optionally substituted alkyl, alkenyl, alkynyl, cycloalkyl, heteroaryl and $CR^2R^3$. $R^2$ and $R^3$ are taken together along with the carbon to which they are attached to form a three to seven membered saturated, partially unsaturated or unsaturated heterocyclic ring in which up to 4 carbon atoms are replaced by heteroatoms chosen from the group consisting of O, S or N and may optionally be substituted at a substitutable position with one or more $R^4$ radicals, wherein the $R^4$ radicals are independently selected at each occurrence from the group consisting of alkyl, cycloalkyl, heterocycloalkyl, aryl, heteroaryl, alkylcarbonyl, formyl, halo, alkylphosphate, phosphate, cyano, nitro, alkoxy, alkoxycarbonyl, alkenyl, alkynyl, alkylthio and arylcarbonyl;

b)

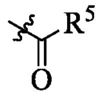

wherein $R^5$ is

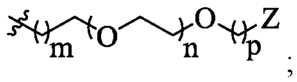

wherein m and p are independently selected from 0 to 5, n refers to degree of polymerization and is selected from 1 to 250 and Z is optionally substituted alkyl or cycloalkyl;

c)

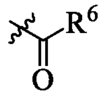

wherein $R^6$ is selected from $NH_2$, $NHR^7$ and $NR^8R^9$. $R^7$ is selected from optionally substituted alkyl, alkenyl, alkynyl, cycloalkyl, heterocycloalkyl, aryl and heteroaryl. $R^8$ and $R^9$ are taken together along with the nitrogen to which they are attached to form a three to seven membered saturated, partially unsaturated or unsaturated heterocyclic ring and may optionally be substituted at a substitutable position with one or more $R^{10}$ radicals, wherein the $R^{10}$ radicals are independently selected at each occurrence from the group consisting of alkyl, alkylcarbonyl, formyl, halo, haloalkyl, alkylphosphate, phosphate, oxo, cyano, nitro, amino, alkoxy, alkoxycarbonyl, carboxyalkyl, hydroxyalkyl, alkenyl, alkynyl, alkylthio, cycloalkyl, aryl, aralkyl, heterocycloalkyl, alkylthioalkyl, arylcarbonyl, aralkylcarbonyl and alkoxyalkyl;

d)

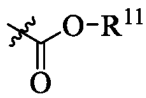

wherein $R^{11}$ is selected from group comprising of optionally substituted alkyl, alkenyl, alkynyl, cycloalkyl, heterocycloalkyl, aryl and heteroaryl;

e)

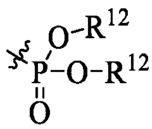

wherein each $R^{12}$ is selected independently from group comprising of hydrogen; optionally substituted alkyl, aryl, acyl and heteroaryl;
with a proviso that when A is hydrogen and one of $R^{12}$ substitution is selected from alkyl and aryl, then another $R^{12}$ substitution is hydrogen;

f)

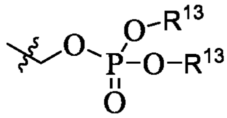

wherein each $R^{13}$ is selected independently from group comprising of hydrogen; optionally substituted alkyl, cycloalkyl, heterocycloalkyl, aryl and heteroaryl;

g)

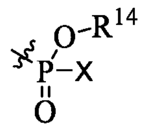

wherein $R^{14}$ is selected from group comprising of hydrogen; optionally substituted alkyl, aryl, acyl, heteroaryl; and X is optionally substituted heterocycloalkyl; and

h) hydrogen with proviso that when R is hydrogen A is not hydrogen;

and pharmaceutically acceptable excipients.

In yet another aspect, the present invention relates to pharmaceutical composition comprising prodrugs of fulvestrant of formula II and enantiomers, diastereomers, racemates, pharmaceutically acceptable salts and solvates thereof

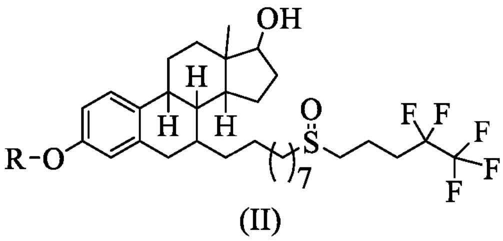

(II)

wherein R is selected from group comprising of:

a)

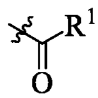

wherein $R^1$ is selected from group comprising of substituted aryl; optionally substituted alkyl, alkenyl, alkynyl, cycloalkyl, heteroaryl and $CR^2R^3$. $R^2$ and $R^3$ are taken together along with the carbon to which they are attached to form a three to seven membered saturated, partially unsaturated or unsaturated heterocyclic ring in which up to 4 carbon atoms are replaced by heteroatoms chosen from the group consisting of O, S or N and may optionally be substituted at a substitutable position with one or more $R^4$ radicals, wherein the $R^4$ radicals are independently selected at each occurrence from the group consisting of alkyl, cycloalkyl, heterocycloalkyl, aryl, heteroaryl, alkylcarbonyl, formyl, halo, alkylphosphate, phosphate, cyano, nitro, alkoxy, alkoxycarbonyl, alkenyl, alkynyl, alkylthio and arylcarbonyl;

b)

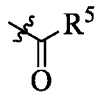

wherein $R^5$ is

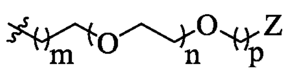

;

wherein m and p are independently selected from 0 to 5, n refers to degree of polymerization and is selected from 1 to 250 and Z is optionally substituted alkyl or cycloalkyl;

c)

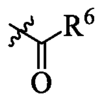

wherein $R^6$ is selected from $NH_2$, $NHR^7$ and $NR^8R^9$. $R^7$ is selected from optionally substituted alkyl, alkenyl, alkynyl, cycloalkyl, heterocycloalkyl, aryl and heteroaryl. $R^8$ and $R^9$ are taken together along with the nitrogen to which they are attached to form a three to seven membered saturated, partially unsaturated or unsaturated heterocyclic ring and may optionally be substituted at a substitutable position with one or more $R^{10}$ radicals, wherein the $R^{10}$ radicals are independently selected at each occurrence from the group consisting of alkyl, alkylcarbonyl, formyl, halo, haloalkyl, alkylphosphate, phosphate, oxo, cyano, nitro, amino, alkoxy, alkoxycarbonyl, carboxyalkyl, hydroxyalkyl, alkenyl, alkynyl, alkylthio, cycloalkyl, aryl, aralkyl, heterocycloalkyl, alkylthioalkyl, arylcarbonyl, aralkylcarbonyl and alkoxyalkyl;

d)

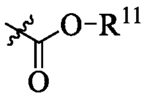

wherein $R^{11}$ is selected from group comprising of optionally substituted alkyl, alkenyl, alkynyl, cycloalkyl, heterocycloalkyl, aryl and heteroaryl;

e)

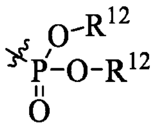

wherein each $R^{12}$ is selected independently from group comprising of hydrogen; optionally substituted alkyl, aryl, acyl and heteroaryl;
with a proviso that when one of $R^{12}$ is selected from alkyl and aryl, then another $R^{12}$ substitution is hydrogen;

f)

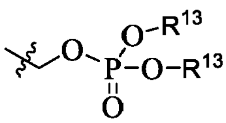

wherein $R^{13}$ is selected independently from group comprising of hydrogen; optionally substituted alkyl, cycloalkyl, heterocycloalkyl, aryl and heteroaryl; and

g)

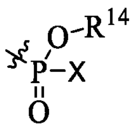

wherein $R^{14}$ is selected from group comprising of hydrogen; optionally substituted alkyl, aryl, acyl, heteroaryl; and X is optionally substituted heterocycloalkyl;
and pharmaceutically acceptable excipients.

The pharmaceutical compositions of the present disclosure can be in any form known to those of skill in the art. The pharmaceutical compositions of this invention may be administered orally, parenterally, by inhalation spray, topically,

rectally, nasally, buccally or via an implanted reservoir, preferably oral administration or administration by injection. For instance, in some embodiments the pharmaceutical compositions are in a form of a product for oral delivery, said product form being selected from a group consisting of a concentrate, dried powder, liquid, capsule, pellet, and pill. The pharmaceutical compositions disclosed herein may also further comprise carriers, binders, diluents, and excipients.

In an embodiment, the disclosure provides for a pharmaceutical composition in the form of fulvestrant prodrug for treatment of diseases and/or symptoms that are meant to be treated by the original drug molecule. The composition may comprise fulvestrant prodrug in an amount that is as therapeutically effective as or more therapeutically effective than the original drug.

In another embodiment, the invention provides a method of treating a benign or malignant diseases of the breast or reproductive tract, preferably treating breast cancer, comprising administration of a compound of formula I-A and enantiomers, diastereomers, racemates, pharmaceutically acceptable salts and solvates thereof,

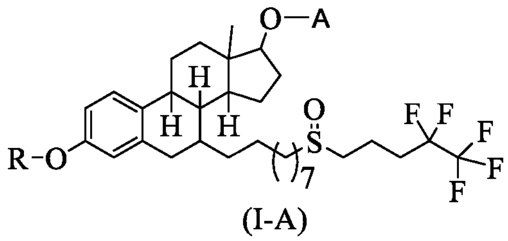

(I-A)

wherein

A is selected from hydrogen,

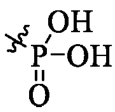 , 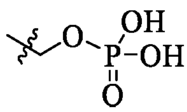 ;

and

R is selected from group comprising of:

a)

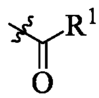

wherein $R^1$ is selected from group comprising of substituted aryl; optionally substituted alkyl, alkenyl, alkynyl, cycloalkyl, heteroaryl and $CR^2R^3$. $R^2$ and $R^3$ are taken together along with the carbon to which they are attached to form a three to seven membered saturated, partially unsaturated or unsaturated heterocyclic ring in which up to 4 carbon atoms are replaced by heteroatoms chosen from the group consisting of O, S or N and may optionally be substituted at a substitutable position with one or more $R^4$ radicals, wherein the $R^4$ radicals are independently selected at each occurrence from the group consisting of alkyl, cycloalkyl, heterocycloalkyl, aryl, heteroaryl, alkylcarbonyl, formyl, halo, alkylphosphate, phosphate, cyano, nitro, alkoxy, alkoxycarbonyl, alkenyl, alkynyl, alkylthio and arylcarbonyl;

b)

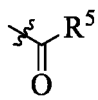

wherein $R^5$ is

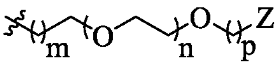 ;

wherein m and p are independently selected from 0 to 5, n refers to degree of polymerization and is selected from 1 to 250 and Z is optionally substituted alkyl or cycloalkyl;

c)

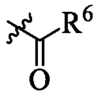

wherein $R^6$ is selected from $NH_2$, $NHR^7$ and $NR^8R^9$. $R^7$ is selected from optionally substituted alkyl, alkenyl, alkynyl, cycloalkyl, heterocycloalkyl, aryl and heteroaryl. $R^8$ and $R^9$ are taken together along with the nitrogen to which they are attached to form a three to seven membered saturated, partially unsaturated or unsaturated heterocyclic ring and may optionally be substituted at a substitutable position with one or more $R^{10}$ radicals, wherein the $R^{10}$ radicals are independently selected at each occurrence from the group consisting of alkyl, alkylcarbonyl, formyl, halo, haloalkyl, alkylphosphate, phosphate, oxo, cyano, nitro, amino, alkoxy, alkoxycarbonyl, carboxyalkyl, hydroxyalkyl, alkenyl, alkynyl, alkylthio, cycloalkyl, aryl, aralkyl, heterocycloalkyl, alkylthioalkyl, arylcarbonyl, aralkylcarbonyl and alkoxyalkyl;

d)

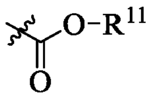

wherein $R^{11}$ is selected from group comprising of optionally substituted alkyl, alkenyl, alkynyl, cycloalkyl, heterocycloalkyl, aryl and heteroaryl;

e)

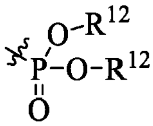

wherein each $R^{12}$ is selected independently from group comprising of hydrogen; optionally substituted alkyl, aryl, acyl and heteroaryl;
with a proviso that when A is hydrogen and one of $R^{12}$ substitution is selected from alkyl and aryl, then another $R^{12}$ substitution is hydrogen;

f)

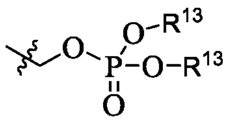

wherein each $R^{13}$ is selected independently from group comprising of hydrogen; optionally substituted alkyl, cycloalkyl, heterocycloalkyl, aryl and heteroaryl;

g)

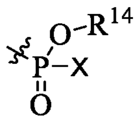

wherein $R^{14}$ is selected from group comprising of hydrogen; optionally substituted alkyl, aryl, acyl, heteroaryl; and X is optionally substituted heterocycloalkyl; and

h) hydrogen with proviso that when R is hydrogen A is not hydrogen.

In another embodiment, the invention provides a method of treating a benign or malignant diseases of the breast or reproductive tract, preferably treating breast cancer, comprising administration of a compound of formula II and enantiomers, diastereomers, racemates, pharmaceutically acceptable salts and solvates thereof,

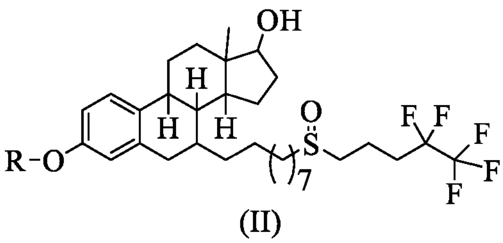

(II)

wherein R is selected from group comprising of:

a)

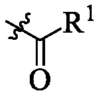

wherein $R^1$ is selected from group comprising of substituted aryl; optionally substituted alkyl, alkenyl, alkynyl, cycloalkyl, heteroaryl and $CR^2R^3$. $R^2$ and $R^3$ are taken together along with the carbon to which they are attached to form a three to seven membered saturated, partially unsaturated or unsaturated heterocyclic ring in which up to 4 carbon atoms are replaced by heteroatoms chosen from the group consisting of O, S or N and may optionally be substituted at a substitutable position with one or more $R^4$ radicals, wherein the $R^4$ radicals are independently selected at each occurrence from the group consisting of alkyl, cycloalkyl, heterocycloalkyl, aryl, heteroaryl, alkylcarbonyl, formyl, halo, alkylphosphate, phosphate, cyano, nitro, alkoxy, alkoxycarbonyl, alkenyl, alkynyl, alkylthio and arylcarbonyl;

b)

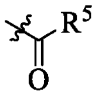

wherein $R^5$ is

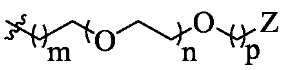

;

wherein m and p are independently selected from 0 to 5, n refers to degree of polymerization and is selected from 1 to 250 and Z is optionally substituted alkyl or cycloalkyl;

c)

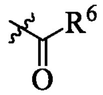

wherein $R^6$ is selected from $NH_2$, $NHR^7$ and $NR^8R^9$. $R^7$ is selected from optionally substituted alkyl, alkenyl, alkynyl, cycloalkyl, heterocycloalkyl, aryl and heteroaryl. $R^8$ and $R^9$ are taken together along with the nitrogen to which they are attached to form a three to seven membered saturated, partially unsaturated or unsaturated heterocyclic ring and may optionally be substituted at a substitutable position with one or more $R^{10}$ radicals, wherein the $R^{10}$ radicals are independently selected at each occurrence from the group consisting of alkyl, alkylcarbonyl, formyl, halo, haloalkyl, alkylphosphate, phosphate, oxo, cyano, nitro, amino, alkoxy, alkoxycarbonyl, carboxyalkyl, hydroxyalkyl, alkenyl, alkynyl, alkylthio, cycloalkyl, aryl, aralkyl, heterocycloalkyl, alkylthioalkyl, arylcarbonyl, aralkylcarbonyl and alkoxyalkyl;

d)

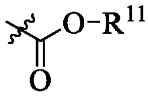

wherein $R^{11}$ is selected from group comprising of optionally substituted alkyl, alkenyl, alkynyl, cycloalkyl, heterocycloalkyl, aryl and heteroaryl;

e)

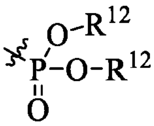

wherein each $R^{12}$ is selected independently from group comprising of hydrogen; optionally substituted alkyl, aryl, acyl and heteroaryl;
with a proviso that when one of $R^{12}$ is selected from alkyl and aryl, then another $R^{12}$ substitution is hydrogen;

f)

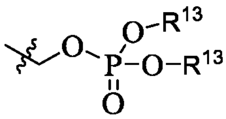

wherein each $R^{13}$ is selected independently from group comprising of hydrogen; optionally substituted alkyl, cycloalkyl, heterocycloalkyl, aryl and heteroaryl; and

g)

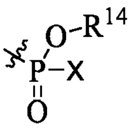

**wherein** $R^{14}$ is selected from group comprising of hydrogen; optionally substituted alkyl, aryl, acyl, heteroaryl; and X is optionally substituted heterocycloalkyl.

Also, in other aspects, the present disclosure relates to new fulvestrant prodrug compounds and any stereochemically isomeric form, hydrate, solvate or pharmaceutically acceptable salt thereof; pharmaceutical compositions comprising the new fulvestrant prodrug compounds, either alone or in combination with at least one additional therapeutic agent, with a pharmaceutically acceptable carrier; and uses of the new fulvestrant prodrug compounds, either alone or in combination with at least one additional therapeutic agent, in the treatment of diseases and/or symptoms meant to be

treated by the original drugs. The combination with an additional therapeutic agent may take the form of combining the new fulvestrant prodrug compounds with any known therapeutic agent.

In yet another aspect, the present invention relates to use of prodrugs of fulvestrant of formula II and enantiomers, diastereomers, racemates, pharmaceutically acceptable salts and solvates thereof in treatment of cancer. The disclosure also relates to use of fulvestrant prodrug according to Formula II for treatment of diseases and/or symptoms that are meant to be treated by the original drug molecule. Moreover, the presently taught fulvestrant prodrug allows facile oxidative cleavage to yield aryl-hydroxyl structure of the desired active drug fulvestrant, after administration. Thus, the presently disclosed prodrugs can be useful in treating any symptoms that are currently treated by fulvestrant, but at a significantly lower dosage, thereby making it unlikely to have any accidental overdose.

In yet another aspect, fulvestrant prodrug offers an oral administration formulation that has the potential to improve efficacy over existing fulvestrant therapy. It has been surprisingly found that the disclosed prodrug compounds exhibit superior solubility compared to the parent compound. The results of solubility studies are presented in table 1 and demonstrate a significant increase in solubility.

**Solubility test procedure I:**

A 50 mmol stock solution for each test compound was prepared using dimethyl sulfoxide. From this stock solution, a working solution of 1 mmol was prepared by diluting with dimethyl sulfoxide. The working solution of 1 mmol was then serially diluted using dimethyl sulfoxide. Then, 5-20 μl of stock solution is added to 1 ml of phosphate buffer solution of pH 7.4. The solution was then stirred for 4 h at 25° C in order to reach equilibrium. After this incubation period, the solution was filtered using 0.45 micron PVDF injector filters to remove the insoluble fraction of the compound and subsequently filtrate is taken for quantification using UV Detector. The concentration of test sample was calculated using the linearity/calibration curve.

Table 1: Kinetic solubility of test compounds and fulvestrant

| **Compound of Example No.** | Aqueous solubility (μM) at pH 7.4 | Increase in solubility compare to fulvestrant (Fold) |
|---|---|---|
| Fulvestrant | 0.005 | - |
| 1 | 0.04 | 8 |
| 3 | 3.3 | 669 |
| 4 | 0.5 | 97 |
| 5 | 1.4 | 295 |
| 6 | 2.7 | 562 |
| 7 | 0.2 | 35 |
| 8 | 17.1 | 3500 |
| 9 | 0.1 | 22 |
| 10 | 0.2 | 32 |

As from the results shown in Tables 1, the solubility of the compounds according to the present invention is significantly high. Solubility enhancement in the tested compound was observed in varying extents. In general, the increase in solubility observed for tested compounds as compared to the fulvestrant in the same aqueous medium ranged from about 8 to about 3500 fold.

In yet another aspect, fulvestrant prodrug offers an oral administration formulation that has the potential to improve efficacy over existing fulvestrant therapy. It has been surprisingly found that the disclosed prodrug compounds exhibit superior thermodynamic solubility compared to the parent compound. The results of solubility studies are presented in table 2 and demonstrate a significant increase in thermodynamic solubility.

**Solubility test procedure II:**

Stock solutions of the test compounds and fulvestrant were prepared in phosphate buffered saline (PBS) pH 7.4 and sodium acetate buffer pH 4.0, which was incubated for 24 h at room temperature with constant mixing at 100 rpm in the same tube. After incubation, the tubes were centrifuged for 20 min. at 10000 rpm. Later 200 μL of sample was taken and filtered. The filtrate was analysed by HPLC-UV against 0.0012 mg/ml, 0.037 mg/mL, 0.3 mg/ml, 0.1 mg/ml and 1mg/ml of

dimethylsulfoxide (DMSO) stock. Further the data analysis was carried out as per the following formula:

Solubility ($\mu$g/ml) = [Peak area of sample / Peak area of standard] $\times$ Concentration of standard % Aqueous solubility: [Test solubility ($\mu$g/ml)/ incubation concentration ($\mu$g/ml)] $\times$ 100

**Table** 2: Thermodynamic solubility of test compounds and fulvestrant

| Compound of Example No. | Thermodynamic solubility (mg/mL) pH 7.4 | Thermodynamic solubility (mg/mL) pH 4.0 | Test* compound concentration (mg/mL) |
|---|---|---|---|
| Fulvestrant | 0.0012 | 0.0029 | 1 |
| 4 | - | 0.79 | 1 |
| 9 | - | 0.70 | 1 |
| 29 | - | 0.75 | 1 |
| 20 | 1.22 | 0.74** | 2 |
| 21 | >5 | - | 5 |
| 15 | - | 0.63 | 1 |
| 19 | - | 1.05 | 2 |
| 44 | >15 | >1 ** | 15 |
| 45 | 1.25 | - | 2 |
| *Test compound concentration used for the assay.<br>** Test compound concentration used for the assay was 1mg/ml. | | | |

In general, the increase in solubility was observed for test compounds as compared to the fulvestrant in the same medium.

The following abbreviations are used in the specification:

EDCI: l-Ethyl-3-(3-dimethylaminopropyl)carbodiimide
HOBt: Hydroxybenzotriazole
DCM: Dichloromethane
$CHCl_3$: Chloroform
ACN: Acetonitrile
NMM: N-Methylmorpholine
DIEA: N, N-Diisopropylethylamine
TEA: Triethylamine
DBU: 1,8-Diazabicyclo[5.4.0]undec-7-ene
$K_2CO_3$: Potassium carbonate
NaOH: Sodium hydroxide
DMAP: 4-Dimethylaminopyridine
TBAB: Tetra-n-butylammonium bromide
DCC: N,N'-dicyclohexylcarbodiimide
TLC: Thin layer chromatography
LCMS: Liquid chromatography-mass spectrometry
HPLC: High performance liquid chromatography

The following examples are given for the purpose of illustrating the present invention and should not be considered as limiting the scope of the invention.

**Example-1: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6H-cyclopenta[a]phenanthren-3-yl dimethylglycinate**

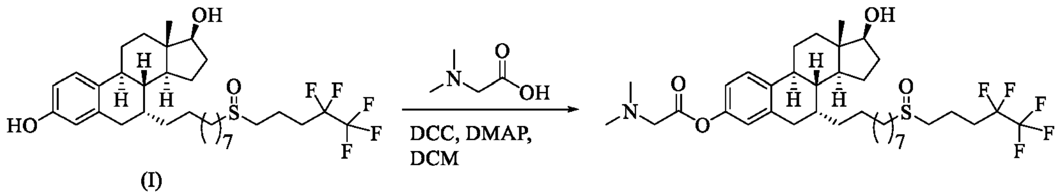


To a stirred solution of fulvestrant (0.150 g) and dimethylglycine (0.025 g) in dichloromethane (5 ml) was added 4-dimethylaminopyridine (0.006 g) and stirred at 0° C for 10 min. Then a solution of DCC (0.076 g) in dichloromethane (1 ml) was added dropwise to the reaction mixture and stirred at room temperature for 16 h. The reaction was monitored by TLC and LCMS. After completion of reaction, the reaction mixture was evaporated under vacuum to obtain crude material. The crude material was purified by preparative HPLC using mobile phase A) 5 mmol ammonium bicarbonate + 0.1% $NH_3$ in water and B) acetonitrile. The fractions were lyophilized to afford (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl dimethylglycinate (0.080 g, 46.77%) as an off white solid.

$^1$H-NMR (400 MHz, DMSO): δ 2.269 (s, 6H), 2.591-2.556 (t, 2H), 3.313 -3.280 (t, 2H), 3.735 (s, 2H), 3.822 (s, 2H), 5.310 (s, 2H), 7.413 (s, 1H) and 8.639-8.627 (s, 1H).

**Example-2: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6H-cyclopenta[a]phenanthren-3-yl 2-(piperidin-1-yl)acetate**

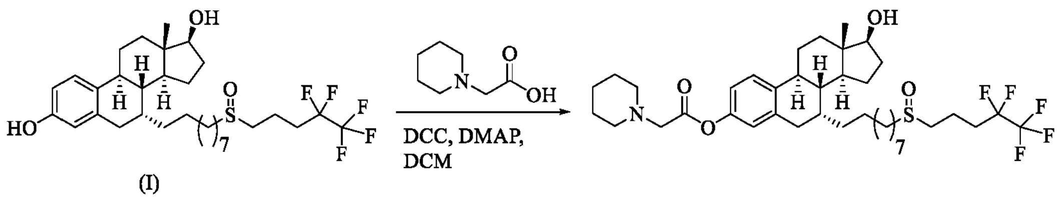


To a stirred solution of fulvestrant (0.2 g) in dichloromethane (4 ml) was added 2-(piperidin-1-yl)acetic acid (0.052 g) followed by DMAP (0.02 g) at 0° C and stirred for 5 min. Then a solution of DCC (0.102 g) in dichloromethane (1 ml) was added dropwise to the reaction mixture and stirred at 0° C to room temperature for 16 h. The reaction was monitored by TLC and LCMS. After completion of reaction, the reaction mixture was evaporated under vacuum to obtain crude material. The crude material was purified by preparative HPLC using A) 10 mmol ammonium acetate in water and B) acetonitrile. The product fraction was lyophilized to afford (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl 2-(piperidin-1-yl)acetate (0.170 g) as a colorless oil.

$^1$H-NMR (400 MHz, DMSO): δ 7.337 (d, 1H), 6.863-6.842 (m, 1H), 6 881 (s, 1H), 4.543 (s, 1H), 3.587-3.536 (m, 5H), 3.50 (s, 2H), 2.898-2.687 (m, 11H), 2.546-2.325 (m, 7H), 2.01-1.75 (m, 4H), 1.78-1.48 (m, 4H), 1.48-1.08 (m, 22H), 0.90 (dd, 2H) and 0.69 (s, 3H).

**Example-3: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6H-cyclopenta[a]phenanthren-3-yl 2-(piperidin-1-yl)acetate hydrochloride**

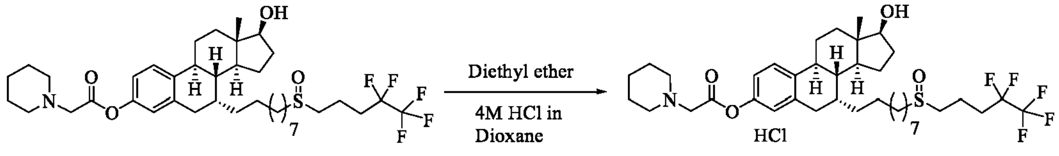


To a stirred solution of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl 2-(piperidin-1-yl)acetate (0.08 g) in diethyl ether (4 ml), 4M hydrochloric acid in dioxane (0.03 ml) was added dropwise at 0° C and stirred for 1 h. The reaction

mixture was allowed to warm at room temperature and evaporated under vacuum to provide diethyl (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl 2-(piperidin-1-yl)acetate hydrochloride (0.05 g, 59.65%) as a white solid.

$^1$H-NMR (400 MHz, DMSO): δ 10.315 (s, 1H-HCl salt), 7.337 (d, 1H), 6.863-6.842 (m, 1H), 6.881 (s, 1H), 4.543 (s, 1H), 3.587-3.536 (m, 5H), 3.50 (s, 2H), 2.898-2.687 (m, 11H), 2.546-2.325 (m, 7H), 2.01-1.75 (m, 4H), 1.78-1.48 (m, 4H), 1.48-1.08 (m, 22H), 0.90 (dd, 2H) and 0.69 (s, 3H).

**Example-4: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6H-cyclopenta[a]phenanthren-3-yl 4-methylpiperazine-1-carboxylate**

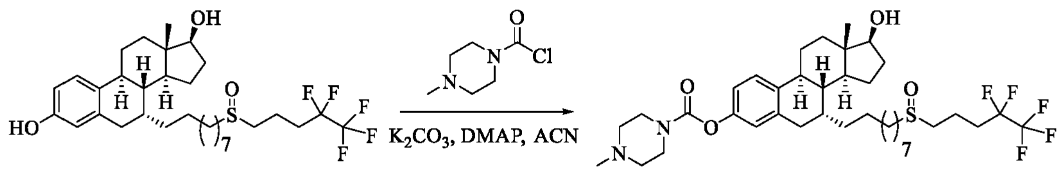


(I)

To a stirred solution of fulvestrant (0.100 g) in acetonitrile (5 ml) was added potassium carbonate (0.045 g) followed by 4-dimethylaminopyridine (0.002 g) and stirred at room temperature for 30 min. Then 4-methylpiperazine-1-carbonyl chloride (0.030 g) was added to the reaction mixture at 0° C and stirred for 2 h. Reaction mixture was continue stirred for 16 h at room temperature. The reaction was monitored by TLC and LCMS. After completion of reaction, the reaction mixture was diluted with water (20 ml) and extracted in dichloromethane (20 ml). The organic fractions were washed with brine (25 ml), dried over sodium sulphate and evaporated under vacuum to obtain crude material. The crude material was purified by preparative HPLC using A) 5 mmol ammonium bicarbonate + 0.1% $NH_3$ in water and B) acetonitrile. The fractions were lyophilized to provide (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl 4-methylpiperazine-1-carboxylate (0.038 g, 31.46%) as a white solid.

$^1$H-NMR (400 MHz, DMSO): δ 7.281 (d, 1H), 6.857-6.804 (m, 2H), 4.541 (d, 1H), 3.560-3.363 (m, 6H), 2.900-2.648 (m, 7H), 2.541 (s, 7H), 2.521-2.351 (m, 4H), 2.291 (s, 3H), 1.901-1.861 (m, 1H), 1.852-1.218 (m, 20 H), 0.90 (dd, 2H) and 0.69 (s, 3H).

**Example-5: Preparation of 4-(((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl)oxy)-4-oxobutanoic acid**

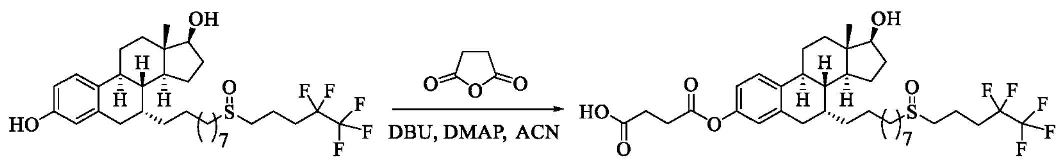


(I)

To a stirred solution of fulvestrant (0.200 g) and succinic anhydride (0.036 g) in acetonitrile (4 ml) was added 4-dimethylaminopyridine (0.008 g) followed by 1,8-diazabicyclo(5.4.0)undec-7-ene (0.076 g) at room temperature and stirred at 50° C for 16 h. The reaction was monitored by TLC and LCMS. After completion of reaction, the reaction mixture was evaporated under vacuum to obtain crude material. The crude material was purified by preparative HPLC using A) 0.1% formic acid in water and B) acetonitrile. The fractions were lyophilized to provide 4-(((7R,8R,9S, 13 S, 14S, 17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl)oxy)-4-oxobutanoic acid (0.126 g, 54.08%) as a white solid.

$^1$H-NMR (400 MHz, DMSO): δ 9.074 (s, 1H), 7.061 (d, 1H), 6.527-6.441 (m, 2H), 4.531 (d, 1H), 2.900-2.648 (m, 9H), 2.564- 2.491 (m, 4H), 2.481-2.109 (m, 7H), 2.011-1.952 (m, 3H), 1.901-1.102 (m, 26H), 0.90 (dd, 2H) and 0.69 (s, 3H).

**Example-6: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 2-hydroxybenzoate**

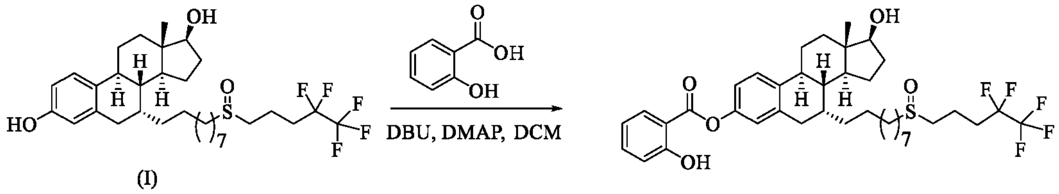


To a stirred solution of fulvestrant (0.15 g) in dichloromethane (4 ml) was added salicylic acid (0.038 g) followed by DMAP (0.015 g) at 0° C and stirred for 5 min. Then a solution of DCC (0.077 g) in dichloromethane (1 ml) was added dropwise to the reaction mixture. Reaction mixture was allowed to warm at room temperature and stirred for 16 h. The reaction was monitored by TLC and LCMS. After completion of reaction, the reaction mixture was evaporated under vacuum to obtain crude material. The crude material was purified by preparative HPLC using A) 10 mmol ammonium acetate in water and B) acetonitrile. The product fraction was lyophilized to afford (7R,8R,9S, 13S,14S, 17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl 2-hydroxybenzoate (0.034 g, 18.92%) as a white solid.

$^1$H-NMR (400 MHz, DMSO): δ 10.423 (s, 1H), 7.987(d, 1H), 7.601 (t, 1H), 7.395 (d, 1H), 7.071-6.995 (m, 3H), 4.554 (d, 1H), 3.587 (d, 2H), 3.587-3.536 (m, 5H), 3.50 (s, 2H), 2.873-2.660 (m, 6H), 2.453-2.338 (m, 4H), 1.995-1.883 (m, 4H), 1.863-1.831 (m, 2H), 1.734-1.601 (m, 5H), 1.525-1.262 (m, 16H), 0.90 (dd, 2H) and 0.69 (s, 3H).

**Example-7: Preparation of diethyl ((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl) phosphate**

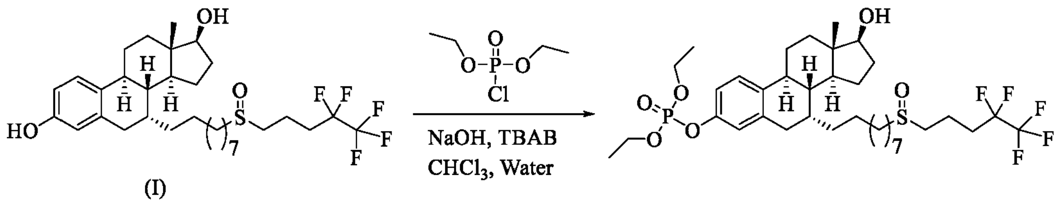


To a stirred solution of fulvestrant (0.2 g) in chloroform (5 ml) and water (1 ml) was added sodium hydroxide (0.132 g) followed by TBAB (0.128 g) at room temperature. Reaction mixture was vigorously stirred at room temperature for 30 min. Diethyl phosphorochloridate (0.074 g) was added to the reaction mixture and stirred at room temperature for 16 h. The reaction was monitored by TLC and LCMS. After completion of reaction, the reaction mixture was evaporated under vacuum to obtain crude material. The crude material was purified by preparative HPLC using mobile phase A) 10 mmol ammonium acetate in water and B) acetonitrile. The product fraction was lyophilized to provide diethyl ((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta [a] phenanthren-3-yl) phosphate (0.060 g, 24.50%) as a colorless semisolid.

$^1$H-NMR (400 MHz, DMSO): δ 7.312 (d, 1H), 6.945 (d, 1H), 6.890 (s, 1H), 4.545 (d, 1H), 4.185-4.111 (m, 4H), 3.560 (d, 1H), 3.587-3.536 (m, 5H), 3.50 (s, 2H), 2.880-2.784 (m, 2H), 2.759-2.645 (m, 4H), 2.459-2.279 (m, 4H), 1.996-1.885 (m, 4H), 1.872 (d, 1H), 1.771-1.514 (m, 2H), 1.734-1.601 (m, 5H), 1.525-1.262 (m, 18H), 0.90 (dd, 2H) and 0.69 (s, 3H)

**Example-8: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 2-(pyrrolidin-1-yl)acetate**

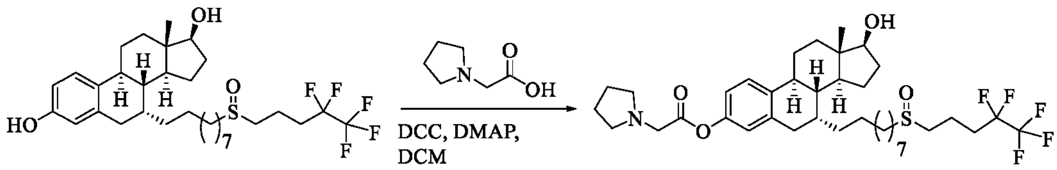


(I)

To a stirred solution of fulvestrant (0.2 g) in dichloromethane (8 ml) was added 2-(pyrrolidin-1-yl)acetic acid (0.05 g) followed by 4-dimethylaminopyridine (0.008 g). A solution of N,N'-dicyclohexylcarbodiimide (0.102 g) in dichloromethane (1 ml) was added dropwise to the reaction mixture at 0° C and then stirred at room temperature for 6 h. The reaction was monitored by TLC and LCMS. After completion of reaction, the reaction mixture was evaporated under vacuum to provide crude residue. The obtained crude residue was purified by flash chromatography using ethyl acetate and hexane. The fractions were evaporated under vacuum to obtain title product. The obtained product was further purified using preparative HPLC using mobile phase A) 5 mmol ammonium bicarbonate + 0.1% $NH_3$ in water and B) acetonitrile. The fractions were lyophilized to afford (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl 2-(pyrrolidin-1-yl)acetate (0.055 g, 23.24%) as a white solid.

$^1$H-NMR (400 MHz, DMSO): δ 7.336 (d, 1H), 6.865 (d, 1H), 6.813 (s, 1H), 4.544 (d, 1H), 3.575 (s, 3H), 2.893-2.633 (m, 10H), 2.477-2.269 (m, 5H), 1.954-1.492 (m, 13H), 1.392-1.205 (m, 18H), 0.901 (m, 2H) and 0.695 (s, 3H)

**Example-9: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl [1,4'-bipiperidine]-1'-carboxylate**

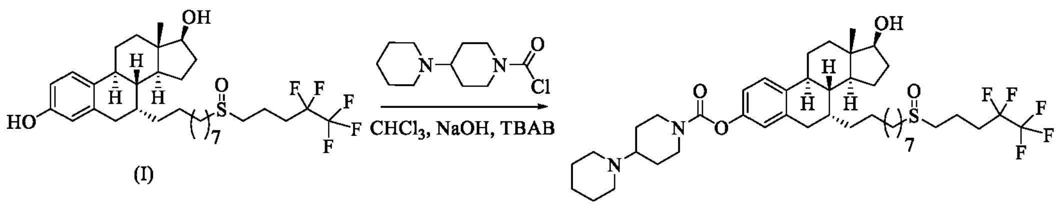


(I)

To a stirred solution of fulvestrant (0.2 g) in chloroform (6 ml) and water (1 ml), sodium hydroxide (0.132 g) and tetra-n-butylammonium bromide (0.128 g) was added at room temperature and vigorously stirred at room temperature for 30 min. 4-Piperidinopiperidine-1-carbonyl chloride (0.097 g) was added to the reaction mixture and stirred at room temperature for 16 h. The reaction was monitored by TLC and LCMS. After completion of reaction, the reaction mixture was evaporated under vacuum to obtain crude material. The crude material was purified by preparative HPLC using mobile phase A) 5 mmol ammonium bicarbonate + 0.1% $NH_3$ in water and B) acetonitrile. Product fraction was lyophilized to afford (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl [1,4'-bipiperidine]-1'-carboxylate (0.019 g, 7.20%) as a white solid.

$^1$H-NMR (400 MHz, DMSO): δ 7.272 (d, 1H), 6.821 (d, 1H), 6.781 (s, 1H), 4.559 (s, 1H), 4.131 (s, 1H), 4.047 (s, 1H), 3.558 (m, 2H), 3.501-3.486 (m, 5H), 2.961 (s, 2H), 2.892-2.623 (m, 10H), 2.446-2.279 (m, 12H), 1.996-1.885 (m, 4H), 1.835-1.746 (m, 5H), 1.771-1.514 (m, 4H), 1.734-1.601 (m, 8H), 1.525-1.262 (m, 24H), 0.901 (dd, 2H) and 0.684 (s, 3H).

**Example-10: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 2-morpholinoacetate**

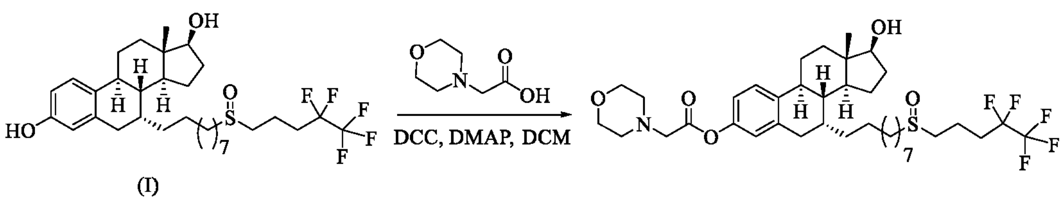


(I)

To a stirred solution of 2-morpholinoacetic acid (0.03 g) in dichloromethane (5 ml), fulvestrant (0.125 g) followed by DMAP (0.005 g) was added at 0° C and stirred for 5 mins. Then a solution of N,N'-dicyclohexylcarbodiimide (0.051 g) in dichloromethane (1 ml) was added dropwise to the reaction mixture and stirred at 0° C for 2 h. The reaction was monitored by TLC and LCMS. After completion of reaction, the reaction mixture was evaporated under vacuum to obtain crude residue. The obtained crude residue was purified by flash chromatography using silica gel and ethyl acetate in hexane. The obtained product was further purified by preparative HPLC using mobile phase A) 5 mmol ammonium bicarbonate + 0.1% $NH_3$ in water and B) acetonitrile. The fractions were lyophilized to afford (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl 2-morpholinoacetate (0.050 g, 33.07%) as a white solid.

$^1$H-NMR (400 MHz, DMSO): δ 7.342 (d, 1H), 6.871 (d, 1H), 6.823 (s, 1H), 4.549 (d, 1H), 3.681-3.544 (m, 5H), 3.503 (s, 2H), 2.895-2.867 (m, 2H), 2.841-2.726 (m, 4H), 2.596 (t, 4H), 2.444-2.157 (m, 4H), 2.019-1.756 (m, 5H), 1.787-1.487 (m, 4H), 1.448-1.087 (m, 18H), 0.903 (d, 2H) and 0.669 (s, 3H).

**Example-11: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl piperidin-4-yl carbonate**

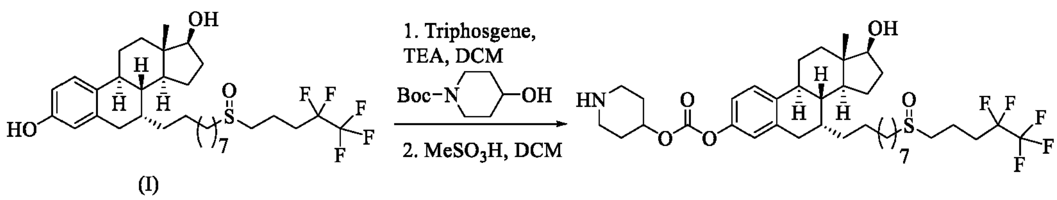


(I)

Step-1: To a stirred mixture of triphosgene (0.036g) in dichloromethane (6 ml), a solution of tert-butyl 4-hydroxypiperidine-1-carboxylate (0.052 g) and triethylamine (0.045 ml) in dichloromethane (2 ml) was added dropwise and stirred at 0° C for 30 mins. A solution of fulvestrant (0.150 g) in dichloromethane (2 ml) was added to the reaction mixture at 0° C. Reaction mixture was allowed to warm at room temperature and stirred for 3 h. The reaction was monitored by TLC and LCMS. After completion of reaction, the reaction mixture was evaporated under vacuum to provide tert-butyl 4-(((((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl)oxy)carbonyl)oxy)piperidine-1-carboxylate (0.280 g). The obtained material was used for next step without purification.

Step-2: To a stirred solution of tert-butyl 4-(((((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl)oxy)carbonyl)oxy)piperidine-1-carboxylate (0.280 g) in dichloromethane (2 ml), methanesulfonic acid (0.023 g) was added at 0° C and stirred for 2 h. The reaction was monitored by TLC and LCMS. After completion of reaction, the reaction mixture was evaporated under vacuum to provide crude material. The obtained crude material was purified using preparative HPLC using mobile phase A) 0.1% formic acid in water and B) acetonitrile. The fractions were lyophilized to provide (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl piperidin-4-yl carbonate (0.010 g, 5.36%) as a white solid.

$^1$H-NMR (400 MHz, DMSO): δ 8.399 (s, 0.5H-formic salt), 7.345 (d, 1H), 6.945(s, 1H), 6.908 (s, 1H), 4.701 (s, 1H), 4.536 (s, 1H), 3.525 (q, 2H), 2.998-2.981 (m, 4H), 2.752-2.714 (m, 5H), 2.664-2.599 (m, 4H), 2.398-2.385 (m, 4H), 1.927-1.888 (m, 5H), 1.827-1.669 (m, 4H), 1.604-1.550 (m, 7H), 1.358-1.257 (m, 21H), 0.881 (s, 1H) and 0.677 (s, 3H).

**Example-12: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl (1-methylpiperidin-4-yl) carbonate**

Step-1: Preparation of 1-methylpiperidin-4-yl (4-nitrophenyl) carbonate

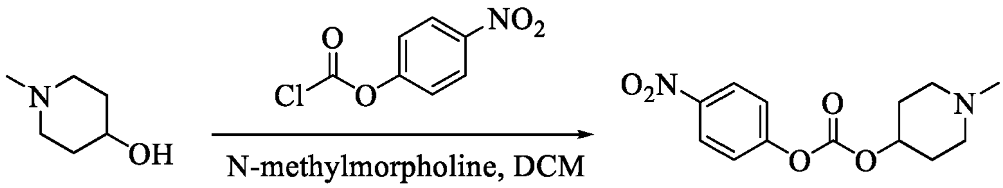


To a stirred solution of 1-methylpiperidin-4-ol (0.5 g) in dichloromethane (8 ml), N-methylmorpholine (0.48 g) followed by portionwise 4-nitrophenyl chloroformate (0.96 g) was added at 0° C. Reaction mixture was warmed to room temperature and stirred vigorously for 3 h. The progress of reaction was monitored by TLC. After completion of reaction, the reaction mixture was filtered and washed with dichloromethane (2x4 ml) to provide title compound as a cream solid (0.850 g, 69.86%).

$^1$H-NMR (400 MHz, DMSO): δ 8.356 (d, 2H), 7.612 (d, 2H), 5.101 (m, 1H), 2.724-2.682 (m, 4H), 1.938-1.894 (m, 2H) and 1.741-1.612 (m, 2H).

Step-2: Preparation of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl (1-methylpiperidin-4-yl) carbonate

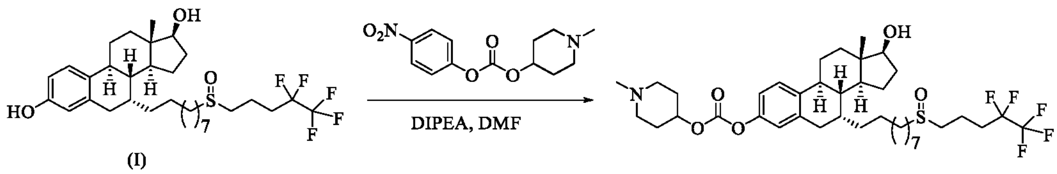


(I)

To a stirred solution of fulvestrant (0.05 g) in dimethylformamide (2.5 ml), N, N-diisopropylethylamine (0.053 g) followed by 1-methylpiperidin-4-yl (4-nitrophenyl) carbonate (0.046 g) was added at room temperature. The reaction mixture was stirred at 70° C for 36 h. The progress of reaction was monitored by TLC and LCMS. After completion of reaction, reaction mixture was poured into water (50 ml). Reaction mixture was extracted in ethyl acetate (3x30ml). Combined organic layer was washed with brine solution (3x50ml) and dried over anhydrous $Na_2SO_4$. Organic layer was concentrated to get a crude residue. The crude compound was purified by preparative HPLC using mobile phase (A) 5 mM ammonium bicarbonate + 0.1% $NH_3$ in water and (B) 100% acetonitrile. The fractions were lyophilized to afford (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl (1-methylpiperidin-4-yl) carbonate as a colourless semisolid (0.028 g, 9.09%).

$^1$H-NMR (400 MHz, DMSO): δ 7.234 (d, 1H), 6.954 (d, 1H), 6.912 (s, 1H), 4.639 (s, 1H), 4.543 (s, 1H), 3.561 (m, 1H), 2.876-2.841 (s, 2H), 2.743-2.623 (m, 4H), 2.407-2.229 (m, 4H), 2.217 (s, 4H), 1.919 (t, 5H), 1.823 (d, 2H), 1.651-1562 (m, 9H), 1.364-1.242 (m, 18H), 0.905 (dd, 1H) and 0.684 (s, 3H).

**Example-13: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 3-morpholinopropanoate**

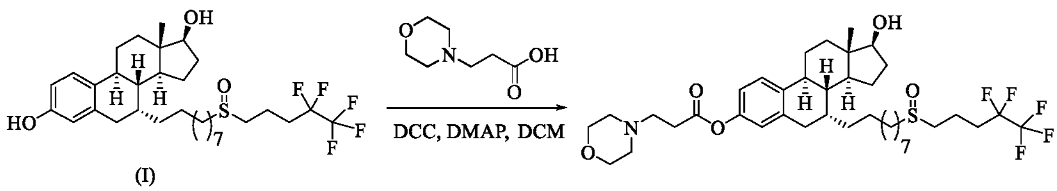


(I)

To a stirred solution of fulvestrant (0.200 g) and 3-morpholinopropanoic acid (0.062 g) in dichloromethane (8 ml), DMAP (0.008 g) followed by a solution of DCC (0.102 g) in dichloromethane (1 ml) was added at 0° C. Reaction mixture was allowed to warm at room temperature and stirred for 6 h. The progress of reaction was monitored by TLC & LCMS. After completion of reaction, the reaction mixture was evaporated under vacuum to obtain crude residue. The residue was

purified by flash chromatography and the desired product eluted in 90-100% ethyl acetate in hexane. The obtained product was further purified by preparative HPLC using mobile phase (A) 10 mM ammonium acetate in water and (B) 100% acetonitrile. The fractions were lyophilized to afford (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl 3-morpholinopropanoate (0.065 g, 26.37%) as a white solid.

$^{1}$H-NMR (400 MHz, DMSO): δ 7.337 (d, 1H), 6.856 (d, 1H), 6.801 (s, 1H) 4.545 (d, 1H), 3.596 (t, 4H), 3.523-3.501 (m, 4H), 2.892-2.624 (m, 10H), 2.42-2.296 (m, 9H), 1.953-1.816 (m, 4H), 1.717-1.515 (m, 5H), 1.372-1.104 (m, 20H), 0.914 (d, 2H) and 0.697 (s, 3H).

**Example-14: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 2-(2-(2-methoxyethoxy)ethoxy) acetate**

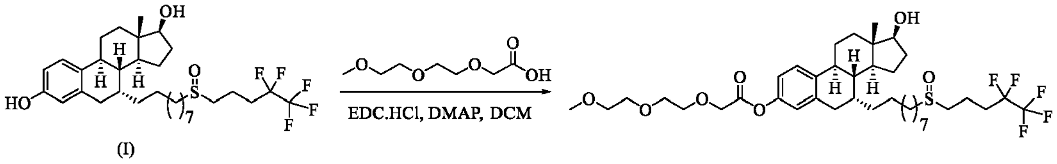


To a stirred solution of fulvestrant (0.2 g) and 2-(2-(2-methoxyethoxy) ethoxy)acetic acid (0.082 g) in dichloromethane (5 ml), DMAP (0.008 g) followed by EDCI HCl (0.094 g) was added at 0° C. Reaction mixture was allowed to warm at room temperature and stirred for 16 h. The progress of reaction was monitored by TLC and LCMS. After completion of reaction, the reaction mixture was evaporated under vacuum to provide crude residue. The residue was purified by flash chromatography using ethyl acetate and hexane as a mobile phase. The fractions were evaporated under vacuum to provide residue. The obtained residue was further purified by preparative HPLC using mobile phase (A) 0.1% formic acid in water and (B) 100% acetonitrile. The fractions were lyophilized to afford (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H cyclopenta[a]phenanthren-3-yl 2-(2-(2-methoxyethoxy)ethoxy)acetate as a pale yellow liquid (0.025 g, 9.89%).

$^{1}$H-NMR (400 MHz, DMSO): 7.334 (d, 1H), 6.898 (d, 1H), 6.852 (s, 1H), 4.542 (d, 1H), 4.401 (s, 2H), 3.687-3.544 (m, 7H), 3.465-3.445 (m, 2H), 3.259 (s, 3H), 2.891-2.823 (m, 2H), 2.781-2.716 (m, 4H), 2.441-2.271 (m, 4H), 2.011-1.891 (m, 5H), 1.842 (d, 2H), 1.781-1.482 (m, 4H), 1.480-1.081 (m, 18H), 0.90 (d, 1H) and 0.69 (s, 3H).

**Example-15: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6H-cyclopenta[a]phenanthren-3-yl 2-(1-methylpiperidin-4-yl)acetate**

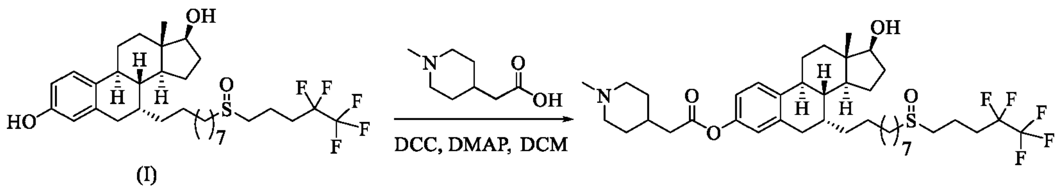


To a stirred solution of fulvestrant (0.2 g) and 2-(1-methylpiperidin-4-yl)acetic acid (0.062 g) in dichloromethane (5 ml) was added DMAP (0.008 g) followed by DCC (0.099 g) at 0° C. Reaction mixture was warmed to room temperature and stirred for 12 h. The progress of reaction was monitored by TLC and LCMS. After completion of reaction, the reaction mixture was evaporated under vacuum to obtain crude residue. The residue was purified by flash chromatography and the desired product eluted in 4-5% methanol in dichloromethane. The obtained product was further purified by preparative HPLC using mobile phase (A) 0.1% formic acid in water and (B) 100% acetonitrile. The fractions were lyophilized to afford (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl 2-(1-methylpiperidin-4-yl)acetate (0.040 g, 16.27%) as a white solid.

$^{1}$H-NMR (400 MHz, DMSO): δ 7.321 (d, 1H), 6.835 (d, 1H), 6.81 (s, 1H), 4.542 (d, 1H), 3.564 (s, 1H), 2.89-2.86 (m, 4H), 2.84-2.72 (m, 4H), 2.59 (t, 2H), 2.44-2.15 (m, 4H), 2.01-1.75 (m, 2H), 1.78-1.48 (m, 11H), 1.48-1.08 (m, 18H), 0.90 (d,

1H) and 0.69 (s, 3H).

**Example-16: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 2-(4-methylpiperazin-1-yl)acetate**

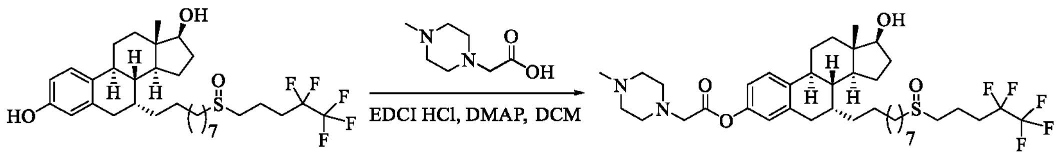


(I)

To a stirred solution of fulvestrant (0.200 g) and 2-(4-methylpiperazin-1-yl)acetic acid (0.062 g) in dichloromethane (5 ml), DMAP (0.008 g) followed by EDCI HCl (0.094 g) was added at 0° C. Reaction mixture was warmed to room temperature and stirred for 16 h. The progress of reaction was monitored by TLC and LCMS. After completion of reaction, the reaction mixture was evaporated under vacuum to obtain crude residue. The residue was purified by flash chromatography and the desired product eluted in 4-5% methanol in dichloromethane. The obtained product was further purified by preparative HPLC using mobile phase (A) 0.1% formic acid in water and (B) 100% acetonitrile. The fractions were lyophilized to afford (7R,8R,9S, 13S,14S, 17S)-17-hydroxy-13-methyl-7-(9-((4,4,5,5,5-pentafluoropentyl)sulfinyl) nonyl)-7,8,9,11,12,13,14,15,16,17-decahydro-6*H*-cyclopenta[a]phenanthren-3-yl 2-(4-methylpiperazin-1-yl)acetate (0.060 g, 24.37%) as a white solid.

HPLC purity: 100%

$^1$H-NMR (400 MHz, DMSO): δ 7.321 (d, 1H), 6.856 (d, 1H), 6.813 (s, 1H), 4.541 (d, 1H), 3.563 (d, 1H), 3.487-3.423 (m, 2H), 2.876-2.219 (m, 19H), 2.01-1.75 (m, 6H), 1.78-1.48 (m, 4H), 1.48-1.08 (m, 18H), 0.90 (d, 2H) and 0.69 (s, 3H).

**Example-17: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 3-(dimethylamino)propanoate**

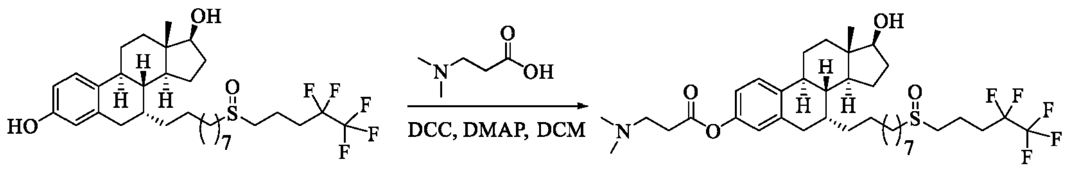


(I)

To a stirred solution of fulvestrant (0.3 g) and 3-(dimethylamino)propanoic acid (0.069 g) in dichloromethane (10 ml), DMAP (0.012 g) followed by DCC (0.166 g) was added at 0° C. Reaction mixture was warmed to room temperature and stirred for 12 h. The progress of reaction was monitored by TLC and LCMS. After completion of reaction, the reaction mixture was evaporated under vacuum to provide crude residue. The residue was purified by flash chromatography and the desired product eluted in 4-5% methanol in dichloromethane. The obtained product was further purified by preparative HPLC using mobile phase (A) 0.1% formic acid in water and (B) 100% acetonitrile. The fractions were lyophilized to afford (7R,8R,9S, 13S,14S, 17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl 3-(dimethylamino)propanoate (0.065 g, 18.62%) as a gummy solid.

$^1$H-NMR (400 MHz, DMSO): δ 7.321 (d, 1H), 6.843-6.542 (m, 2H), 4.542 (d, 1H), 3.551 (t, 1H), 2.896-2.726 (m, 6H), 2.691-2.581 (m, 5H), 2.441-2.261 (m, 4H), 2.194 (s, 6H), 1.951-1.895 (m, 4H), 1.841-1.491 (m, 6H), 1.448-1.081 (m, 19H), 0.90 (d, 2H) and 0.69 (s, 3H).

**Example-18: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 2-aminobenzoate**

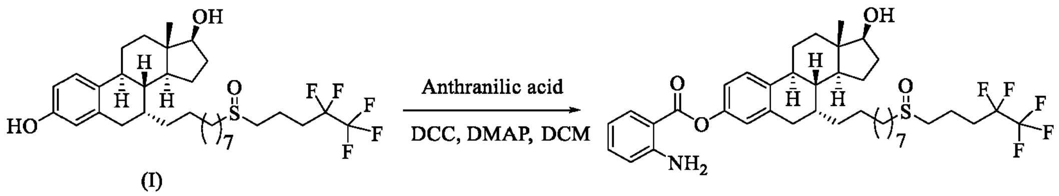


(I)

To a stirred solution of fulvestrant (0.25 g) in dichloromethane (5 ml), anthranilic acid (0.067 g) followed by DMAP (0.01 g) was added at room temperature. A solution of DCC (0.127 g) in dichloromethane (1 ml) was added to the reaction mixture. The reaction mixture was vigorously stirred at room temperature for 16 h. The progress of reaction was monitored by TLC and LCMS. After completion of reaction, the reaction mixture was evaporated under vacuum to obtain crude residue. The residue was purified by flash chromatography and the desired product eluted in 56% ethyl acetate in hexane. The obtained product was further purified by preparative HPLC using mobile phase (A) 0.1% formic acid in water and (B) 0.1% formic acid in acetonitrile. The fractions were lyophilized to afford (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl 2-aminobenzoate (0.025 g, 8.36%) as a white solid.

$^1$H-NMR (400 MHz, DMSO): δ 7.883 (d, 1H), 7.361-7.312 (m, 2H), 6.945 (d, 1H), 6.903 (s, 1H), 6.823 (d, 1H), 6.726 (s, 2H), 6.597 (t, 1H), 4.535 (s, 1H), 2.875-2.614 (m, 9H), 2.401-2.345 (m, 5H), 1.923-1.815 (m, 5H), 1.772 (s, 1H), 1.623-1.523 (m, 5H), 1.376-1. 248 (m, 22H), 0.914 (dd, 1H) and 0.696 (s, 3H).

**Example-19: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 4-methyl-1,4-diazepane-1-carboxylate**

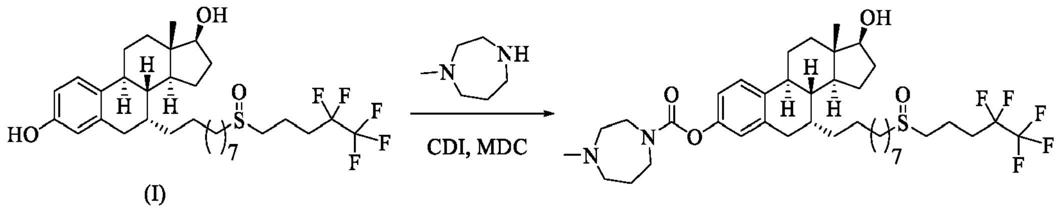


(I)

To a stirred solution of fulvestrant (0.3 g) and 1-methyl-1,4-diazepane (0.067 g) in dichloromethane (5 ml), 1,1'-carbonyldiimidazole (0.120 g) was added at 0° C. Reaction mixture was warmed to room temperature and stirred for 48 h. The progress of reaction was monitored by TLC and LCMS. After completion of reaction, the reaction mixture was evaporated under vacuum to provide crude residue. The residue was purified by flash chromatography and the desired product eluted in 7-8% methanol in dichloromethane. The obtained product was further purified by preparative HPLC using mobile phase (A) 0.1% formic acid in water and (B) 100% acetonitrile. The fractions were lyophilized to afford (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl 4-methyl-1,4-diazepane-1-carboxylate (0.035 g, 9.48%) as a white solid.

$^1$H-NMR (400 MHz, DMSO): δ 7.267 (d, 1H), 6.831(d, 1H), 6.783 (s, 1H), 4.523 (d, 1H), 3.58 (s, 3H), 3.465 (m, 3H), 2.834-2.501 (m, 10H), 2.391-2.283 (m, 4H), 1.923-1.834 (m, 6H), 1.693-1.499 (m, 4H), 1.357-1.341 (m, 23H), 0.90 (d, 1H) and 0.69 (s, 3H).

**Example-20: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl dihydrogen phosphate**

Step-1: Preparation of dibenzyl ((7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl) phosphate

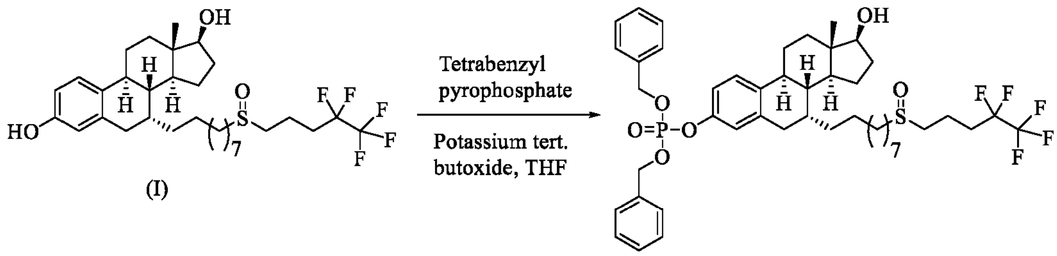


To a stirred solution of fulvestrant (0.25 g) in tetrahydrofuran (10 ml), potassium tert. Butoxide (0.05 g) was added at room temperature. Reaction mixture was stirred at 70° C for 5 min. Tetrabenzyl pyrophosphate (0.244 g) was added to reaction mixture at 70° C. Reaction mixture was vigorously stirred at 70° C for 18 h. The progress of reaction was monitored by TLC and LCMS. After completion of reaction, reaction mixture was cooled to room temperature and poured into water (50 ml). Reaction mixture was extracted in ethyl acetate (3x25 ml). Combined organic layer was washed with saturated brine solution (50 ml) and dried over anhydrous $Na_2SO_4$. Organic layer concentrated to provide crude residue. The crude residue was purified by flash chromatography to afford the title compound (0.225 g, 62.99%).

$^1$H-NMR (400 MHz, DMSO): δ 7.384 (s, 10H), 7.291 (d, 1H), 6.921 (d, 1H), 6.827 (s, 1H), 5.168 (s, 2H), 5.148 (s, 2H), 4.559 (s, 1H), 4.534 (s, 1H), 3.558 (m, 2H), 2.891-2.871 (s, 2H), 2.832-2.623 (m, 4H), 2.456-2.249 (m, 4H), 1.956-1.801 (m, 5H), 1.694 (s, 1H), 1.632-1.492 (m, 4H), 1.525- 1.102 (m, 22H), 0.865 (dd, 1H) and 0.684 (s, 3H).

Step-2: Preparation of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl dihydrogen phosphate

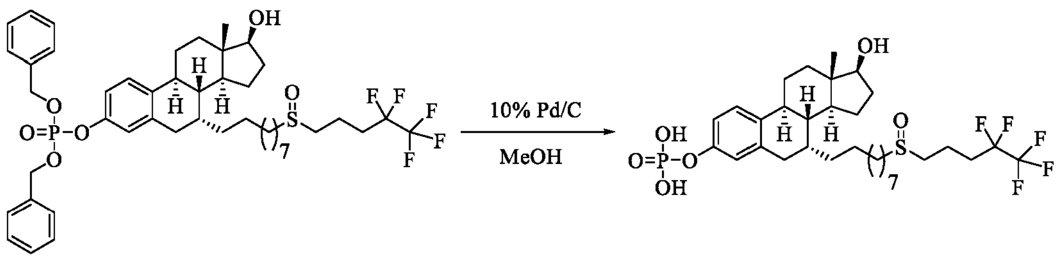


To a stirred solution of dibenzyl ((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl) phosphate (0.2 g) in methanol (15 ml), 10% Pd/C (with 50% water) was added at room temperature. Reaction mixture was stirred at room temperature for 3 h with continuous hydrogen gas purging. The progress of reaction was monitored by TLC. After completion of reaction, the reaction mixture was filtered through celite bed and washed with methanol (2x5 ml). Filtrate was evaporated under vacuum to provide crude residue. The residue was purified by trituration with diethyl ether to provide (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl dihydrogen phosphate (0.02 g, 12.62%).

$^1$H-NMR (400 MHz, DMSO): δ 7.165 (d, 1H), 6.911 (d, 1H), 6.857 (s, 1H), 4.559 (s, 1H), 3.558-3.532 (m, 2H), 2.881-2.591 (m, 6H), 2.406-2.249 (m, 6H), 1.956-1.901 (m, 3H), 1.823-1.501 (m, 8H), 1.525-1.102 (m, 22H), 0.905 (dd, 1H) and 0.684 (s, 3H).

**Example-21: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl dihydrogen phosphate disodium**

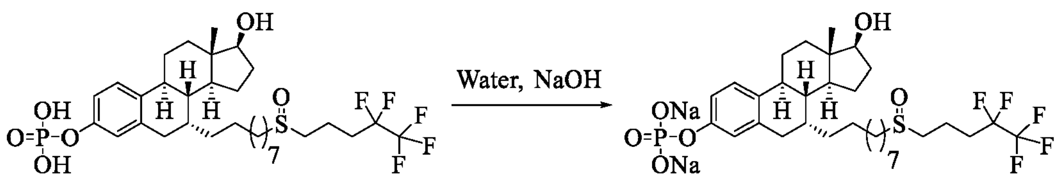

To a stirred solution of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl dihydrogen phosphate (0.05 g) in water (10 ml), 1M NaOH solution in water (0.15 ml) was added at room temperature. The reaction mixture was stirred at room temperature for 1 h. After completion of reaction, the reaction mixture was filtered and lyophilized to provide (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl dihydrogen phosphate disodium (0.035 g) as an off white sticky solid.

HPLC purity: 93.06%

$^1$H-NMR (400 MHz, DMSO): δ 7.05-6.80 (m, 3H), 4.47 (s, 1H), 3.52 (s, 1H), 2.83-2.63 (m, 5H), 2.36-2.19 (m, 4H), 1.89-1.77 (m, 4H), 1.59-1.23 (m, 25H), 0.80 (m, 1H) and 0.67 (s, 3H).

**Example-22: Preparation of di-tert-butyl ((((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl)oxy) methyl) phosphate**

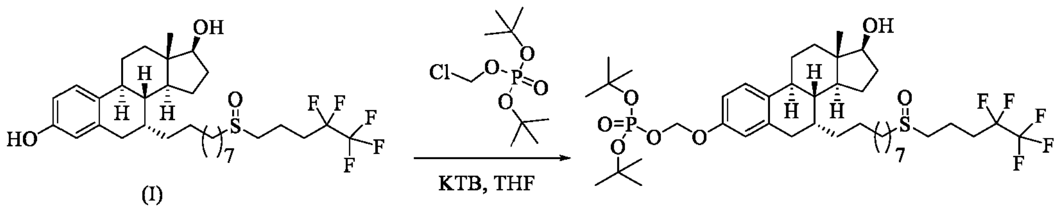


To a stirred solution of fulvestrant (0.4 g) in tetrahydrofuran (8 ml), potassium tert-butoxide (0.081 g) was added at room temperature. The reaction mixture was heated at 70° C and stirred for 15 min. To this was added a solution of di-tert-butyl (chloromethyl) phosphate (0.19 g) in tetrahydrofuran (1 ml). The reaction mixture was further stirred at 70° C for 7 h. The progress of reaction was monitored by TLC and LCMS. After completion of reaction, the reaction mixture was diluted with chilled water (70 ml) and extracted in ethyl acetate (3x25 ml). The combined organic layer was washed with brine (2x20 ml), dried over sodium sulphate and evaporated under vacuum at 35-40° C to obtain crude material. The obtained product was purified by preparative HPLC using mobile phase (A) 0.1% formic acid in water and (B) 100% acetonitrile. The fractions were lyophilized to afford di-tert-butyl ((((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl)oxy)methyl) phosphate (0.025 g, 26.14%) as a colourless gum.

$^1$H-NMR (400 MHz, DMSO): δ 7.245 (d, 1H), 6.801 (d, 1H), 6.764 (s, 1H), 5.434 (s, 2H), 4.542 (d, 1H), 3.554(s, 1H), 2.846-2.812 (m, 2H), 2.751-2.716 (m, 5H), 2.331-2.201 (m, 6H), 2.011-1.891 (m, 6H), 1.842 (d, 2H), 1.781-1.482 (m, 4H), 1.480-1.281 (m, 33H), 0.901 (d, 1H) and 0.692 (s, 3H).

**Example-23: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl L-valinate hydrochloride**

Step-1: Preparation of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl (tert-butoxycarbonyl)-L-valinate

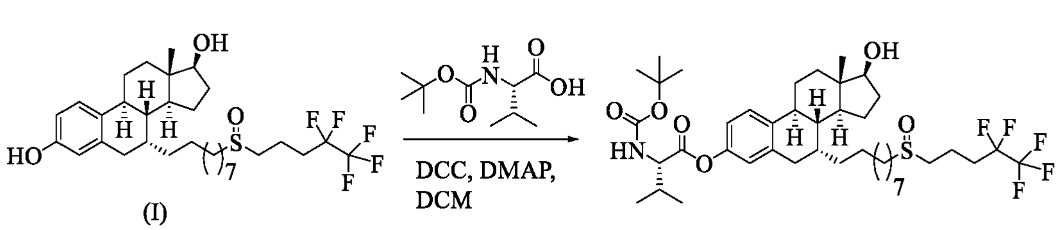


To a stirred solution of fulvestrant (0.150 g) in dichloromethane (6 ml) was added DMAP (0.009 g) followed by DCC (0.102 g) at 0° C and stirred for 30 min. To this was added a solution of (tert-butoxycarbonyl)-L-valine (0.080 g) in dichloromethane (2 ml) at 0° C and stirred at room temperature for 4 h. The reaction monitored by TLC and LCMS. After

completion of reaction, the reaction mixture was evaporated under vacuum to afford crude (7R,8R,9S,13S,14S,17S)-17-hydroxy-13-methyl-7-(9-((4,4,5,5,5-pentafluoropentyl)sulfinyl) nonyl)-7,8,9,11,12,13,14,15,16,17-decahydro-6*H*-cyclopenta[a]phenanthren-3-yl (tert-butoxycarbonyl)-L-valinate (0.285 g) as a sticky solid.

Step-2: Preparation of (7R,8R,9S5,13S,1485,17S)-17-hydroxy-13-methyl-7-(9-((4,4,5,5,5-pentafluoropentyl)sulfinyl) nonyl)-7,8,9,11,12,13,14,15,16,17-decahydro-6*H*-cyclopenta[a]phenanthren-3-yl L-valinate hydrochloride

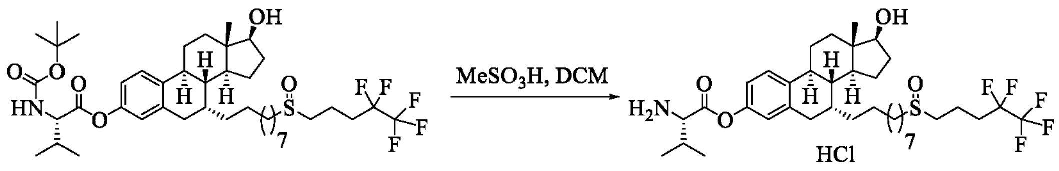


To a stirred solution of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl (tert-butoxycarbonyl)-L-valinate (0.285 g) in dichloromethane (5 ml), methanesulfonic acid (0.033 g) was added at 0° C and stirred for 4 h. The reaction was monitored by TLC and LCMS. After completion of reaction, the reaction mixture was evaporated under vacuum to obtain crude material. The obtained crude material was poured in saturated aqueous solution of sodium bicarbonate and then extracted in dichloromethane. The organic layer was washed with water, dried over anhydrous sodium sulphate and evaporated under vacuum to obtain crude material. The obtained crude material was purified using preparative HPLC using mobile phase A) 0.1% HCl in water and B) acetonitrile. The fractions were lyophilized to provide ((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl L-valinate hydrochloride (0.041 g) as an off white solid.

HPLC purity: 97.12%

$^1$H-NMR (400 MHz, DMSO): δ 8.59 (s, 3H), 7.38 (d, 1H), 6.92 (d, 1H), 6.87 (s, 1H), 4.52 (d, 1H), 4.17 (s, 1H), 3.56 (t, 1H), 2.77 (m, 6H), 2.37 (m, 5H), 1.91 (m, 4H), 1.57 (m, 4H), 1.23 (m, 25H), 0.89 (d, 1H) and 0.69 (s, 3H).

**Example-24: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl piperidin-4-yl carbonate hydrochloride**

Step-1: Preparation of tert-butyl 4-(((((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl)oxy)carbonyl)oxy)piperidine-1-carboxylate

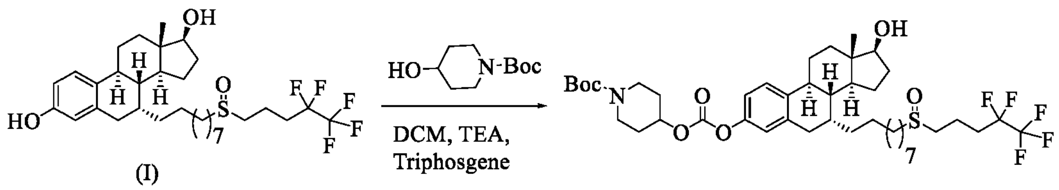


To a stirred solution of triphosgene (0.036 g) in dichloromethane (6 ml) was dropwise added a solution of tert-butyl 4-hydroxypiperidine-1-carboxylate (0.052 g) in triethylamine (0.045 ml) and dichloromethane (2 ml) at 0° C and stirred for 30 min. Then a solution of fulvestrant (0.150 g) in dichloromethane (2 ml) was added to the reaction mixture at 0° C and stirred at 25-27° C for 3 h. The reaction was monitored by TLC and LCMS. After completion of reaction, the reaction mixture was evaporated under vacuum to obtain crude tert-butyl 4-(((((7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl)oxy)carbonyl)oxy)piperidine-1-carboxylate (0.280 g). This was used for next step without purification.

Step-2: Preparation of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl piperidin-4-yl carbonate hydrochloride

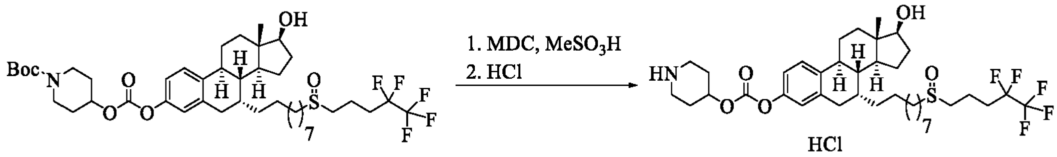


To a stirred solution of tert-butyl 4-(((((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl)oxy)carbonyl)oxy)piperidine-1-carboxylate (0.280 g) in dichloromethane (2 ml), methanesulfonic acid (0.023 g) was added at 0° C and stirred for 2 h at 25-27° C. The reaction was monitored by TLC and LCMS. After completion of reaction, the reaction mixture was evaporated under vacuum to obtain crude material. The obtained crude material was purified using preparative HPLC using mobile phase A) 0.1% formic acid in water and B) acetonitrile. The fractions were lyophilized to provide (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl piperidin-4-yl carbonate (0.063 g) as a white solid. The obtained (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl piperidin-4-yl carbonate (0.040 g) was further purified using preparative HPLC using mobile phase A) 0.1% HCl in water and B) acetonitrile. The fractions were lyophilized to provide (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl piperidin-4-yl carbonate hydrochloride (0.017 g) as an off white solid.

HPLC purity: 99.85%

$^1$H-NMR (400 MHz, DMSO): δ 8.56 (s, 2H), 7.31 (d, 1H), 6.96 (dd, 1H), 6.91 (d,1H), 4.93-4.87 (m, 1H), 4.50 (d, 1H), 3.57-3.52 (m, 1H), 3.21-3.19 (m, 2H), 3.13-3.07 (m, 2H), 2.88-2.80 (m, 2H), 2.76-2.62 (m, 4H), 2.42-2.22 (m, 4H), 2.13-2.07 (m, 2H), 19.4-1.79 (m, 6H), 1.71 (d, 1H), 1.64-1.46 (m, 4H), 1.38-1.16 (m, 18H), 0.80 (m,1 H) and 0.67 (s, 3H).

**Example-25: Preparation of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl 4-methyl-1,4-diazepane-1-carboxylate hydrochloride**

Step-1: Preparation of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl (4-nitrophenyl) carbonate

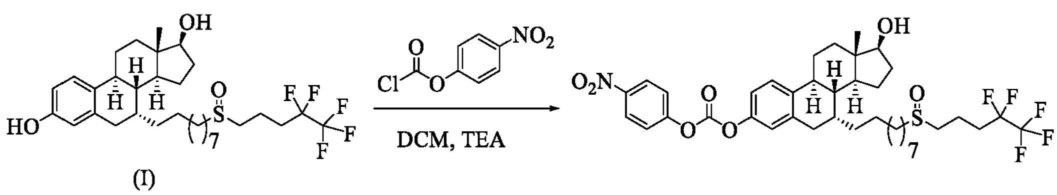


(I)

To a stirred solution of fulvestrant (0.200 g) in dichloromethane (10 ml) was added triethylamine (0.068 ml) followed by 4-nitrophenyl chloroformate (0.079 g) at 0° C. The reaction mixture was vigorously stirred at room temperature for 30 min. The reaction was monitored by TLC. After completion of reaction, the reaction mixture was directly used for next step.

Step-2: Preparation of (7R,8R,9S5,13S,1485,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl 4-methyl-1,4-diazepane-1-carboxylate hydrochloride

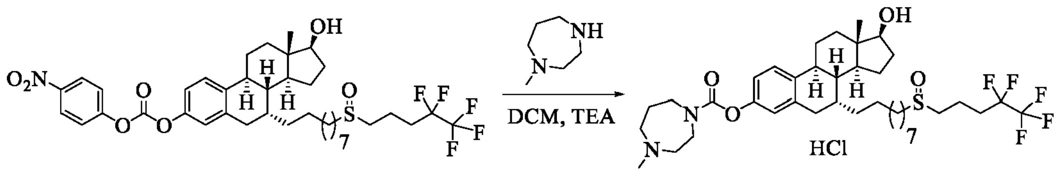


To a stirred solution of 1-methyl-1,4-diazepane (0.037 g) in dichloromethane (3 ml) was added triethyamine (0.05 ml). To this was portionwise added reaction mixture prepared in step-1 at 0° C and stirred at room temperature for 16 h. The reaction was monitored by TLC and LCMS. After completion of reaction, the reaction mixture was evaporated under vacuum to provide crude material. The obtained crude material was purified using preparative HPLC using mobile phase A) 0.1% HCl in water and B) acetonitrile. The fractions were lyophilized to provide (7R,8R,9S,13S,14S,17S)-17-hydroxy-13-methyl-7-(9-((4,4,5,5,5-pentafluoropentyl)sulfinyl) nonyl)-7,8,9,11,12,13,14,15,16,17-decahydro-6*H*-cyclopenta[a]phenanthren-3-yl 4-methyl-1,4-diazepane-1-carboxylate hydrochloride (0.045 g) as an off white solid.

HPLC purity: 99.06%

$^1$HNMR (400 MHz, DMSO): δ 10.22 (s, 1H), 7.29 (d, 1H), 6.90-6.82 (m, 2H), 4.49 (s, 1H), 4.0-3.86 (m, 1H), 3.65-3.45 (m, 6H), 3.27-3.16 (m, 2H), 2.88-2.81 (m, 5H), 2.76-2.60 (m, 4H), 2.45-2.25 (m, 4H), 2.17-2.07 (m, 2H), 1.94-1.70 (m, 5H), 1.64-1.48(m, 4H), 1.38-1.19 (m, 18H), 0.80 (m, 1H) and 0.67 (s, 3H).

**Example-26: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl dimethylglycinate hydrochloride**

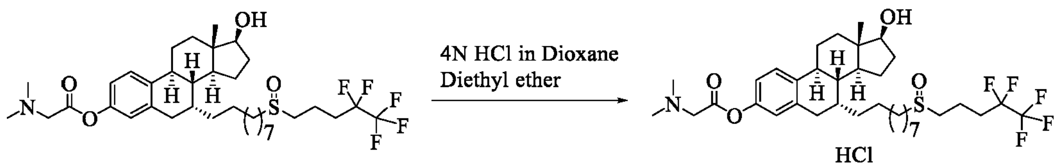


To a stirred solution of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H* cyclopenta[a]phenanthren-3-yl dimethylglycinate (0.02 g) in diethyl ether (0.5 ml) was added 4M HCl in dioxane (0.007 ml). The reaction mixture was stirred at room temperature for 30 min. After completion of reaction, the reaction mixture was evaporated under vacuum to obtain crude material. The obtained crude material was purified using preparative HPLC using mobile phase A) 0.1% HCl in water and B) acetonitrile. The fractions were lyophilized to provide (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl dimethylglycinate hydrochloride (0.006 g) as an off white sticky solid.

HPLC purity: 97.14%

$^1$H-NMR (400 MHz, DMSO): δ 10.1 (s, 1H), 7.36 (d, 1H), 6.94 (dd, 1H), 6.90 (d, 1H), 4.51 (s, 1H), 4.35 (s, 2H), 3.55 (t, 1H), 2.88-2.64 (m, 12H), 2.37-2.32 (m, 4H), 2.80-1.80 (m, 4H), 1.72 (d, 1H), 1.64-1.49 (m, 4H), 1.38-1.23 (m, 18H), 0.80 (m,1H) and 0.67 (s, 3H).

**Example-27: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 2-morpholinoacetate hydrochloride**

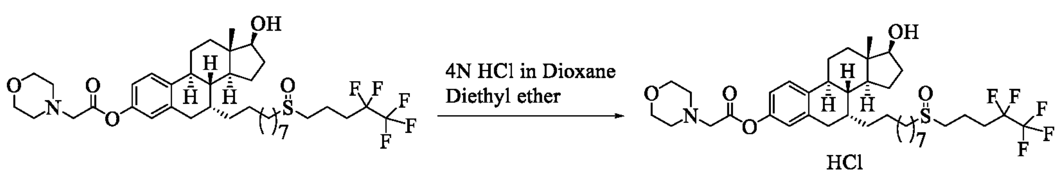


To a stirred solution of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl 2-morpholinoacetate (0.02 g) in

diethyl ether (0.5 ml) was added 4M HCl in dioxane (0.007 ml). The reaction mixture was stirred at room temperature for 30 min. After completion of reaction, the reaction mixture was evaporated under vacuum to obtain crude material. The obtained crude material was lyophilized to provide (7R,8R,9S,13S,14S,17S)-17-hydroxy-13-methyl-7-(9-((4,4,5,5,5-pentafluoropentyl)sulfinyl) nonyl)-7,8,9,11,12,13,14,15,16,17-decahydro-6*H*-cyclopenta[a]phenanthren-3-yl 2-morpholinoacetate hydrochloride (0.015 g) as a white solid.

HPLC purity: 95.43%.

$^1$H-NMR (400 MHz, DMSO): δ 10.8 (s, 1H), 7.35 (d, 1H), 6.92 (d, 1H), 6.88 (s, 1H), 4.43 (s, 3H), 3.76 (s, 4H), 3.54 (d, 1H), 3.12 (s, 4H), 2.88-2.81 (m, 2H), 2.76-2.60 (m, 4H), 2.39-2.26 (m, 4H), 1.94-1.80 (m, 4H), 1.71 (d, 1H), 1.64-1.48 (m, 4H), 1.38-1.23 (m, 18H), 0.80 (m, 1H) and 0.67 (s, 3H).

**Example-28: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 4-methylpiperazine-1-carboxylate hydrochloride**

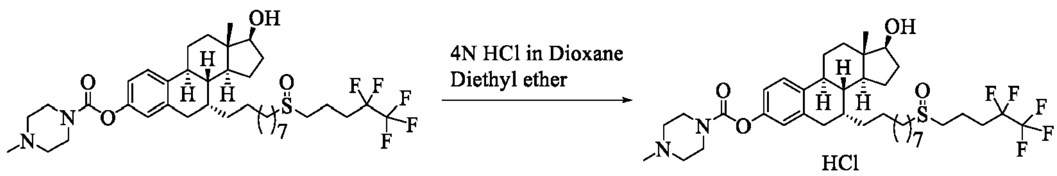


To a stirred solution of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl 4-methylpiperazine-1-carboxylate (0.01 g) in diethyl ether (0.5 ml) was added 4M HCl in dioxane (0.04 ml). The reaction mixture was stirred at room temperature for 30 min. After completion of reaction, the reaction mixture was evaporated and lyophilized to provide (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl 4-methylpiperazine-1-carboxylate hydrochloride (0.006 g) as a white solid.

HPLC purity: 97.85%

$^1$H-NMR (400 MHz, DMSO): δ 10.08 (s, 1H), 7.29 (d, 1H), 6.86 (dd, 1H), 6.82 (d, 1H), 4.50 (d, 1H), 4.13 (s, 2H) 3.54 (s, 1H), 3.41 (s, 2H), 3.1 (s, 2H), 2.88-2.59 (m, 8H), 2.50 (s, 3H), 2.40-2.38 (m, 4H), 1.94-1.80 (m, 4H), 1.71 (d, 1H), 1.62-1.48 (m, 4H), 1.38-1.19 (m, 18H), 0.80 (m, 1H) and 0.67 (s, 3H).

**Example-29: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl [1,4'-bipiperidine]-1'-carboxylate hydrochloride**

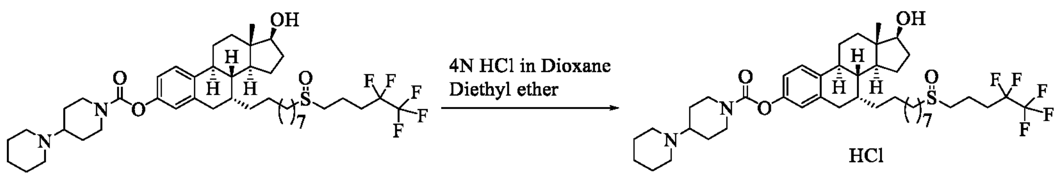


To a stirred solution of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl [1,4'-bipiperidine]-1'-carboxylate (0.03 g) in diethyl ether (4 ml) was added 4M HCl in dioxane (0.06 ml). The reaction mixture was stirred at room temperature for 30 min. After completion of reaction, the reaction mixture was evaporated and lyophilized to provide (7R,8R,9S, 13S, 14S, 17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl [1,4'-bipiperidine]-1'-carboxylate hydrochloride (0.017 g) as a white solid.

HPLC purity: 92.30%

$^1$H-NMR (400 MHz, DMSO): δ 9.45 (s, 1H), 7.29 (d, 1H), 6.83 (dd, 1H), 6.79 (s, 1H), 4.49-4.15 (m, 3H), 3.55 (t, 1H), 3.41-3.37 (m, 4H), 2.98-2.80 (m, 6H), 2.76-2.57 (m, 3H), 2.37-2.24 (m, 4H), 2.08 (d, 2H), 1.94-1.48 (m, 16H), 1.38-1.19 (m, 19H), 0.80 (m, 1H) and 0.67 (s, 3H).

**Example-30: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl (1-methylpyrrolidin-3-yl) carbonate hydrochloride**

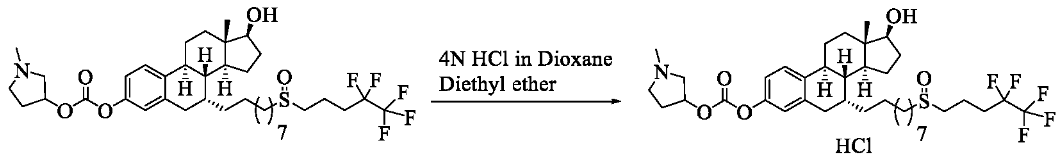


To a stirred solution of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl (1-methylpyrrolidin-3-yl) carbonate (0.02 g) in diethyl ether (0.5 ml) was added 4M HCl in dioxane (0.07 ml). The reaction mixture was stirred at room temperature for 30 min. After completion of reaction, the reaction mixture was evaporated and lyophilized to provide (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl (1-methylpyrrolidin-3-yl) carbonate hydrochloride (0.015 g) as a white solid.

HPLC purity: 99.40%

$^1$H-NMR (400 MHz, DMSO): δ 10.29 (s, 1H), 7.34 (d, 1H), 6.98 (d, 1H), 6.92 (s, 1H), 5.33 (s, 1H), 4.50 (s, 1H), 3.84-3.81 (m, 2H),3.55 (s, 1H), 3.16(s, 2H), 2.86-2.81 (m, 5H), 2.76-2.60 (m, 4H), 2.33-2.26 (m, 6H), 1.94-1.79 (m, 4H), 1.70 (s, 1H), 1.64-1.54 (m, 4H), 1.38-1.19 (m, 18H), 0.80 (m, 1H) and 0.67 (s, 3H).

**Example-31: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 2-(1-methylpiperidin-4-yl)acetate hydrochloride**

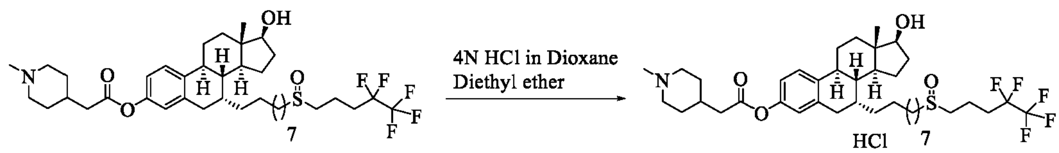


To a stirred solution of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl 2-(1-methylpiperidin-4-yl)acetate (0.02 g) in diethyl ether (0.5 ml) was added 4M HCl in dioxane (0.01 ml). The reaction mixture was stirred at room temperature for 30 min. After completion of reaction, the reaction mixture was evaporated and lyophilized to provide (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl 2-(1-methylpiperidin-4-yl)acetate hydrochloride (0.020 g) as an off white sticky solid.

HPLC purity: 90.09%

$^1$H-NMR (400 MHz, DMSO): δ 9.35 (s, 1H), 7.32 (d, 1H), 6.82 (dd, 1H), 6.80 (d, 1H), 4.50 (d, 1H), 3.53 (d, 1H), 3.32 (s, 2H), 2.88-2.81 (m, 4H), 2.76-2.62 (m, 8H), 2.42-2.25 (m, 5H), 1.96-1.46 (m, 14H), 1.38-1.19 (m, 18H), 0.80(m, 1H) and 0.67(s, 3H).

**Example-32: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 2-(4-methylpiperazin-1-yl)acetate hydrochloride**

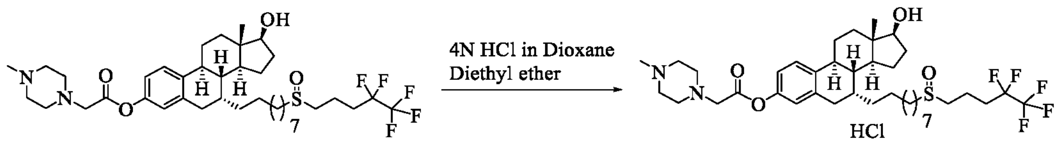


To a stirred solution of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl 2-(4-methylpiperazin-1-yl)acetate (0.02 g) in diethyl ether (0.5 ml) was added 4M HCl in dioxane (0.01 ml). The reaction mixture was stirred at room temperature for 30 min. After completion of reaction, the reaction mixture was evaporated under vacuum to provide crude material. The obtained crude material was purified using preparative HPLC using mobile phase A) 0.1% HCl in water and B) acetonitrile. The fractions were lyophilized to provide (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl 2-(4-methylpiperazin-1-yl)acetate hydrochloride (0.007 g) as an off white sticky solid.

HPLC purity: 97.43%

$^1$H-NMR (400 MHz, DMSO): δ 9.77 (s, 1H), 7.32 (d, 1H), 6.86 (dd, 1H), 6.82 (d, 1H), 3.65 (s, 2H), 3.55(t, 1H), 3.32(s, 2H), 3.05 (d, 4H), 2.86-2.66 (m, 11H), 2.35-2.31 (m, 4H), 1.94-1.80 (m, 4H), 1.71 (d, 1H), 1.64-1.48 (m, 4H), 1.38-1.16 (m, 18H), 0.80 (m, 1H) and 0.67 (s, 3H).

**Example-33: Preparation of (((7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6H-cyclopenta[a]phenanthren-3-yl)oxy)methyl dihydrogen phosphate**

Step: 1 Preparation of dibenzyl ((((7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6H-cyclopenta[a]phenanthren-3-yl)oxy)methyl) phosphate

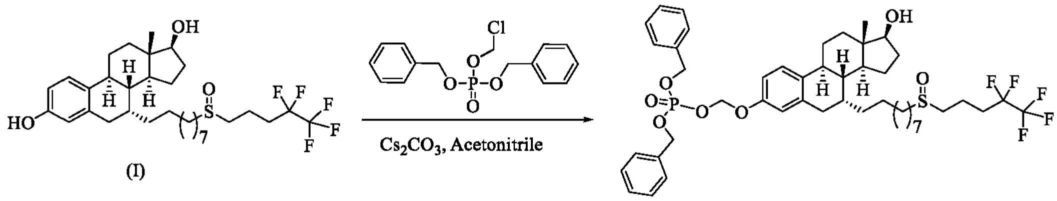

(I)

To a stirred solution of fulvestrant (1.0 g) in acetonitrile (20 ml), cesium carbonate (0.804 g) and dibenzyl (chloromethyl) phosphate (0.806 g) were added at 25° C and the reaction mixture was stirred for 16 h. The reaction was monitored by TLC and LCMS. Reaction mass was quenched with water (50 ml) and extracted in ethyl acetate (2x50 ml). Combined organic layer was washed with brine solution (50 ml) and dried over anhydrous sodium sulphate. The organic layer was evaporated under reduced pressure to provide crude compound. The obtained crude compound was purified using preparative HPLC using mobile phase A) 0.1% formic acid in water and B) acetonitrile. The fractions were lyophilized and used directly in the next step without further purification.

Step-2: Preparation of (((7R,8R,9S,13S,14S,17S)-17-hydroxy-13-methyl-7-(9-((4,4,5,5,5-pentafluoropentyl)sulfinyl) nonyl)-7,8,9,11,12,13,14,15,16,17-decahydro-6H-cyclopenta[a]phenanthren-3-yl)oxy)methyl dihydrogen phosphate

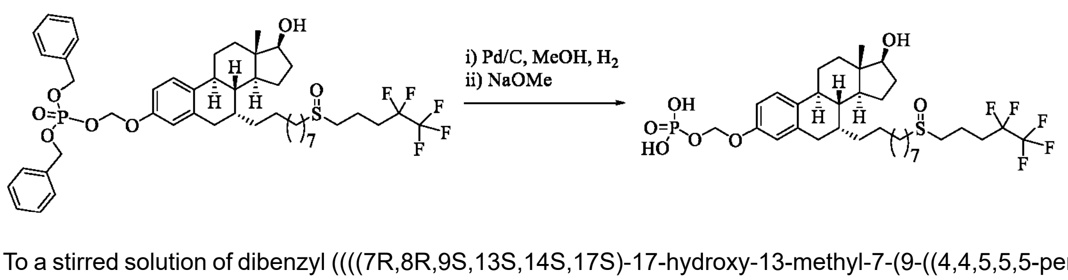


To a stirred solution of dibenzyl ((((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl)oxy)methyl) phosphate (0.040 g) in methanol (2 ml), 10% Pd/C (0.010 g) was added at room temperature. Hydrogen gas was purged to the reaction mixture at 25° C for 30 min. The reaction was monitored by TLC. After completion of reaction, the reaction mixture was filtered and washed with methanol (2x5 ml). Sodium methoxide (2.5 eq.) was added to the combined methanol solution at room temperature and stirred for 3 h. The reaction mixture was filtered and concentrated to provide crude compound (0.040 g). The obtained crude material was purified using preparative HPLC using mobile phase A) 0.05% triethyl ammonium acetate in water and B) acetonitrile to provide (((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl)oxy)methyl dihydrogen phosphate as a semi solid.
HPLC purity: 66.6%

**Example-34: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 2-amino-2-methylpropanoate hydrochloride**

Step: 1 Preparation of 7R,8R,9S,138S,148S,17S)-17-hydroxy-13-methyl-7-(9-((4,4,5,5,5-pentafluoropentyl)sulfinyl) nonyl)-7,8,9,11,12,13,14,15,16,17-decahydro-6*H*-cyclopenta[a]phenanthren-3-yl 2-((tert-butoxycarbonyl)amino)-2-methylpropanoate

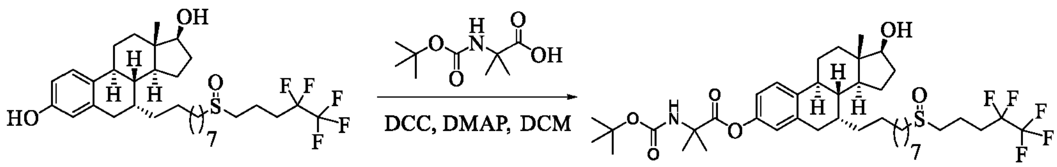


To a stirred solution of fulvestrant (0.300 g) in dichloromethane (6 ml) was added DMAP (0.012 g) followed by DCC (0.153 g) at 0° C and stirred for 30 min. A solution of 2-(tert-butoxycarbonylamino)-2-methylpropionic acid (0.100 g) in dichloromethane (2 ml) was added to the reaction mixture at 0° C and stirred at 25-27° C for 16 h. The reaction was monitored by TLC and LCMS. After completion of reaction, the reaction mixture was evaporated under reduced pressure to provide crude material which was purified by column chromatography to afford 7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta [a]phenanthren-3-yl 2-((tert-butoxycarbonyl)amino)-2-methylpropanoate (0.180 g) as a sticky solid.

Step:2 Preparation of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl 2-amino-2-methylpropanoate hydrochloride

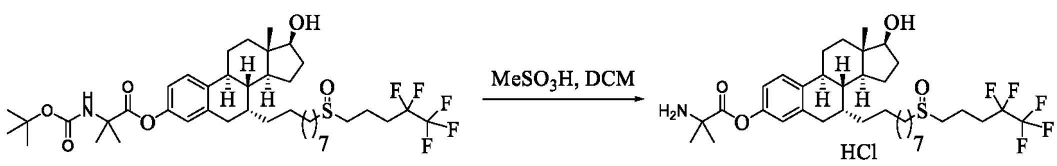


To a stirred solution of 7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl 2-((tert-butoxycarbonyl)amino)-2-methylpropanoate (0.100 g) in dichloromethane (5 ml) was added methanesulfonic acid (0.012 g) at 0° C and the reaction mixture was stirred at 25° C for 4 h. The reaction was monitored by TLC and LCMS. After completion of reaction, the reaction mixture was evaporated under reduced pressure to provide crude material which was purified using preparative HPLC using mobile phase A) 0.1% HCl in water and B) acetonitrile. The fractions were lyophilized to provide 7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl 2-amino-2-methylpropanoate hydrochloride (0.021 g) as a white solid.

HPLC purity: 96.43%
$^1$H-NMR (400 MHz, DMSO): δ 8.61 (s, 3H), 7.38 (d, 1H), 6.94 (d, 1H), 6.87 (s, 1H), 4.51 (d, 1H), 3.58-3.53 (m, 1H), 2.88 2.61 (m, 6H), 2.42-2.25 (m, 4H), 1.94-1.79 (m, 4H), 1.71 (s, 1H), 1.62-1.46 (m, 10H), 1.38-1.18 (m, 18H), 0.89 (m, 1H) and 0.68

(s, 3H).

**Example-35: Preparation of 4-(((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl)oxy)-4-oxobut-2-enoic acid**

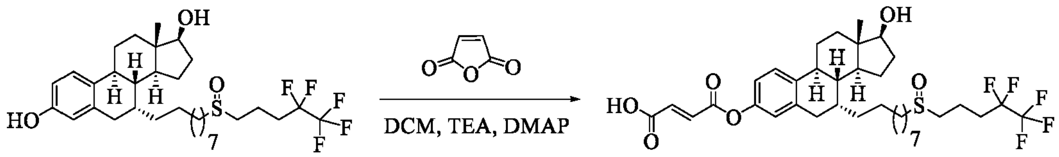


To a stirred solution of fulvestrant (0.300 g) in dichloromethane (4 ml), triethylamine (0.2 ml) and DMAP (0.012 g) were added at 0° C and stirred for 20 min. Maleic anhydride (0.150 g) was added portionwise to the reaction mixture and stirred at 25° C for 16 h. The reaction was monitored by TLC and LCMS. After completion of reaction, the reaction mixture was evaporated under reduced pressure to provide crude material which was purified using preparative HPLC using mobile phase A) 0.1% formic acid in water and B) acetonitrile. The fractions were lyophilized to provide 4-(((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl)oxy)-4-oxobut-2-enoic acid (0.025 g).

HPLC purity: 97.27%

$^1$H NMR (400 MHz, DMSO): δ 12.983 (bs, 1H), 9.026 (bs, 1H), 7.068 (d, 1H), 6.532-6.300 (m, 2H), 4.701 (t, 1H), 2.898-2.608 (m, 6H), 2.456-2.344 (m, 3H), 2.277-2.129 (m, 3H), 1.959-1.883 (m, 2H), 1.831-1.803 (m, 1H), 1.679-1.487 (m, 6H), 1.459-1.385 (m, 14H), 0.983-0.896 (m, 1H) and 0.853 (s, 3H).

**Example 36: Preparation of 5-(((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl)oxy)-5-oxopentanoic acid**

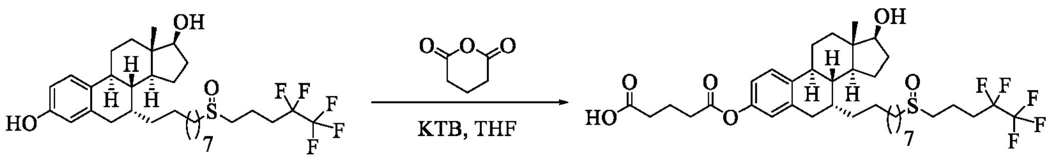


To a stirred solution of fulvestrant (0.300 g) in THF (5 ml), potassium tert-butoxide (0.061 g) was added. The reaction mixture was stirred at 70-72° C for 30 min. A solution of glutaric anhydride (0.062 g) in tetrahydrofuran (1 ml) was added to the reaction mixture and stirred at 70-72° C for 3 h. The reaction was monitored by TLC and LCMS. After completion of reaction, the reaction mixture was quenched with chilled water (40 ml). The reaction mixture was extracted in ethyl acetate (3x20 ml). The combined organic layer was washed with brine solution (2x20 ml) and dried over anhydrous sodium sulphate. The organic layer was concentrated below 40° C under reduced pressure to provide crude material. The crude material was purified by flash chromatography to afford 5-(((7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl)oxy)-5-oxopentanoic acid as an off white solid (0.085 g).

HPLC purity: 98.56%

$^1$H NMR (400 MHz, DMSO): δ 7.32 (d, 1H), 6.83 (dd, 2H), 4.53 (s, 1H), 3.56 (s, 1H), 2.89-2.82 (m, 2H), 2.78-2.64 (m, 6H), 2.61-2.57 (m, 3H), 2.33-2.30 (m, 6H), 1.93 (t, 3H), 1.89-1.82 (m, 3H), 1.71 (bs, 1H), 1.63-1.51 (m, 3H), 1.51 (bs, 1H), 1.36-1.25 (m, 20H), 0.89 (s, 1H) and 0.68 (s, 3H).

**Example-37: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl (1-methylpyrrolidin-3-yl) carbonate**

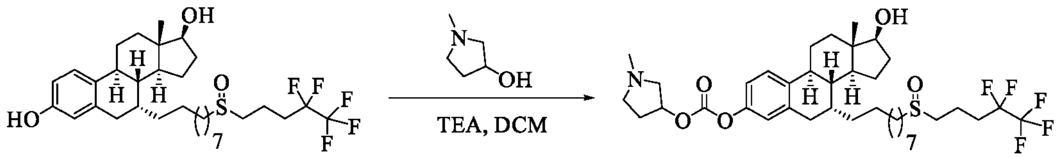


To a stirred solution of fulvestrant (0.150 g) in dichloromethane (3 ml), 4-nitrophenyl chloroformate (0.036 g) and triethylamine (0.037 g) were added at 0° C and the reaction mixture was stirred for 30 min. A solution of methylpyrrolidin-3-ol (0.101 g) in dichloromethane (2 ml) was added to the reaction mixture at 0° C and stirred at 25° C for 16 h. The reaction was monitored by TLC and LCMS. After completion, the reaction mixture was evaporated under reduced pressure to provide crude material which was purified using preparative HPLC using mobile phase A) ammonium bicarbonate in water and B) acetonitrile. The fractions were lyophilized to provide (7R,8R,9S, 13 S, 14S, 17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl (1-methylpyrrolidin-3-yl) carbonate (0.060 g) as an off white sticky solid.

HPLC purity: 99.86%

$^1$H NMR (400 MHz, DMSO): δ 7.32 (d, 1H), 6.95 (d, 1H), 6.90 (s, 1H), 5.08 (m, 1H), 4.50 (d, 1H), 3.54 (m, 1H), 2.86-2.76 (m, 2H), 2.76-2.59 (m, 7H), 2.35-2.31 (m, 9H), 1.92-1.79 (m, 5H), 1.71 (s, 1H), 1.62-1.46 (m, 4H), 1.38-1.16 (m, 18H), 0.89 (m, 1H) and 0.67 (s, 3H).

**Example-38: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl (1-methylpiperidin-4-yl)carbamate**

Step-1: Preparation of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6H-cyclopenta[a]phenanthren-3-yl (4-nitrophenyl) carbonate

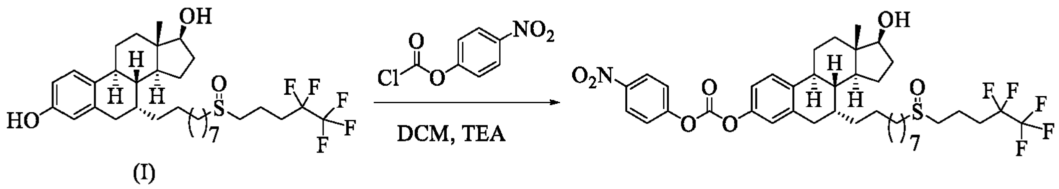


(I)

To a stirred solution of fulvestrant (0.150 g) in dichloromethane (10 ml) was added triethylamine (0.05 ml) followed by 4-nitrophenyl chloroformate (0.055 g) at 0° C. The reaction mixture was warmed to 25-27° C and vigorously stirred for 3 h. The reaction was monitored by TLC. After completion of reaction, the reaction mixture was directly used for next step.

Step-2: Preparation of (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl (1-methylpiperidin-4-yl)carbamate

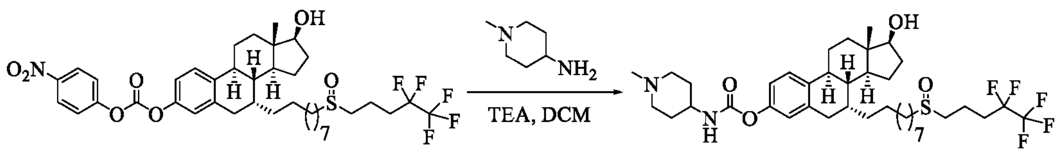


To a solution of 1-methylpiperidin-4-amine (0.031 g) in dichloromethane (5 ml) was added triethyamine (0.05 ml). To this was portionwise added reaction mixture prepared in step-1 at 0° C and stirred at 25-27° C for 6 h. The reaction was monitored by TLC and LCMS. After completion of reaction, the reaction mixture was evaporated under vacuum to provide crude material. The obtained crude material was purified using preparative HPLC using mobile phase A) 10 mM ammonium acetate in water and B) acetonitrile. The fractions were lyophilized to provide (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl (1-methylpiperidin-4-yl)carbamate (0.045 g) as an off white solid.

HPLC purity: 99.25%
$^1$H NMR (400 MHz, DMSO): δ 7.65 (d, 1H), 7.25 (d, 1H), 6.80 (q, 1H), 6.75 (d, 1H), 4.49 (d, 1H), 3.55 (m, 1H), 3.23 (S, 1H) 2.88-2.60 (m, 7H), 2.43-2.23 (m, 4H), 2.13 (s, 3H), 1.93-1.67 (m, 10H), 1.63-1.15 (m, 24H), 0.90 (m, 1H) and 0.67 (s, 3H).

**Example-39: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 2-(pyrrolidin-1-yl)acetate hydrochloride**

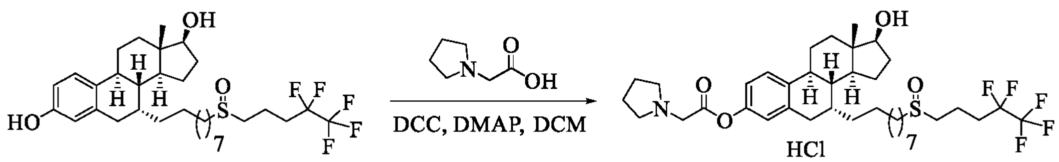


To a stirred solution of fulvestrant (0.150 g) and 2-(pyrrolidin-1-yl)acetic acid (0.038 g) in dichloromethane (6 ml), 4-dimethylaminopyridine (0.006 g) followed by a solution of DCC (0.076 g) in dichloromethane (1 m) was added dropwise at 0° C. Reaction mixture warmed and stirred at 25-27° C for 16 h. The reaction was monitored by TLC and LCMS. After completion of reaction, the reaction mixture was evaporated under reduced pressure to provide crude material which was purified using preparative HPLC using mobile phase A) 0.1% HCl in water and B) acetonitrile. The fractions were lyophilized to provide (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl 2-(pyrrolidin-1-yl)acetate hydrochloride (0.030 g) as a sticky white solid.
HPLC purity: 98.95%
$^1$H NMR (400 MHz, DMSO): δ 10.51 (s, 1H), 7.37 (d, 1H), 6.94 (d, 1H), 6.90 (s, 1H), 4.50 (s, 3H), 3.56 (m, 3H), 3.21 (d, 2H), 2.76 (m, 6H), 2.35 (m, 4H), 1.88 (m, 8H), 1.72 (d, 1H), 1.57 (m, 4H), 1.28 (m, 18H), 0.89 (t, 1H) and 0.68 (s, 3H).

**Example-40: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 2,5,8,11-tetraoxatridecan-13-oate**

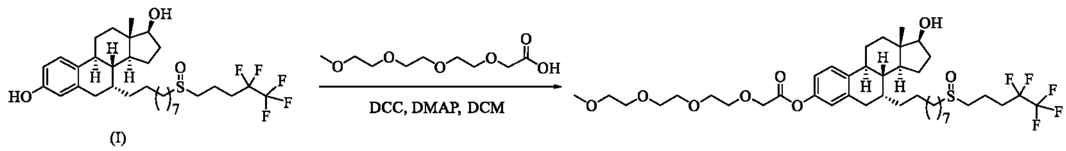


To a stirred solution of fulvestrant (0.150 g) and 2,5,8,11-tetraoxatridecan-13-oic acid (0.549 g) in dichloromethane (10 ml), DMAP (0.006 g) followed by DCC (0.076 g) was added at 0° C. Reaction mixture was warmed to room temperature and stirred for 6 h. The reaction was monitored by TLC and LCMS. After completion of reaction, the reaction mixture was concentrated under reduced pressure to provide crude material. The obtained crude material was purified using preparative HPLC using mobile phase A) 10 mM ammonium bicarbonate in water and B) acetonitrile. The fractions were lyophilized to provide (7R,8R,9S,138S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl 2,5,8,11-tetraoxatridecan-13-oate (0.075 g) as a colourless thick liquid.
HPLC: 97.06%
$^1$H NMR (400 MHz, DMSO): δ 7.33 (d, 1H), 6.89 (d, 1H), 6.83 (s, 1H), 4.50 (d, 1H), 4.38 (s, 2H), 3.68.3.66 (m, 2H), 3.58-3.49 (m, 9H), 3.43-3.41 (m, 2H), 3.23 (s, 3H), 2.87-2.62 (m, 6H), 2.42-2.25 (m, 4H), 1.94-1.79 (m,4H), 1.71 (s, 1H), 1.63-1.48 (m, 4H), 1.38-1.18 (m, 18H), 0.90 (m, 1H) and 0.67 (s, 3H).

**Example-41: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 2,5,8,11,14-pentaoxaheptadecan-17-oate**

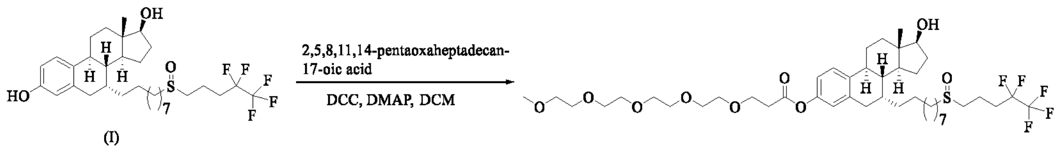


(I)

To a stirred solution of fulvestrant (0.150 g) and 2,5,8,11,14-pentaoxaheptadecan-17-oic acid (0.549 g) in dichloromethane (10 ml), DMAP (0.006 g) followed by DCC (0.076 g) was added at 0° C. The reaction mixture was warmed to room temperature and stirred for 6 h. The reaction was monitored by TLC and LCMS. After completion of reaction, the reaction mixture was concentrated under reduced pressure to provide crude material. The obtained crude material was purified using preparative HPLC using mobile phase A) 10 mM ammonium bicarbonate in water and B) acetonitrile. The fractions were lyophilized to provide (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 2,5,8,11,14-pentaoxaheptadecan-17-oate (0.110 g) as a colourless thick liquid.

HPLC: 97.74%

$^1$H NMR (400 MHz, DMSO): δ 7.31 (d, 1H), 6.84 (d, 1H), 6.77 (s, 1H),4.50 (d, 1H), 3.72 (t, 2H), 3.54-3.48 (m, 15H), 3.42-3.41 (m, 2H), 3.22 (s, 3H), 2.87-2.67 (m, 8H), 2.37-2.38 (m, 4H), 1.94-1.79 (m, 4H), 1.71 (s, 1H), 1.63-1.48 (m, 4H), 1.38-1.1 8 (m, 18H), 0.90 (m, 1H) and 0.67 (s, 3H).

**Example-42: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 2,5,8,11,14,17-hexaoxaicosan-20-oate**

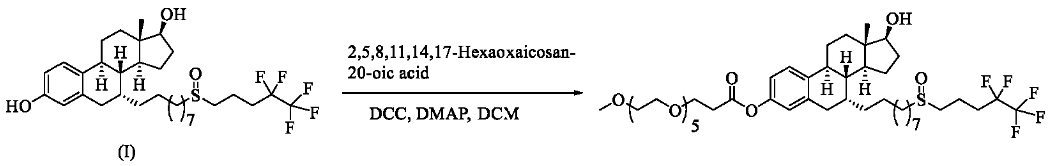


(I)

To a stirred solution of fulvestrant (0.100 g) and 2,5,8,11,14,17,20-heptaoxatricosan-23-oic acid (0.066 g) in dichloromethane (10 ml), DMAP (0.006 g) followed by DCC (0.051 g) was added at 0° C and stirred for 6 h. The reaction was monitored by TLC and LCMS. After completion of reaction, the reaction mixture was evaporated under vacuum to provide crude material. The crude material was purified using preparative HPLC using mobile phase A) 10 mM ammonium bicarbonate in water and B) acetonitrile. The fractions were lyophilized to provide (7R,8R,9S, 13 S, 14S, 17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl 2,5,8,11,14,17-hexaoxaicosan-20-oate (0.043 g) as a white semi solid.

HPLC: 96.83%

$^1$H NMR (400 MHz, DMSO): δ 7.31 (d, 1H), 6.84 (d, 1H), 6.77 (s, 1H), 4.50 (d, 1H), 3.74 (t, 2H), 3.55-3.48 (m, 22 H), 3.42-3.40 (m,2H), 3.23 (s, 3H), 2.88-2.64 (m, 8H), 2.39-2.25 (m, 5H), 1.94-1.79 (m, 4H), 1.71 (s, 1H), 1.62-1.48 (m ,4H), 1.38-1.18 (m, 18H), 0.90 (m, 1H) and 0.67 (s, 3H).

**Example-43: Preparation of (7R,8R,9S,13S,14S,17S)-17-hydroxy-13-methyl-7-(9-((4,4,5,5,5-pentafluoropentyl) sulfinyl)nonyl)-7,8,9,11,12,13,14,15,16,17-decahydro-6*H*-cyclopenta[a]phenanthren-3-yl 2,5,8,11,14,17,20-heptaoxatricosan-23-oate**

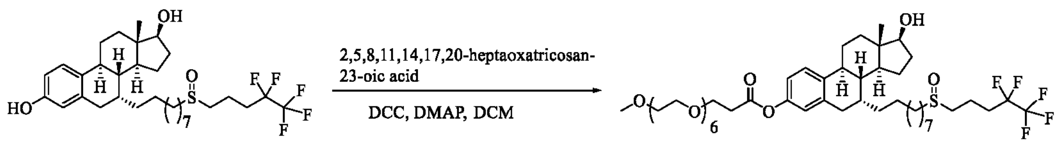


(I)

To a stirred solution of fulvestrant (0.100 g) and 2,5,8,11,14,17-hexaoxaicosan-20-oic acid (0.058 g) in dichloromethane (10 ml), DMAP (6.04 mg) followed by DCC (50.99 mg) was added at 0° C. The reaction mixture was warmed to room temperature and stirred for 16 h. The reaction was monitored by TLC and LCMS. After completion, the

reaction mixture was evaporated under vacuum to provide crude material. The obtained crude material was purified using preparative HPLC using mobile phase A) 10 mM ammonium bicarbonate in water and B) acetonitrile. The fractions were lyophilized to provide (7R,8R,9S,13S,14S,17S)-17-hydroxy-13-methyl-7-(9-((4,4,5,5,5-pentafluoropentyl)sulfinyl)nonyl)-7,8,9,11,12,13,14,15,16,17-decahydro-6*H*-cyclopenta[a]phenanthren-3-yl 2,5,8,11,14,17-hexaoxaicosan-20-oate (0.055 g) as a colourless thick liquid.

HPLC: 99.91%

$^1$H NMR (400 MHz, DMSO): δ 7.31 (d, 1H), 6.84 (d, 1H), 6.77 (s, 1H),4.49 (d, 1H), 3.74 (t, 2H), 3.55-3.48 (m, 18H), 3.42-3.40 (m, 2H), 3.23 (s, 3H), 2.88-2.62 (m, 8H), 2.37-2.28 (m, 5H), 1.94-1.79 (m, 4H), 1.72 (s, 1H), 1.62-1.48 (m, 4H), 1.38-1.16 (m, 18H), 0.90 (m, 1H) and 0.67 (s, 3H).

**Example-44: Preparation of ((7R,8R,13S,14S,17S)-17-hydroxy-13-methyl-7-(9-(4,4,5,5,5-pentafluoropentylsulfinyl)nonyl)-7,8,9,11,12,13,14,15,16,17-decahydro-6H-cyclopenta[a]phenanthren-3-yloxy)methyl dihydrogen phosphate**

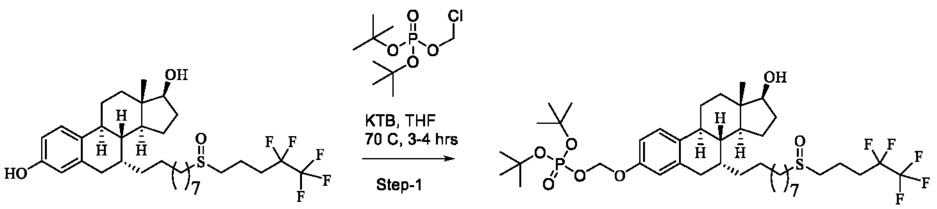


Step-1: Preparation of di-tert-butyl ((7R,8R,13 S,14S,17S)-17-hydroxy-13-methyl-7-(9-(4,4,5,5,5-pentafluoropentyl sulfinyl)nonyl)-7,8,9,11,12,13,14,15,16,17-decahydro-6H-cyclopenta[a]phenanthren-3-yloxy)methyl phosphate

To a stirred solution of fulvestrant (1.0g) in tetrahydrofuran (10.0mL) under nitrogen gas atmosphere at room temperature was added potassium tert-butoxide (0.28g) and this reaction mixture was heat to 70°C for 5 minutes. Di-tert-butyl chloromethyl phosphate (0.60g) in tetrahydrofuran (1 mL) was added drop wise and this reaction mixture was stirred at 70°C for 3-4 h. The reaction was monitored on TLC. The reaction mixture was cooled to room temperature, the reaction mixture was diluted with brine solution (10mL), ethyl acetate (20 mL) and extracted with ethyl acetate. The organic layer was dried over anhydrous sodium sulphate and concentrated under reduced pressure. The crude thus isolated to afford di-tert-butyl ((7R,8R,13S,14S,17S)-17-hydroxy-13-methyl-7-(9-(4,4,5,5,5-pentafluoropentylsulfinyl)nonyl)-7,8,9,11,12,13,14,15,16,17-decahydro-6H-cyclopenta[a]phenanthren-3-yloxy)methyl phosphate as a gummy solid (1.3g, 95.58%). LCMS: 81.63% (m/z: 830.6, 831.6, [M+1]+, [M+2]+). The crude product was used in next step.

Step-2: Preparation of ((7R,8R,13S,14S,17S)-17-hydroxy-13-methyl-7-(9-(4,4,5,5,5-pentafluoropentylsulfinyl)nonyl)-7,8,9,11,12,13,14,15,16,17-decahydro-6H-cyclopenta[a]phenanthren-3-yloxy)methyl dihydrogen phosphate

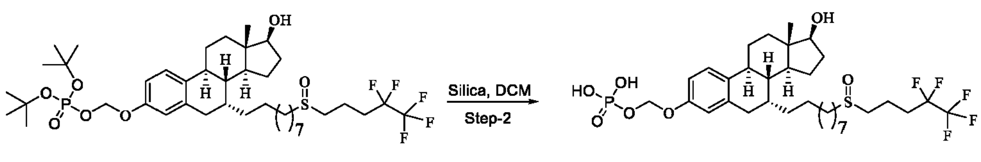


To a stirred solution of di-tert-butyl ((7R,8R,13S,14S,17S)-17-hydroxy-13-methyl-7-(9-(4,4,5,5,5-pentafluoropentyl sulfinyl)nonyl)-7,8,9,11,12,13,14,15,16,17-decahydro-6H-cyclopenta[a]phenanthren-3-yloxy)methyl phosphate (1.3g) in dichloromethane (5.0mL) at room temperature was added silica gel 60-120 mesh (2.6g). The reaction mixture was put at room temperature for 3 h. The reaction was monitored on TLC. The reaction mixture was diluted with dichloromethane (5.0mL) and filtered the slurry. The silica was slurry washed with 10 % methanol in dichloromethane and filtered to get clear solution. The filtrate was combined and concentrated under reduced pressure. The crude thus isolated was purified by PREP HPLC using mobile Phase A) 10 mmol ammonium bicarbonate in water B) 100% acetonitrile. Product fraction was lyophilized to afford ((7R,8R,13S,14S,17S)-17-hydroxy-13-methyl-7-(9-(4,4,5,5,5-pentafluoropentylsulfinyl)nonyl)-7,8,9,11,12,13,14,15,16,17-decahydro-6H-cyclopenta[a]phenanthren-3-yloxy)methyl dihydrogen phosphate as an off-white solid (0.123g, 10.98%).

HPLC purity: 96.33%
1H NMR (400 MHz, DMSO-d6): 1H NMR (400 MHz, DMSO) δ 7.13 (d, J = 8.7 Hz, 1H), 6.77 (d, J = 8.4 Hz, 1H), 6.67 (s, 1H), 5.30 (d, J = 4.6 Hz, 2H), 3.61 - 3.47 (m, 2H), 2.89 - 2.59 (m, 6H), 2.39 - 2.16 (m, 4H), 1.90 (dd, J = 15.5, 7.9 Hz, 3H), 1.78 (d, J = 11.5 Hz, 1H), 1.70 - 1.41 (m, 5H), 1.21 (t, J = 25.0 Hz, 14H), 0.88 (s, 1H), 0.65 (s, 3H).

**Example-45: Preparation of 2S)-2-(((7R,8R,13S,14S,17S)-17-hydroxy-13-methyl-7-(9-(4,4,5,5,5-pentafluoropentylsulfinyl)nonyl)-7,8,9,11,12,13,14,15,16,17-decahydro-6H-cyclopenta[a]phenanthren-3-yloxy)carbonylamino)-3-methylbutanoic acid**

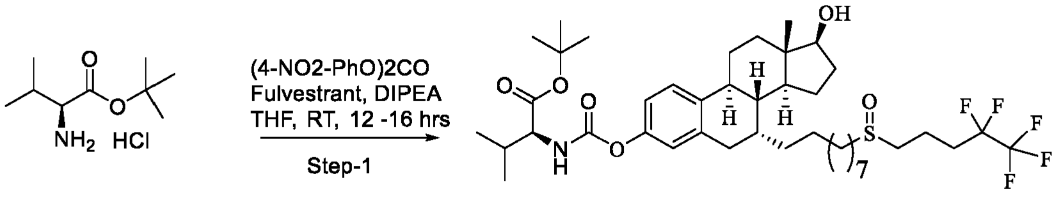


Step-1: Preparation of (2S)-tert-butyl 2-(((7R,8R,13S,14S,17S)-17-hydroxy-13-methyl-7-(9-(4,4,5,5,5-pentafluoropentylsulfinyl)nonyl)-7,8,9,11,12,13,14,15,16,17-decahydro-6H-cyclopenta[a]phenanthren-3-yloxy) carbonylamino)-3-methylbutanoate

To a stirred solution of (S)-tert-butyl 2-amino-3-methylbutanoate Hydrochloride (0.38g) in tetrahydrofuran (10.0mL) under nitrogen gas atmosphere at room temperature was added bis(4-notrophenyl) carbonate (0.56g) and this reaction mixture was cooled to 0 °C. N,N-Diisopropylethylamine (0.7g) was added drop wise and this reaction mixture was stirred at room temperature for overnight. To this reaction mixture fulvestrant (1.0g), N,N-Diisopropylethylamine (0.7g) was added and this reaction mixture was again stirred at room temperature for overnight. The reaction was monitored on TLC. The reaction mixture was diluted with brine solution (10mL) and ethyl acetate (20ml) and extracted with ethyl acetate. The organic layer was dried over anhydrous sodium sulphate and concentrated under reduced pressure. The crude thus isolated, was purified by PREP HPLC using mobile Phase A) 10 mm ammonium bicarbonate in water B) 100% acetonitrile. Product fraction was lyophilized to afford (2S)-tert-butyl 2-(((7R,8R,13S,14S,17S)-17-hydroxy-13-methyl-7-(9-(4,4,5,5,5-pentafluoro pentylsulfinyl)nonyl)-7,8,9,11,12,13,14,15,16,17-decahydro-6H-cyclopenta[a]phenanthren-3-yloxy)carbonylamino) - 3-methylbutanoate as a white solid (0.65g, 48.8%).
HPLC purity: 99.13%

Step-2: Preparation of (2S)-2-(((7R,8R,13S,14S,17S)-17-hydroxy-13-methyl-7-(9-(4,4,5,5,5-pentafluoropentylsulfinyl) nonyl)-7,8,9,11,12,13,14,15,16,17-decahydro-6H-cyclopenta[a]phenanthren-3-yloxy)carbonylamino)-3-methylbutanoic acid

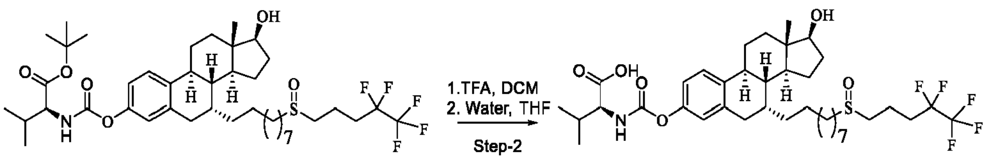


To a stirred solution of (2S)-tert-butyl 2-(((7R,8R,13S,14S,17S)-17-hydroxy-13-methyl-7-(9-(4,4,5,5,5-pentafluoro pentylsulfinyl)nonyl)-7,8,9,11,12,13,14,15,16,17-decahydro-6H-cyclopenta[a]phenanthren-3-yloxy)carbonylamino) -3-methylbutanoate (0.4g,) in dichloromethane (10.0mL) under ice-cooling was added trifluoroaceticacid (0.7 ml) under nitrogen gas atmosphere and this reaction was stirred at room temperature for overnight. The reaction was monitored on TLC. The reaction mixture was concentrated under reduced pressure. The crude thus isolated was dissolved in tetrahydrofuran (5 mL), water (2.5 ml) and this reaction mixture again stirred at room temperature for overnight. The reaction was monitored on LCMS. The reaction mixture was concentrated under reduced pressure. The crude thus isolated, was purified by PREP HPLC using mobile Phase A) 10 mmol ammonium bicarbonate in water B) 100% acetonitrile. Product fraction was lyophilized to afford (2S)-2-(((7R,8R,13S,14S,17S)-17-hydroxy-13-methyl-7-(9-(4,4,5,5,5-pentafluoropentylsulfinyl)nonyl)-7,8,9, 11, 12, 13, 14, 15, 16, 17-decahydro-6H-cyclopenta[a]phenanthren-3-yloxy)carbonylamino)-3-methylbutanoic acid as a white solid (0.047 g, 12.7%).

HPLC purity: 99.32%

1H NMR (400 MHz , DMSO-d6): 1H NMR (400 MHz, DMSO) δ 7.64 (s, 1H), 7.24 (d, J = 8.4 Hz, 1H), 6.79 (d, J = 8.2 Hz, 1H), 6.74 (s, 1H), 4.50 (s, 1H), 3.78 (s, 1H), 3.53 (s, 1H), 2.80 (d, J = 8.4 Hz, 2H), 2.76 - 2.58 (m, 4H), 2.39 - 2.20 (m, 5H), 2.08 (d, J = 6.2 Hz, 1H), 1.95 - 1.82 (m, 3H), 1.79 (d, J = 12.4 Hz, 1H), 1.66 (s, 1H), 1.54 (dd, J = 25.2, 16.4 Hz, 4H), 1.41 - 1.07 (m, 17H), 1.01 (d, J = 6.1 Hz, 2H), 0.88 (d, J = 5.8 Hz, 7H), 0.65 (s, 3H).

**Example-46: Preparation of (2S)-2-(((7R,8R,13S,14S,17S)-17-hydroxy-13-methyl-7-(9-(4,4,5,5,5-pentafluoropentylsulfinyl)nonyl)-7,8,9,11,12,13,14,15,16,17-decahydro-6H-cyclopenta[a]phenanthren-3-yloxy)carbonylamino)-3-phenylpropanoic acid**

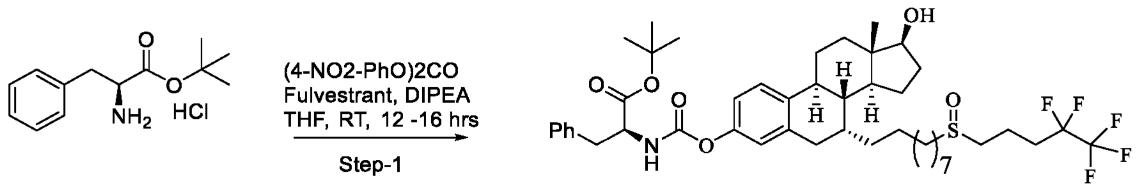


Step-1: Preparation of (2S)-tert-butyl 2-(((7R,8R,13S,14S,17S)-17-hydroxy-13-methyl-7-(9-(4,4,5,5,5-pentafluoro pentylsulfinyl)nonyl)-7,8,9,11,12,13,14,15,16,17-decahydro-6H-cyclopenta[a]phenanthren-3-yloxy)carbonyl amino)-3-phenylpropanoate

To a stirred solution of (S)-tert-butyl 2-amino-3-phenylpropanoate (0.24g) in tetrahydrofuran (8.0mL) under nitrogen gas atmosphere at room temperature was added bis(4-notrophenyl) carbonate (0.28g) and this reaction mixture was cooled to 0 °C. N,N-Diisopropylethylamine (0.35g) was added drop wise and this reaction mixture was stirred at room temperature for overnight. To this reaction mixture fulvestrant (0.5g), N,N-Diisopropylethylamine (0.35g) was added and this reaction mixture was again stirred at room temperature for overnight. The reaction was monitored on TLC. The reaction mixture was diluted with brine solution (10mL) and ethyl acetate (20ml) and extracted with ethyl acetate. The organic layer was dried over anhydrous sodium sulphate and concentrated under reduced pressure. The crude thus isolated, was purified by PREP HPLC using mobile Phase A) 10 mm ammonium bicarbonate in water B) 100% acetonitrile. Product fraction was lyophilized to (2S)-tert-butyl 2-(((7R,8R,13S,14S,17S)-17-hydroxy-13-methyl-7-(9-(4,4,5,5,5-pentafluoropentylsulfinyl)nonyl)-7,8,9,8,9,11,12,13,14,15, 16,17-decahydro-6H-cyclopenta[a]phenanthren-3-yloxy)carbonylamino)-3-phenylpropanoate as a white solid (0.27g, 33.96%).

Step-2: Preparation of (2S)-2-(((7R,8R,13S,14S,17S)-17-hydroxy-13-methyl-7-(9-(4,4,5,5,5-pentafluoropentyl sulfinyl) nonyl)-7,8,9,11,12,13,14,15,16,17-decahydro-6H-cyclopenta[a]phenanthren-3-yloxy)carbonylamino)-3-phenylpropanoic acid

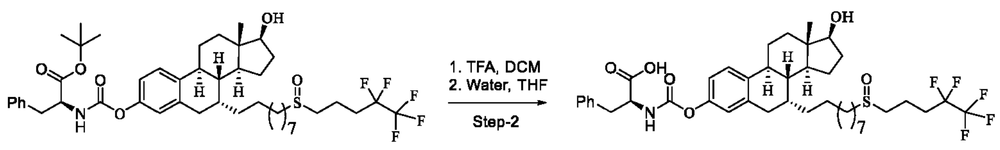


To a stirred solution of (2S)-tert-butyl 2-(((7R,8R,13S,14S,17S)-17-hydroxy-13-methyl-7-(9-(4,4,5,5,5-pentafluoro pentylsulfinyl)nonyl)-7,8,9,11,12,13,14,15,16,17-decahydro-6H-cyclopenta[a]phenanthren-3-yloxy)carbonylamino) -3-phenylpropanoate (0.26g,) in dichloromethane (5.0 mL) under ice-cooling was added trifluoroaceticacid (0.28 ml) under nitrogen gas atmosphere and this reaction was stirred at room temperature for overnight. The reaction was monitored on TLC. The reaction mixture was concentrated under reduced pressure. The crude thus isolated was dissolved in tetrahydrofuran (2.0 mL), water (1.0 mL) and this reaction mixture again stirred at room temperature for overnight. The reaction was monitored on LCMS. The reaction mixture was concentrated under reduced pressure. The crude thus isolated, was purified by PREP HPLC using mobile Phase A) 10 mmol ammonium bicarbonate in water B) 100% acetonitrile. Product fraction was lyophilized to afford (2S)-2-(((7R,8R,1385,14S,175)-17-hydroxy-13-methyl-7-(9-(4,4,5,5,5-pentafluoropentylsulfinyl)nonyl)-7,8,9,11,12,13,14,15,16,17-decahydro-6H-cyclopenta[a]phenanthren-3-yloxy) carbonylamino)-3-phenylpropanoic acid as a white solid (0.105 g, 43.2%).

HPLC purity: 98.16%

$^1$H NMR (400 MHz , DMSO-d6): $^1$H NMR (400 MHz, DMSO) δ 7.37 (s, 1H), 7.23 (s, 5H), 7.19 - 7.14 (m, 1H), 6.68 (d, J = 7.8 Hz, 1H), 6.63 (s, 1H), 4.50 (s, 1H), 3.98 (s, 1H), 3.52 (s, 2H), 3.10 (d, J = 9.5 Hz, 1H), 2.92 - 2.78 (m, 3H), 2.77 - 2.59 (m, 6H), 2.39 - 2.19 (m, 3H), 1.95 - 1.83 (m, 3H), 1.78 (d, J = 12.1 Hz, 1H), 1.66 (s, 1H), 1.54 (dd, J = 25.2, 9.6 Hz, 4H), 1.23 (t, J = 35.2 Hz, 17H), 0.86 (s, 1H), 0.64 (s, 3H).

**Example-47: Preparation of (7R,8R,13S,14S,17S)-17-hydroxy-13-methyl-7-(9-(4,4,5,5,5-pentafluoropentylsulfinyl)nonyl)-7,8,9, 11,12,13,14,15,16,17-decahydro-6H-cyclopenta[a]phenanthren-3-yl (2-methoxyethoxy)methyl hydrogen phosphate**

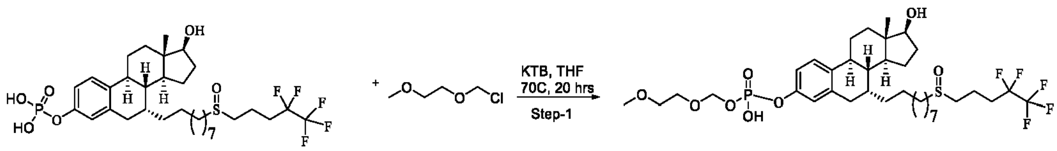


To a stirred solution (7R,8R,13S,14S,17S)-17-hydroxy-13-methyl-7-(9-(4,4,5,5,5-pentafluoropentylsulfinyl) nonyl)-7,8,9,11,12,13,14,15,16,17-decahydro-6H-cyclopenta[a]phenanthren-3-yl dihydrogen phosphate (0.2g) in tetrahydrofuran (1.5 mL) under nitrogen gas atmosphere at room temperature was added potassium tert-butoxide (0.06g) and 1-(chloromethoxy)-2-methoxyethane (0.04g), this reaction mixture was stirred at 70°C for 16-18 hr. The reaction was monitored on TLC. The reaction mixture was filtered and concentrated under reduced pressure. The crude thus isolated, was purified by PREP HPLC using mobile Phase A) 10 mm ammonium bicarbonate in water B) 100% acetonitrile. Product fraction was lyophilized to afford (7R,8R,13S,14S,17S)-17-hydroxy-13-methyl-7-(9-(4,4,5,5,5-pentafluoropentylsulfinyl) nonyl)-7,8,9,11,12,13,14,15,16,17-decahydro-6H-cyclopenta[a]phenanthren-3-yl (2-methoxyethoxy)methyl hydrogen phosphate as an off white foamy solid (0.045g, 20%).

HPLC purity: 99.12%

$^1$H NMR (400 MHz, DMSO-d6): δ 7.106-7.085 (d, J = 8.3 Hz, 1H), 6.832-6.806 (d, J = 10.9 Hz, 2H), 4.866-4.843 (d, J = 9.2 Hz, 2H), 4.509-4.497 (d, J = 4.9 Hz, 1H), 3.568-3.543 (m, 4H), 3.205 (s, 3H), 2.873-2.632 (m, 7H), 2.428-2.191 (m, 5H), 1.948-1.889 (m, 4H), 1.815-1.784 (d, J = 12.3 Hz, 1H), 1.685 (s, 1H), 1.66-1.605 (m, 2H), 1.547-1.495 (d, J = 9.8 Hz, 2H), 1.462-1.32 (m, 4H), 1.374-1.243 (s, 11H), 1.174 (s, 2H), 0.920 (s, 1H), 0.670 (s, 3H).

**Example-48: Preparation of (hydroxy((7R,8R,13S,14S,17S)-17-hydroxy-13-methyl-7-(9-(4,4,5,5,5-pentafluoropentylsulfinyl) nonyl)-7,8,9,11,12,13,14,15,16,17-decahydro-6H-cyclopenta[a]phenanthren-3-yloxy)phosphoryloxy)methyl isopropyl carbonate**

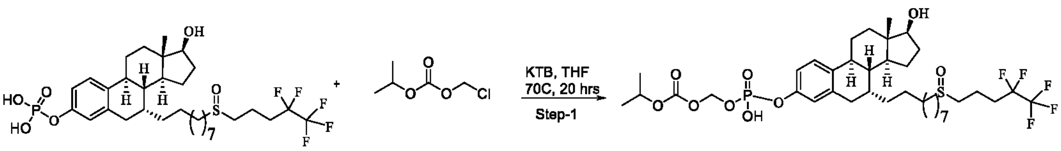


To a stirred solution of (7R,8R,13S,14S,17S)-17-hydroxy-13-methyl-7-(9-(4,4,5,5,5-pentafluoropentylsulfinyl) nonyl)-7,8,9,11,12,13,14,15,16,17-decahydro-6H-cyclopenta[a]phenanthren-3-yldihydrogen phosphate (0.170g) in Dimethyl formamide (4.0mL) under nitrogen gas atmosphere at room temperature was added N,N-Diisopropylethylamine (0.112g) and this reaction mixture was cooled to 0 °C. Chloromethyl isopropyl carbonate (0.113g) was added drop wise and this reaction mixture was stirred at 70 °C for overnight. The reaction was monitored on TLC and LCMS. The reaction mixture was Lyophilized for 2-3 hrs. The crude thus isolated, was purified by PREP HPLC using mobile Phase A) 10 mm ammonium bicarbonate in water B) 100% acetonitrile. Product fraction was lyophilized to afford (hydroxy((7R,8R, 13S,14S, 17S)-17-hydroxy-13-methyl-7-(9-(4,4,5,5,5-pentafluoropentyl sulfinyl)nonyl)-7,8,9,11,12, 13,14,15,16,17-decahydro-6H-cyclopenta[a]phenanthren-3-yloxy)phosphoryloxy) methyl isopropyl carbonate as a cream foamy solid (0.084g, 42.42%).

HPLC purity: 99.77%

$^1$H NMR (400 MHz, DMSO-d6): δ 7.107-7.086 (d, J = 8.4 Hz, 1H), 6.788 (s, 1H), 6.759 (s, 1H), 5.383-5.353 (d, J = 12.8 Hz,

2H), 4.724-4.679 (m, 1H), 4.515 (s, 1H), 3.589-3.534 (m, 2H), 3.078-3.023 (q, J = 14.8 Hz, 1H), 2.856-2.784 (m, 1H), 2.758 - 2.621 (m, 6H), 2..381-2.170 (m, 5H), 1.925 - 1.863 (m, 3H), 1.781-1.752 (d, , J = 11.6 Hz, 1H), 1.633-1.587 (m, 1H), 1.589-1.553 (m, 2H), 1.49-1.144 (m, 2H), 1.324-1.289 (m, 4H), 1.222 - 1.19 (m, 16H), 1.01-1.123 (q, J = 6.4 Hz, 6H), 0.854 (s,1H), 0.621 (s, 3H).

**Example-49: Preparation of (2S)-2-((2S)-2-(((7R,8R,13S,14S,17S)-17-hydroxy-13-methyl-7-(9-(4,4,5,5,5-pentafluoropentylsulfinyl) nonyl)-7,8,9,11,12,13,14,15,16,17-decahydro-6H-cyclopenta[a]phenanthren-3-yloxy)carbonylamino)-3-methyl butanamido)-3-phenylpropanoic acid**

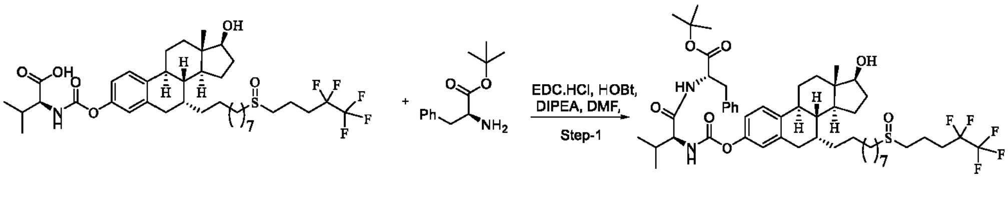


Step-1: Preparation of (2S)-tert-butyl 2-((2S)-2-(((7R,8R,13S,14S,17S)-17-hydroxy-13-methyl-7-(9-(4,4,5,5,5-penta fluoropentylsulfinyl)nonyl)-7,8,9,11,12,13,14,15,16,17-decahydro-6H-cyclopenta[a]phenanthren-3-yloxy) carbonylamino)-3-methylbutanamido)-3-phenylpropanoate

To a stirred solution of ((2S)-2-(((7R,8R,13S,14S,17S)-17-hydroxy-13-methyl-7-(9-(4,4,5,5,5-pentafluoropentyl sulfinyl)nonyl)-7,8,9,11,12,13,14,15,16,17-decahydro-6H-cyclopenta[a]phenanthren-3-yloxy)carbonylamino)-3-methylbutanoic acid (0.3g) in N,N-dimethyl formamide (1.0mL) under nitrogen gas atmosphere at room temperature was added Hydroxybenzotriazole (0.108g), EDC-HCl (0.084g) and this reaction mixture was cooled to 0 °C. (S)-tert-butyl 2-amino-3-phenylpropanoate hydrochloride (0.113g) and N,N-Diisopropylethylamine (0.155g) were added drop wise and this reaction mixture was stirred at room temperature for 2 days. The reaction was monitored on TLC and LCMS. The reaction mixture was diluted with ice-water (10mL) and extracted with ethyl acetate. The organic layer was dried over anhydrous $Na_2SO_4$ and concentrated under reduced pressure. The crude thus isolated, was purified by PREP HPLC using mobile Phase A) 10 mm ammonium bicarbonate in water B) 100% Acetonitrile. Product fraction was lyophilized to afford (2S)-tert-butyl 2-((2S)-2-(((7R,8R,13S,14S,17S)-17-hydroxy-13-methyl-7-(9-(4,4,5,5,5-pentafluoropentylsulfinyl) nonyl)-7,8,9,11,12,13,14,15, 16, 17-decahydro-6H-cyclopenta[a]phenanthren-3-yloxy)carbonylamino)-3-methylbutanamido)-3-phenylpropanoate as a white solid (0.105g, 27.56%).
HPLC purity: 99.23%

Step-2: Preparation of (2S)-2-((2S)-2-(((7R,8R,13S,14S,17S)-17-hydroxy-13-methyl-7-(9-(4,4,5,5,5-pentafluoropentylsulfinyl) nonyl)-7,8,9,11,12,13,14,15,16,17-decahydro-6H-cyclopenta[a]phenanthren-3-yloxy)carbonylamino)-3-methyl butanamido)-3-phenylpropanoic acid

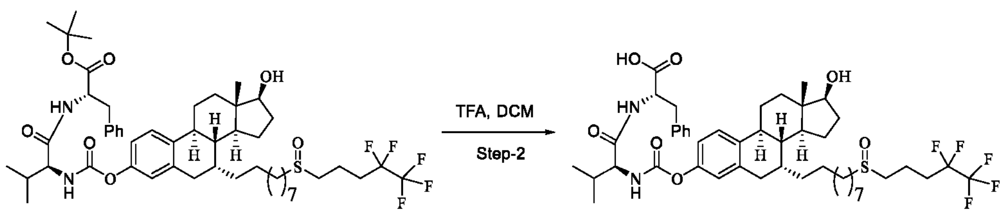


To a stirred solution of ((2S)-tert-butyl 2-((2S)-2-(((7R,8R,13S,14S,17S)-17-hydroxy-13-methyl-7-(9-(4,4,5,5,5-pentafluoropentylsulfinyl)nonyl)-7,8,9,11,12,13,14,15,16,17-decahydro-6H-cyclopenta[a]phenanthren-3-yloxy) carbonylamino)-3-methylbutanamido)-3-phenylpropanoate (0.1g,) in dichloromethane (2.0mL) under ice-cooling was added Trifluoroaceticacid (0.17 ml) under nitrogen gas atmosphere and this reaction was stirred at room temperature for overnight. The reaction was monitored on TLC and LCMS. The reaction mixture was concentrated under reduced pressure. The crude thus isolated was dissolved in tetrahydrofuran (2 mL), water (1 mL) and this reaction mixture again stirred at room temperature for overnight. The reaction was monitored on LCMS. The reaction mixture was concentrated under reduced pressure. The crude thus isolated, was purified by PREP HPLC using mobile Phase A) 10 mmol ammonium

bicarbonate in water B) 100% acetonitrile. Product fraction was lyophilized to (2S)-2-((2S)-2-(((7R,8R,13S,14S,17S)-17-hydroxy-13-methyl-7-(9-(4,4,5,5,5-pentafluoropentylsulfinyl)nonyl)-7,8,9,11,12,13,14,15, 16, 17-decahydro-6H-cyclopenta[a]phenanthren-3-yloxy)carbonylamino)-3-methylbutanamido)-3-phenylpropanoic acid as a white solid (0.035 g, 37.23%).

HPLC purity: 95.27%

$^1$H NMR (400 MHz , DMSO-d6): δ 7.942 (s, 1H), 7.775 (s, 1H), 7.268-7.249 (d, J = 8.3 Hz, 1H), 7.19 (bs, 5H), 6.811 (s, 1H), 6.747 (s, 1H), 4.527 (s, 1H), 4.271 (s, 1H), 3.796 (s, 1H), 3.543 (s, 1H), 3.040 (s, 1H), 2.932 (m, 1H), 2.823 (m, 2H), 2.726-2.672 (m, 4H), 2.395-2.263 (m, 5H), 1.958-1.878 (m, 4H), 1.815-1.789 (d, J = 10.2 Hz, 1H), 1.685 (s, 1H), 1.589 (s, 3H), 1.487 (s, 1H), 1.342 (s, 4H), 1.11-1.3 (m, 11H), 0.85 (s, 1H), 0.837-0.823 (d, J = 5.6 Hz, 6H), 0.666 (s, 3H).

**Example-50: Preparation of (2S)-1-(hydroxy((7R,8R,13S,14S,17S)-17-hydroxy-13-methyl-7-(9-(4,4,5,5,5-pentafluoropentyl sulfinyl)nonyl)-7,8,9,11,12,13,14,15,16,17-decahydro-6H-cyclopenta[a]phenanthren-3-yloxy)phosphoryl) pyrrolidine-2-carboxylic acid**

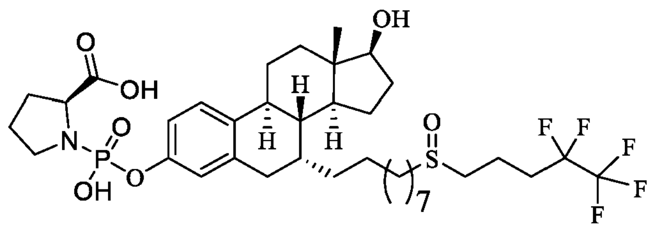

Step-1: Preparation of (2S)-methyl 1-(hydroxy((7R,8R,13S,14S,17S)-17-hydroxy-13-methyl-7-(9-(4,4,5,5,5-penta fluoropentylsulfinyl)nonyl)-7,8,9,11,12,13,14,15,16,17-decahydro-6H-cyclopenta[a]phenanthren-3-yloxy) phosphoryl) pyrrolidine-2-carboxylate

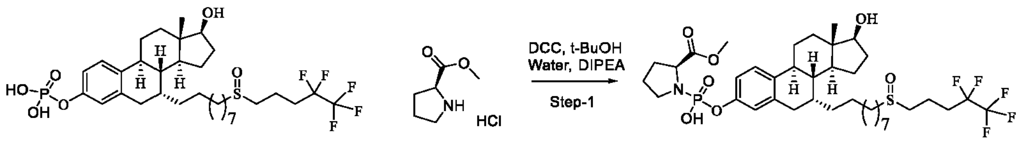


To a stirred solution (7R,8R,13S,14S,17S)-17-hydroxy-13-methyl-7-(9-(4,4,5,5,5-pentafluoropentylsulfinyl) nonyl)-7,8,9,11,12,13,14,15,16,17-decahydro-6H-cyclopenta[a]phenanthren-3-yl dihydrogen phosphate (0.3g) in t-Butanol (5 mL) under nitrogen gas atmosphere at room temperature was added (S)-methyl pyrrolidine-2-carboxylate hydrochloride (0.5g), N,N-di-isopropyl ethylamine (0.4 g) and N,N'-Dicyclohexylcarbodiimide (0.46g), this reaction mixture was stirred at room temperature for 16-18 hr. The reaction was monitored on TLC and LCMS. The reaction mixture was concentrated under reduced pressure. The crude thus isolated, to afford crude (2S)-methyl 1-(hydroxy((7R,8R,13S,14S,17S)-17-hydroxy-13-methyl-7-(9-(4,4,5,5,5-pentafluoropentylsulfinyl)nonyl)-7,8,9,11,12,13,14,15,16,17-decahydro-6H-cyclopenta[a]phenanthren-3-yloxy)phosphoryl)pyrrolidine-2-carboxylate as a light brown gum (0.85g, as crude).

LCMS: 27.87% (m/z: 799.5, [M+1]+, 821.5, [M+23]+

HPLC purity: 27.89%

Step-2: Preparation of (2S)-1-(hydroxy((7R,8R,138S,14S,178S)-17-hydroxy-13-methyl-7-(9-(4,4,5,5,5-pentafluoro pentylsulfinyl)nonyl)-7,8,9,11,12,13,14,15,16,17-decahydro-6H-cyclopenta[a]phenanthren-3-yloxy)phosphoryl) pyrrolidine-2-carboxylic acid

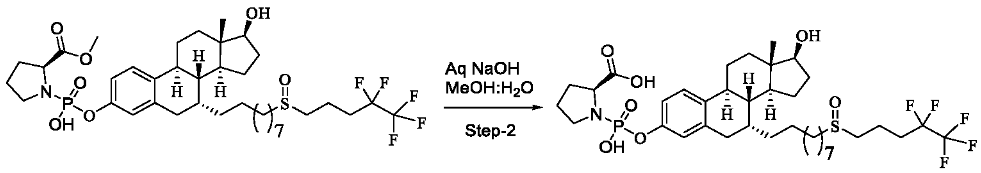


To a stirred solution To a stirred solution (7R(2S)-methyl 1-(hydroxy((7R,8R,13S,14S,17S)-17-hydroxy-13-methyl-7-(9-(4,4,5,5,5-penta fluoropentylsulfinyl)nonyl)-7,8,9,11,12,13,14,15, 16, 17-decahydro-6H-cyclopenta[a]phenanthren-3-yloxy) phosphoryl)pyrrolidine-2-carboxylate (0.85g, crude) in methanol (2 mL), water (4 ml) at room temperature was added sodium hydroxide (0.43g), this reaction mixture was stirred at room temperature for 20-24 hr. The reaction was monitored on TLC and LCMS. The reaction mixture was concentrated under lyophilizer. The crude thus isolated, to afford crude (2S)-1-(hydroxy((7R,8R,13S,14S,17S)-17-hydroxy-13-methyl-7-(9-(4,4,5,5,5-pentafluoropentylsulfinyl)nonyl)-7,8,9,11,12,13,14,15,16,17-decahydro-6H-cyclopenta[a]phenanthren-3-yloxy)phosphoryl)pyrrolidine-2-carboxylic acid (0.6g, as crude).

LCMS: 43.11% (m/z: 785.5, [M+1]+, 807.5, [M+23]+

HPLC: 43.11%

**Example-51: Preparation of acetic ((7R,8R,13S,14S,17S)-17-hydroxy-13-methyl-7-(9-(4,4,5,5,5-pentafluoropentylsulfinyl)nonyl)-7,8,9,11,12,13,14,15,16,17-decahydro-6H-cyclopenta[a]phenanthren-3-yl phosphoric) anhydride**

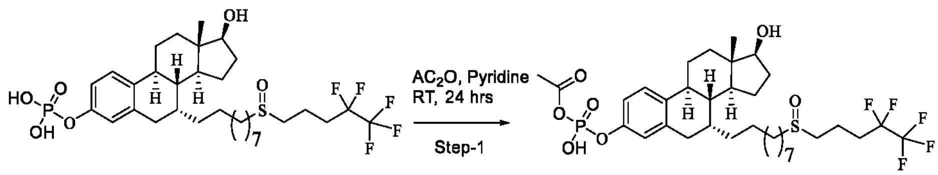


To a stirred solution (7R,8R,13S,14S,17S)-17-hydroxy-13-methyl-7-(9-(4,4,5,5,5-pentafluoropentylsulfinyl) nonyl)-7,8,9,11,12,13,14,15,16,17-decahydro-6H-cyclopenta[a]phenanthren-3-yl dihydrogen phosphate (0.15g) in pyridine (2 mL) under nitrogen gas atmosphere at room temperature was added Acetic anhydride (0.13g) this reaction mixture was stirred at room temperature for 16-18 hr. The reaction was monitored on TLC and LCMS. The reaction mixture was concentrated under reduced pressure. The crude thus isolated, was triturated with hexanes and concentrated to get to afford crude acetic ((7R,8R,13S,14S,17S)-17-hydroxy-13-methyl-7-(9-(4,4,5,5,5-pentafluoropentylsulfinyl)nonyl)-7,8,9,11,12,13,14,15, 16,17-decahydro-6H-cyclopenta[a]phenanthren-3-yl phosphoric) anhydride as a light brown gum (0.15g, as crude).

LCMS: 20.79% (m/z: 730.3, [M+1]+, 752.3, [M+23]+

HPLC: 20.79%

**Example-52: Pharmacokinetic study in mice**

The in vivo conversion of the prodrug to fulvestrant was evaluated in mice pharmacokinetics study in which the prodrug was administered orally to the mice. For comparison, a mice pharmacokinetics study with oral fulvestrant was performed. The study design and measured exposures of fulvestrant are summarized in Table 3. In brief, groups of three male Swiss Albino mice were orally administered (1) compound (I-t) in 90% PBS pH 7.4, 10% ethanol, or (2) Fulvestrant in 90% PBS pH 7.4, 10% ethanol. Blood samples were collected at multiple time points over 24 hours into a collection tube containing heparin. Plasma was separated from blood and subjected to protein precipitation with acetonitrile containing internal standard, vortexed, and centrifuged. From this, supernatant was separated and diluted with methanol: water (1:1) and subjected to LC-MS analysis for quantitation of compound (I-t), and fulvestrant. Compound (I-t) was not detected in plasma of mice at given dose.

**Table 3:*In vivo* exposure in mice**

| | | | Pharmacokinetic parameters of fulvestrant | | |
|---|---|---|---|---|---|
| **Test compound** | **Dose (mg/kg)** | **Fulvestrant equivalent (mg/kg)** | **Cmax (ng/mL)** | **$T_{max}$ (h)** | **Fulvestrant $AUC_{0\text{-}last}$ (ng·h/mL)** |
| compound (I-t) | 5 | 4.15 | 59.2 | 0.5 | 69.7 |
| Fulvestrant | 5 | 5 | ND | ND | ND |

## Claims

1. A compound of formula I-A

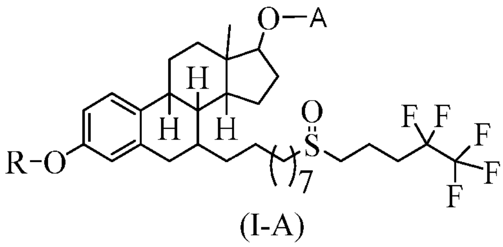

(I-A)

enantiomers, diastereomers, racemates, pharmaceutically acceptable salts or solvates thereof, wherein
A is selected from hydrogen,

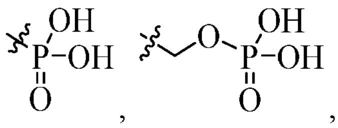

and
R is selected from group comprising of:

a)

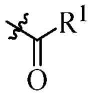

wherein $R^1$ is selected from group comprising of substituted aryl; optionally substituted alkyl, alkenyl, alkynyl, cycloalkyl, heteroaryl and $CR^2R^3$, wherein $R^2$ and $R^3$ are taken together along with the carbon to which they are attached to form a three to seven membered saturated, partially unsaturated or unsaturated heterocyclic ring in which up to 4 carbon atoms are replaced by heteroatoms chosen from the group consisting of O, S, or N and may optionally be substituted at a substitutable position with one or more $R^4$ radicals, wherein the $R^4$ radicals are independently selected at each occurrence from the group consisting of alkyl, cycloalkyl, heterocycloalkyl, aryl, heteroaryl, alkylcarbonyl, formyl, halo, alkylphosphate, phosphate, cyano, nitro, alkoxy, alkoxycarbonyl, alkenyl, alkynyl, alkylthio, and arylcarbonyl; wherein the $R^1$ substituted aryl, optionally substituted alkyl, alkenyl, alkynyl, cycloalkyl, heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;
b)

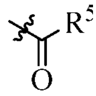

wherein $R^5$ is

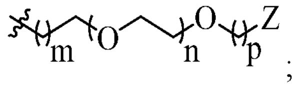

wherein m and p are independently selected from 0 to 5, n refers to degree of polymerization and is selected from 1 to 250 and Z is optionally substituted alkyl or cycloalkyl; wherein the Z is optionally substituted alkyl or cycloalkyl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;
c)

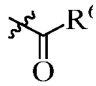

wherein $R^6$ is selected from $NH_2$, $NHR^7$ and $NR^8R^9$; wherein $R^7$ is selected from optionally substituted alkyl, alkenyl, alkynyl, cycloalkyl, heterocycloalkyl, aryl and heteroaryl; $R^8$ and $R^9$ are taken together along with the nitrogen to which they are attached to form a three to seven membered saturated, partially unsaturated or unsaturated heterocyclic ring and may optionally be substituted at a substitutable position with one or more $R^{10}$ radicals, wherein the $R^{10}$ radicals are independently selected at each occurrence from the group consisting of alkyl, alkylcarbonyl, formyl, halo, haloalkyl, alkylphosphate, phosphate, oxo, cyano, nitro, amino, alkoxy, alkoxycarbonyl, carboxyalkyl, hydroxyalkyl, alkenyl, alkynyl, alkylthio, cycloalkyl, aryl, aralkyl, heterocycloalkyl, alkylthioalkyl, arylcarbonyl, aralkylcarbonyl, and alkoxyalkyl; wherein the $R^7$ is optionally substituted alkyl, alkenyl, alkynyl, cycloalkyl, heterocycloalkyl, aryl and heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;
d)

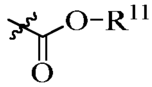

wherein $R^{11}$ is selected from group comprising of optionally substituted alkyl, alkenyl, alkynyl, cycloalkyl, heterocycloalkyl, aryl, and heteroaryl; wherein the $R^{11}$ is optionally substituted alkyl, alkenyl, alkynyl, cycloalkyl, heterocycloalkyl, aryl, and heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;
e)

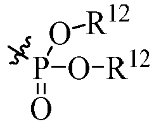

wherein each $R^{12}$ is selected independently from group comprising of hydrogen; optionally substituted alkyl,

aryl, acyl, and heteroaryl; wherein the $R^{12}$ is optionally substituted alkyl, aryl, acyl, and heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;
with a proviso that when A is hydrogen and one of $R^{12}$ substitution is selected from optionally substituted alkyl and aryl, then another $R^{12}$ substitution is hydrogen;
f)

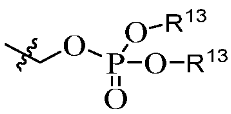

wherein each $R^{13}$ is selected independently from group comprising of hydrogen; optionally substituted alkyl, cycloalkyl, heterocycloalkyl, aryl, and heteroaryl; wherein the $R^{13}$ is optionally substituted alkyl, cycloalkyl, heterocycloalkyl, aryl, and heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;
g)

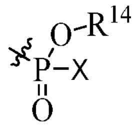

wherein $R^{14}$ is selected from group comprising of hydrogen; optionally substituted alkyl, aryl, acyl, heteroaryl; and X is selected from optionally substituted heterocycloalkyl; wherein the $R^{14}$ is optionally substituted alkyl, aryl, acyl, heteroaryl and X is optionally substituted heterocycloalkyl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro; and
h) hydrogen with the proviso that when R is hydrogen A is not hydrogen.

**2.** A compound of formula II

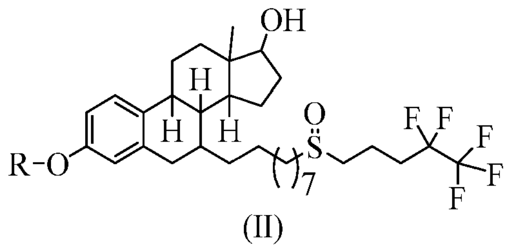

(II)

enantiomers, diastereomers, racemates, pharmaceutically acceptable salts or solvates thereof, wherein R is selected from group comprising of:

a)

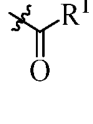

wherein $R^1$ is selected from group comprising of substituted aryl; optionally substituted alkyl, alkenyl, alkynyl, cycloalkyl, heteroaryl and $CR^2R^3$; wherein $R^2$ and $R^3$ are taken together along with the carbon to which they are attached to form a three to seven membered saturated, partially unsaturated or unsaturated heterocyclic ring in which up to 4 carbon atoms are replaced by heteroatoms chosen from the group consisting of O, S or N and may optionally be substituted at a substitutable position with one or more $R^4$ radicals, wherein the $R^4$ radicals are independently selected at each occurrence from the group consisting of alkyl, cycloalkyl, heterocycloalkyl, aryl, heteroaryl, alkylcarbonyl, formyl, halo, alkylphosphate, phosphate, cyano, nitro, alkoxy, alkoxycarbonyl, alkenyl, alkynyl, alkylthio, and arylcarbonyl; wherein the $R^1$ substituted aryl, optionally substituted alkyl, alkenyl, alkynyl, cycloalkyl, heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;

b)

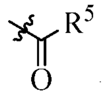

wherein $R^5$ is

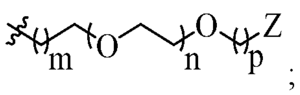

wherein m and p are independently selected from 0 to 5, n refers to degree of polymerization and is selected from 1 to 250 and Z is optionally substituted alkyl or cycloalkyl; wherein the Z is optionally substituted alkyl or cycloalkyl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;

c)

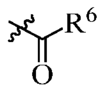

wherein $R^6$ is selected from $NH_2$, $NHR^7$ and $NR^8R^9$, wherein $R^7$ is selected from optionally substituted alkyl, alkenyl, alkynyl, cycloalkyl, heterocycloalkyl, aryl and heteroaryl; $R^8$ and $R^9$ are taken together along with the nitrogen to which they are attached to form a three to seven membered saturated, partially unsaturated or unsaturated heterocyclic ring and may optionally be substituted at a substitutable position with one or more $R^{10}$ radicals, wherein the $R^{10}$ radicals are independently selected at each occurrence from the group consisting of alkyl, alkylcarbonyl, formyl, halo, haloalkyl, alkylphosphate, phosphate, oxo, cyano, nitro, amino, alkoxy, alkoxycarbonyl, carboxyalkyl, hydroxyalkyl, alkenyl, alkynyl, alkylthio, cycloalkyl, aryl, aralkyl, heterocycloalkyl, alkylthioalkyl, arylcarbonyl, aralkylcarbonyl, and alkoxyalkyl; wherein the $R^7$ is optionally substituted alkyl, alkenyl, alkynyl, cycloalkyl, heterocycloalkyl, aryl and heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;

d)

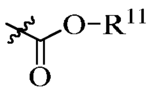

wherein $R^{11}$ is selected from group comprising of optionally substituted alkyl, alkenyl, alkynyl, cycloalkyl,

heterocycloalkyl, aryl, and heteroaryl; wherein the $R^{11}$ is optionally substituted alkyl, alkenyl, alkynyl, cycloalkyl, heterocycloalkyl, aryl, and heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;

e)

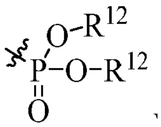

wherein each $R^{12}$ is selected independently from group comprising of hydrogen; optionally substituted alkyl, aryl, acyl, and heteroaryl; wherein the $R^{12}$ is optionally substituted alkyl, aryl, acyl, and heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro; with a proviso that when one of $R^{12}$ is selected from optionally substituted alkyl and aryl, then another $R^{12}$ substitution is hydrogen;

f)

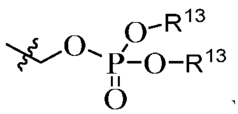

wherein $R^{13}$ is selected from group comprising of hydrogen; optionally substituted alkyl, cycloalkyl, heterocycloalkyl, aryl, and heteroaryl; wherein the $R^{13}$ is optionally substituted alkyl, cycloalkyl, heterocycloalkyl, aryl, and heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro; and

g)

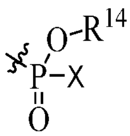

wherein $R^{14}$ is selected from group comprising of hydrogen; optionally substituted alkyl, aryl, acyl, heteroaryl; and X is optionally substituted heterocycloalkyl; wherein the $R^{14}$ is optionally substituted alkyl, aryl, acyl, heteroaryl and X is optionally substituted heterocycloalkyl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro.

**3.** The compound according to claim 2, wherein one or more $R^{10}$ radicals are heterocycloalkyl selected from pyrrolidine, piperidine, piperazine, morpholine, tetrahydrofuran, and tetrahydrothiophenyl.

**4.** The compound according to claim 2, comprising the compound **III** represented by following formulae:

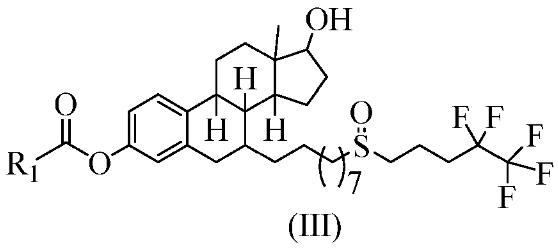

(III)

enantiomers, diastereomers, racemates, pharmaceutically acceptable salts or solvates thereof, wherein $R^1$ is selected from

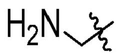,
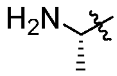,
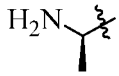,
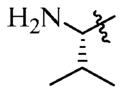,
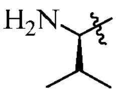,
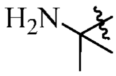,
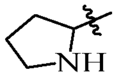,
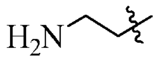,
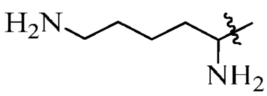,
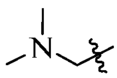,
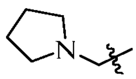,
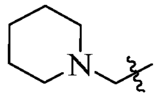,
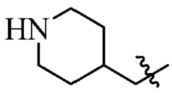,
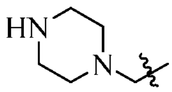,
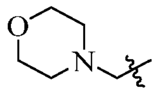,
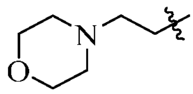,
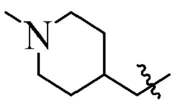,
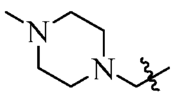,
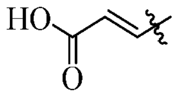,
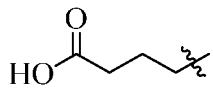,
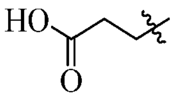,
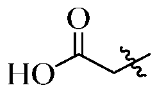,
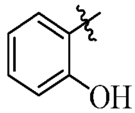,
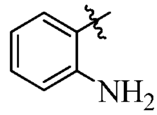,
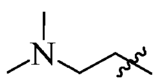.

**5.** The compound according to claim 2, comprising the compound IV represented by following formulae:

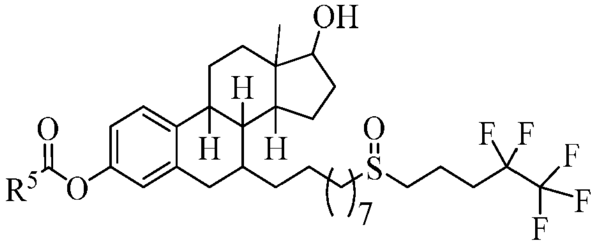

(IV)

enantiomers, diastereomers, racemates, pharmaceutically acceptable salts or solvates thereof, wherein $R^5$ is selected from

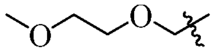,

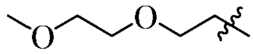,

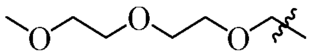,

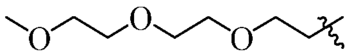,

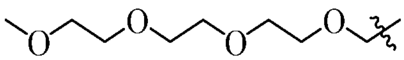,

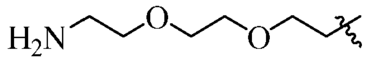,

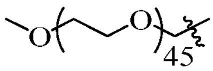,

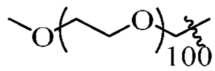,

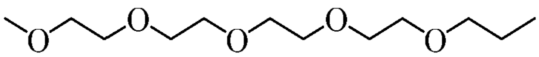,

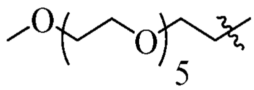,

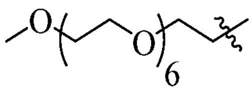.

**6.** The compound according to claim 2, comprising the compound V represented by following formulae:

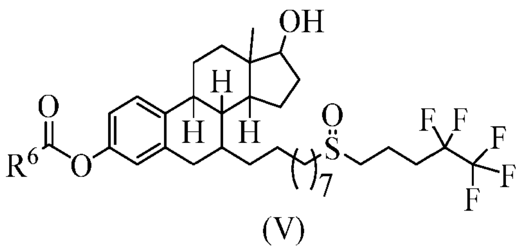

(V)

enantiomers, diastereomers, racemates, pharmaceutically acceptable salts or solvates thereof, wherein $R^6$ is selected from

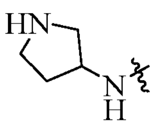,

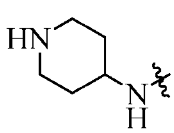,

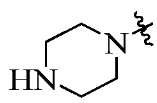,

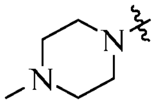,

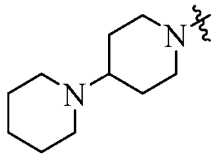,

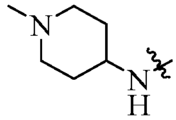,

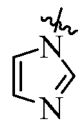, 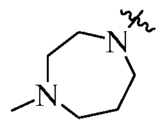.

7. The compound according to claim 2, comprising the compound VI represented by following formulae:

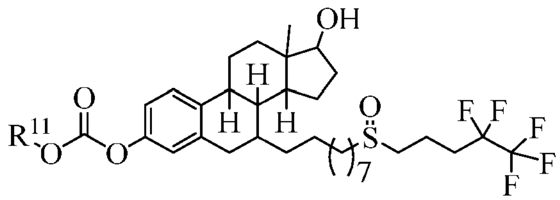

(VI)

enantiomers, diastereomers, racemates, pharmaceutically acceptable salts or solvates thereof, wherein $R^{11}$ is selected from

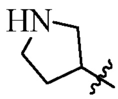, 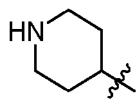, 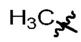,

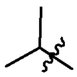, 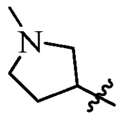, 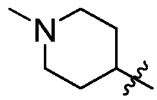.

8. The compound according to claim 2, comprising the compound VII represented by following formulae:

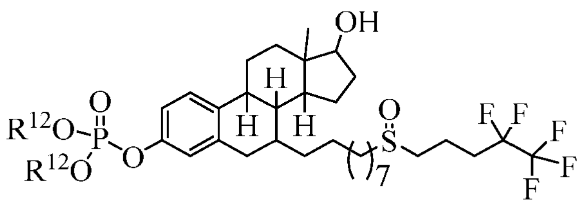

(VII)

enantiomers, diastereomers, racemates, pharmaceutically acceptable salts or solvates thereof, wherein each $R^{12}$ is independently selected from hydrogen, methyl, propyl, isopropyl, n-butyl,

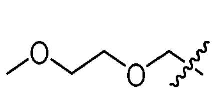, 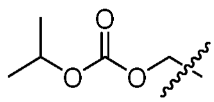;

with a proviso that when one of $R^{12}$ is selected from alkyl and aryl, then another $R^{12}$ substitution is hydrogen.

9. The compound according to claim 2, comprising the compound VIII represented by following formulae:

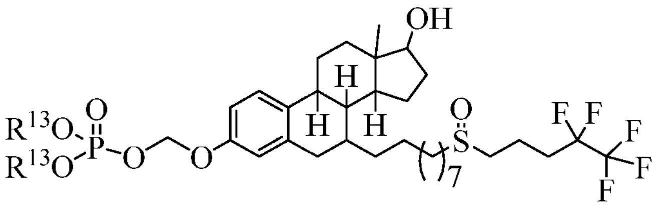

(VIII)

enantiomers, diastereomers, racemates, pharmaceutically acceptable salts or solvates thereof, wherein each $R^{13}$ is independently selected from hydrogen, methyl, ethyl, propyl, isopropyl, n-butyl, t-butyl, cyclopropyl, hexyl, phenyl, and 3-pyridyl.

**10.** The compound according to claim 2, comprising the compound XV represented by following formulae:

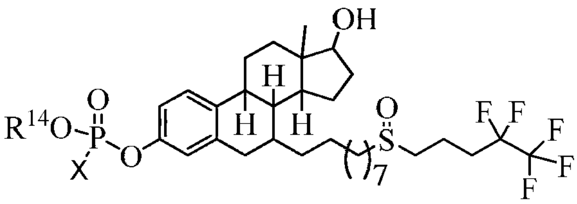

(XV)

enantiomers, diastereomers, racemates, pharmaceutically acceptable salts or solvates thereof, wherein $R^{14}$ is selected from hydrogen, methyl, propyl, isopropyl, n-butyl; and X is selected from optionally substituted aziridine, azetidine, pyrrolidine, piperidine, azepane, and azocane wherein the X is optionally substituted aziridine, azetidine, pyrrolidine, piperidine, azepane, and azocane is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro.

**11.** The compound according to claim 1 selected from the group comprising of

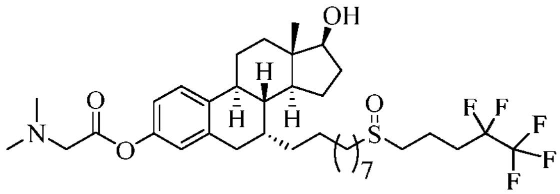

(I-a)

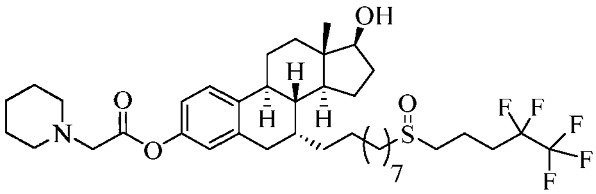

(I-b)

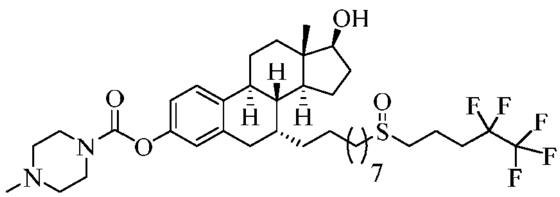

(I-c)

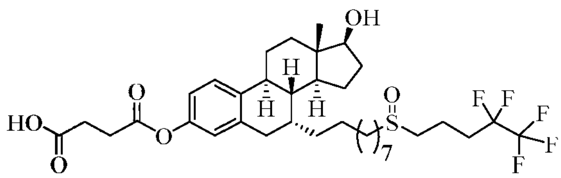

(I-d)

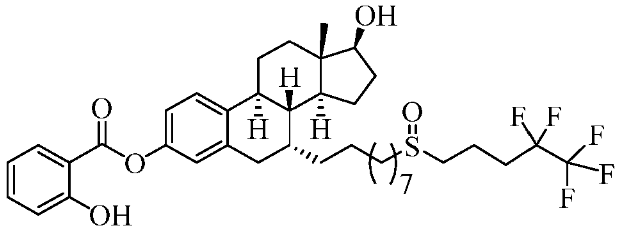

(I-e)

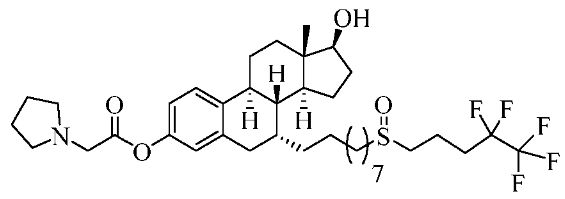

(I-g)

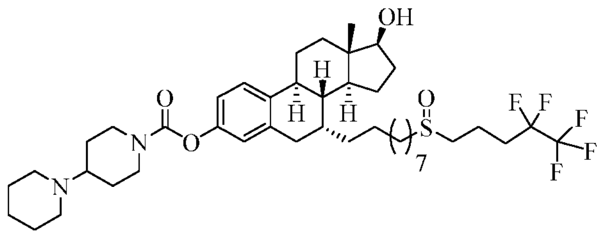

(I-h)

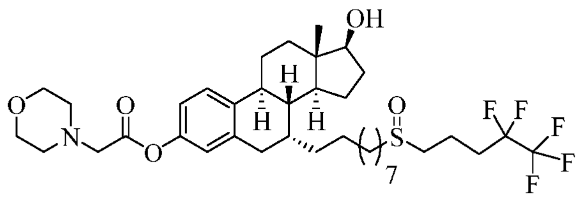

(I-i)

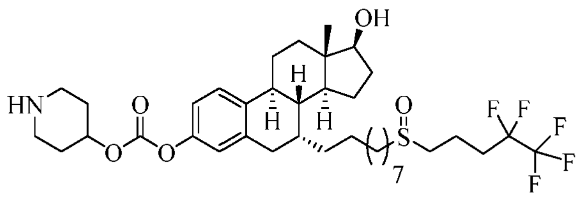

(I-j)

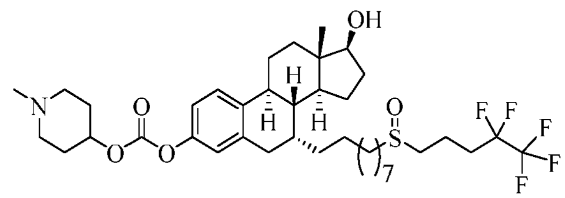

(I-k)

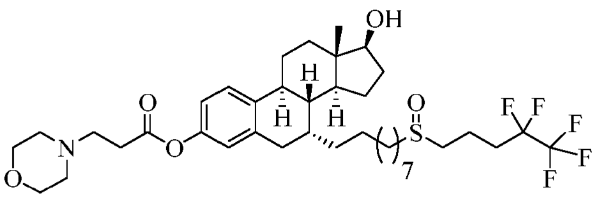

(I-l)

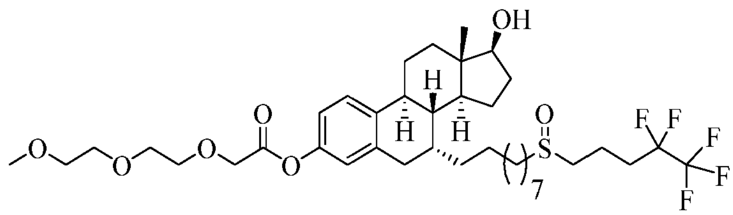

(I-m)

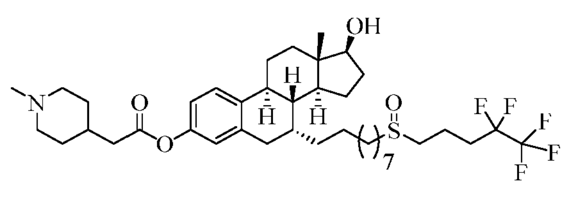

(I-n)

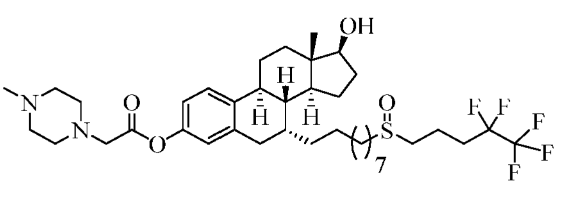

(I-o)

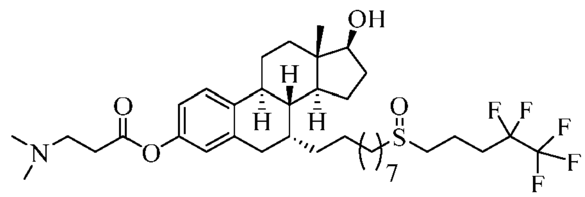

(I-p)

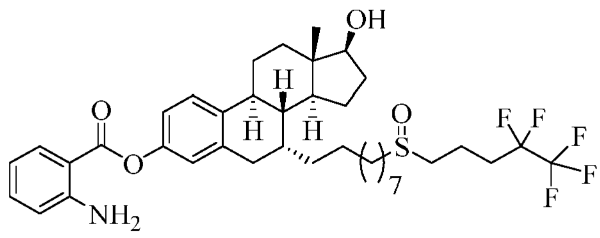

(I-q)

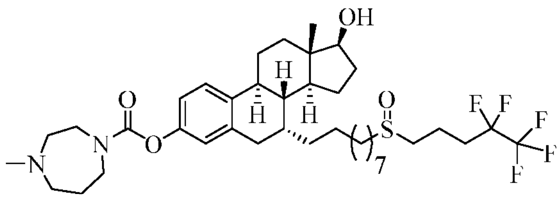

(I-r)

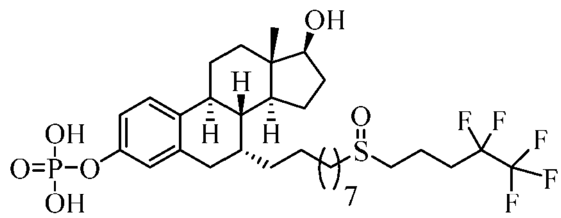

(I-s)

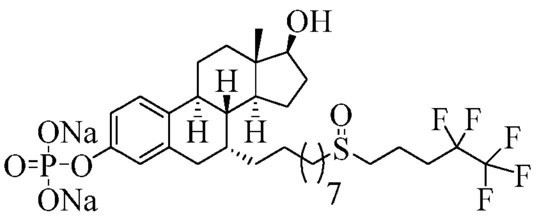

(I-t)

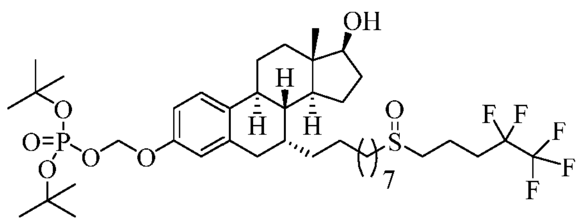

(I-u)

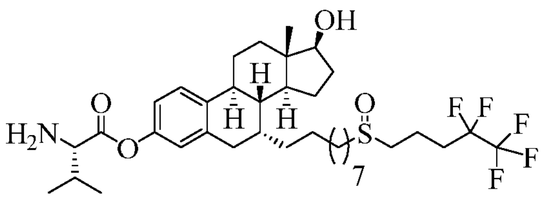

(I-v)

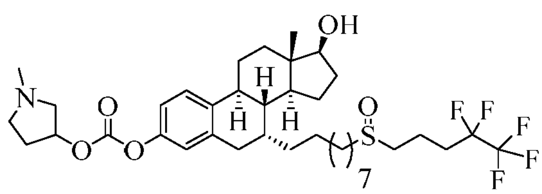

(I-x)

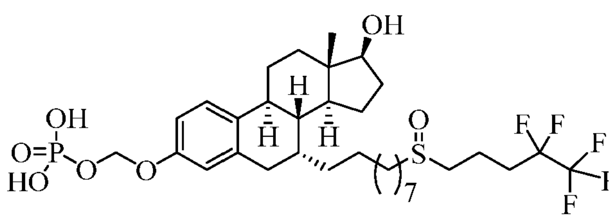

(I-y)

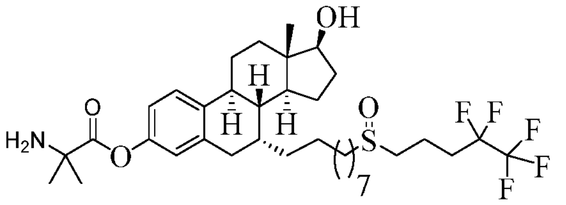

(I-z)

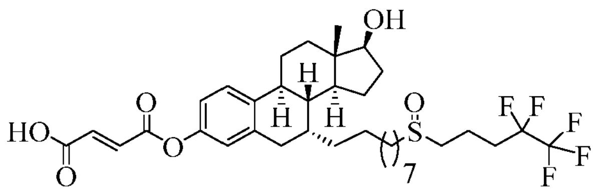

(I-aa)

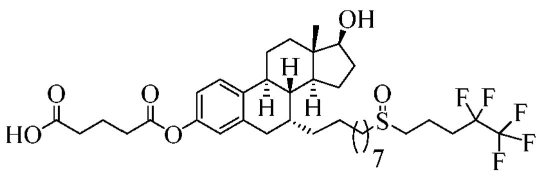

(I-ab)

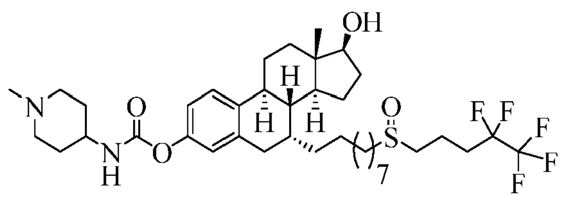

(I-ac)

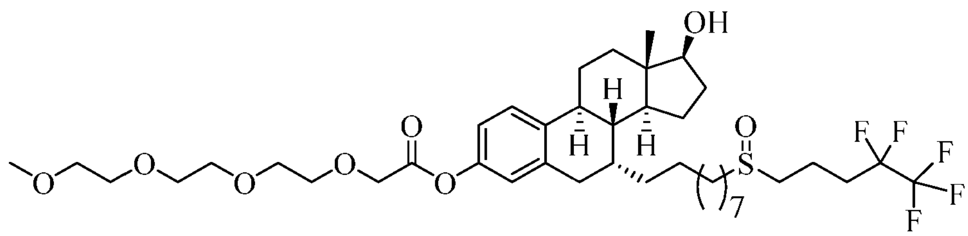

(I-ad)

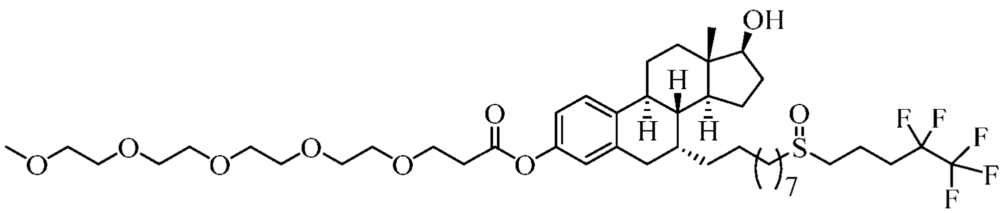

(I-ae)

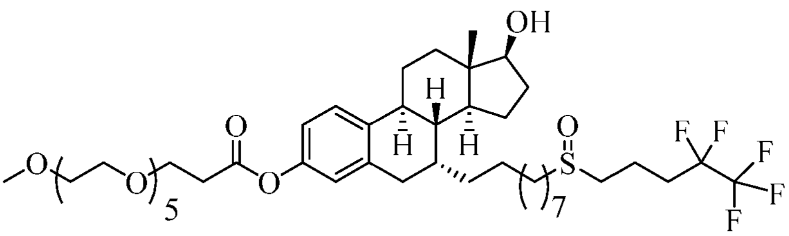

(I-af)

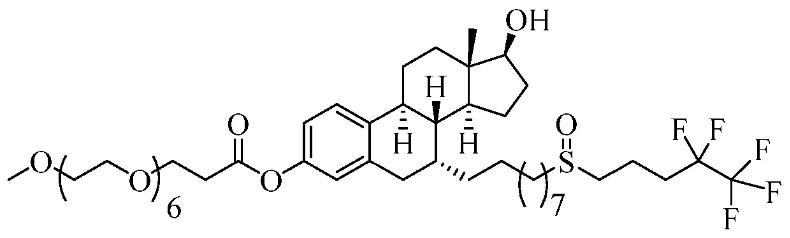

(I-ag)

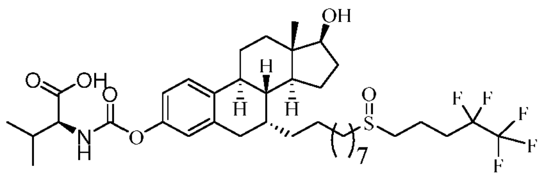

(I-ah)

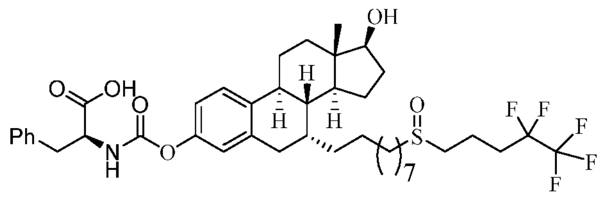

(I-ai)

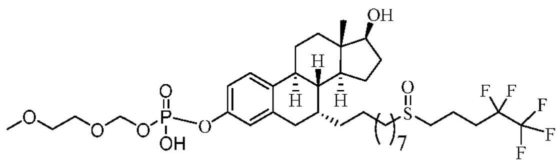

(I-aj)

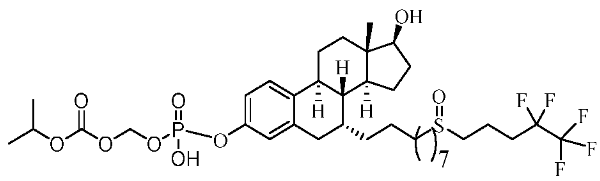

(I-ak)

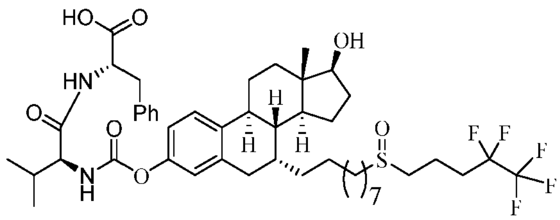

(I-al)

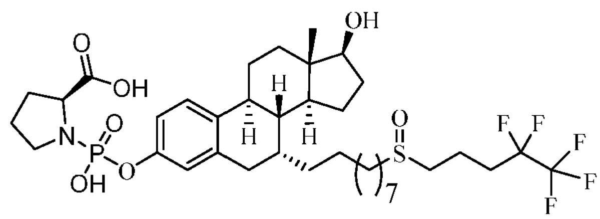

(I-am)

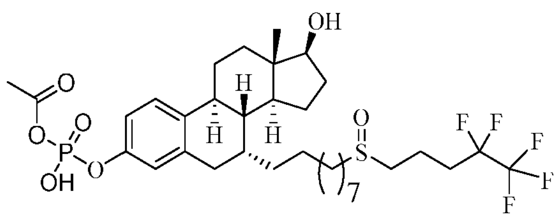

(I-an)

:

enantiomers, diastereomers, racemates, pharmaceutically acceptable salts or solvates thereof.

**12.** A pharmaceutical composition comprising a compound of formula I-A as claimed in claim 1, enantiomers, diastereomers, racemates, pharmaceutically acceptable salts or solvates thereof; and pharmaceutically acceptable excipients.

**13.** A pharmaceutical composition comprising a compound of formula II as claimed in claim 2, enantiomers, diastereomers, racemates, pharmaceutically acceptable salts or solvates thereof; and pharmaceutically acceptable excipients.

**14.** A compound of formula I-A

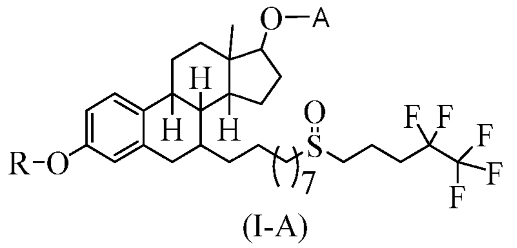

(I-A)

enantiomers, diastereomers, racemates, pharmaceutically acceptable salts or solvates thereof, for use in treating benign or malignant diseases of the breast or reproductive tract,
wherein
A is selected from hydrogen,

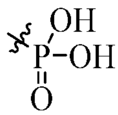

,

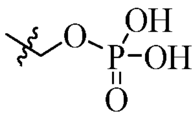

;

and
R is selected from group comprising of:

a)

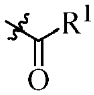

wherein $R^1$ is selected from group comprising of substituted aryl; optionally substituted alkyl, alkenyl, alkynyl, cycloalkyl, heteroaryl and $CR^2R^3$; wherein $R^2$ and $R^3$ are taken together along with the carbon to which they are attached to form a three to seven membered saturated, partially unsaturated or unsaturated heterocyclic ring in which up to 4 carbon atoms are replaced by heteroatoms chosen from the group consisting of O, S or N and may optionally be substituted at a substitutable position with one or more $R^4$ radicals, wherein the $R^4$ radicals are independently selected at each occurrence from the group consisting of alkyl, cycloalkyl, heterocycloalkyl, aryl, heteroaryl, alkylcarbonyl, formyl, halo, alkylphosphate, phosphate, cyano, nitro, alkoxy, alkoxycarbonyl, alkenyl, alkynyl, alkylthio and arylcarbonyl; wherein the $R^1$ substituted aryl, optionally substituted alkyl, alkenyl, alkynyl, cycloalkyl, heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;
b)

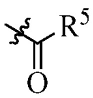

wherein $R^5$ is

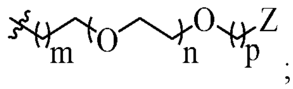

wherein m and p are independently selected from 0 to 5, n refers to degree of polymerization and is selected from 1 to 250 and Z is optionally substituted alkyl or cycloalkyl; wherein the Z is optionally substituted alkyl or cycloalkyl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;

c)

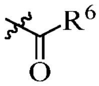

wherein $R^6$ is selected from $NH_2$, $NHR^7$ and $NR^8R^9$; wherein $R^7$ is selected from optionally substituted alkyl, alkenyl, alkynyl, cycloalkyl, heterocycloalkyl, aryl and heteroaryl; $R^8$ and $R^9$ are taken together along with the nitrogen to which they are attached to form a three to seven membered saturated, partially unsaturated or unsaturated heterocyclic ring and may optionally be substituted at a substitutable position with one or more $R^{10}$ radicals, wherein the $R^{10}$ radicals are independently selected at each occurrence from the group consisting of alkyl, alkylcarbonyl, formyl, halo, haloalkyl, alkylphosphate, phosphate, oxo, cyano, nitro, amino, alkoxy, alkoxycarbonyl, carboxyalkyl, hydroxyalkyl, alkenyl, alkynyl, alkylthio, cycloalkyl, aryl, aralkyl, heterocycloalkyl, alkylthioalkyl, arylcarbonyl, aralkylcarbonyl and alkoxyalkyl; wherein the $R^7$ is optionally substituted alkyl, alkenyl, alkynyl, cycloalkyl, heterocycloalkyl, aryl and heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;

d)

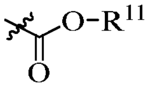

wherein $R^{11}$ is selected from group comprising of optionally substituted alkyl, alkenyl, alkynyl, cycloalkyl, heterocycloalkyl, aryl and heteroaryl; wherein the $R^{11}$ is optionally substituted alkyl, alkenyl, alkynyl, cycloalkyl, heterocycloalkyl, aryl, and heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;

e)

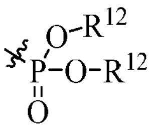

wherein each $R^{12}$ is selected independently from group comprising of hydrogen; optionally substituted alkyl, aryl, acyl and heteroaryl; wherein the $R^{12}$ is optionally substituted alkyl, aryl, acyl, and heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;

with a proviso that when A is hydrogen and one of $R^{12}$ substitution is selected from optionally substituted alkyl and aryl, then another $R^{12}$ substitution is hydrogen;

f)

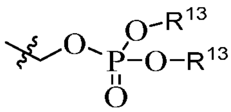

wherein each $R^{13}$ is selected independently from group comprising of hydrogen; optionally substituted alkyl, cycloalkyl, heterocycloalkyl, aryl and heteroaryl; wherein the $R^{13}$ is optionally substituted alkyl, cycloalkyl, heterocycloalkyl, aryl, and heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;

g)

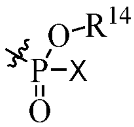

wherein $R^{14}$ is selected from group comprising of hydrogen; optionally substituted alkyl, aryl, acyl, heteroaryl; and X is optionally substituted heterocycloalkyl; wherein the $R^{14}$ is optionally substituted alkyl, aryl, acyl, heteroaryl and X is optionally substituted heterocycloalkyl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro; and

h) hydrogen with proviso that when R is hydrogen A is not hydrogen.

**15.** A compound of formula II

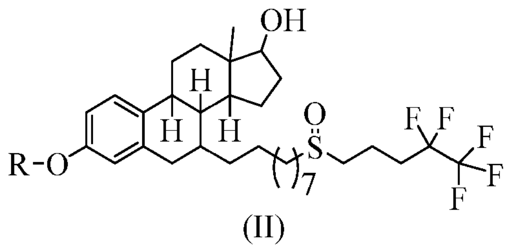

(II)

enantiomers, diastereomers, racemates, pharmaceutically acceptable salts or solvates thereof, for use in treating benign or malignant diseases of the breast or reproductive tract,
wherein R is selected from group comprising of:

a)

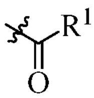

wherein $R^1$ is selected from group comprising of substituted aryl; optionally substituted alkyl, alkenyl, alkynyl, cycloalkyl, heteroaryl and $CR^2R^3$, wherein $R^2$ and $R^3$ are taken together along with the carbon to which they are attached to form a three to seven membered saturated, partially unsaturated or unsaturated heterocyclic ring in which up to 4 carbon atoms are replaced by heteroatoms chosen from the group consisting of O, S or N and may optionally be substituted at a substitutable position with one or more $R^4$ radicals, wherein the $R^4$

radicals are independently selected at each occurrence from the group consisting of alkyl, cycloalkyl, heterocycloalkyl, aryl, heteroaryl, alkylcarbonyl, formyl, halo, alkylphosphate, phosphate, cyano, nitro, alkoxy, alkoxycarbonyl, alkenyl, alkynyl, alkylthio, and arylcarbonyl; wherein the $R^1$ substituted aryl, optionally substituted alkyl, alkenyl, alkynyl, cycloalkyl, heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;
b)

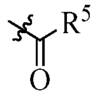

wherein $R^5$ is

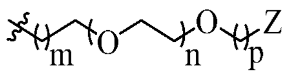
;

wherein m and p are independently selected from 0 to 5, n refers to degree of polymerization and is selected from 1 to 250 and Z is optionally substituted alkyl or cycloalkyl; wherein the Z is optionally substituted alkyl or cycloalkyl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;
c)

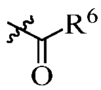

wherein $R^6$ is selected from $NH_2$, $NHR^7$ and $NR^8R^9$, wherein $R^7$ is selected from optionally substituted alkyl, alkenyl, alkynyl, cycloalkyl, heterocycloalkyl, aryl and heteroaryl; $R^8$ and $R^9$ are taken together along with the nitrogen to which they are attached to form a three to seven membered saturated, partially unsaturated or unsaturated heterocyclic ring and may optionally be substituted at a substitutable position with one or more $R^{10}$ radicals, wherein the $R^{10}$ radicals are independently selected at each occurrence from the group consisting of alkyl, alkylcarbonyl, formyl, halo, haloalkyl, alkylphosphate, phosphate, oxo, cyano, nitro, amino, alkoxy, alkoxycarbonyl, carboxyalkyl, hydroxyalkyl, alkenyl, alkynyl, alkylthio, cycloalkyl, aryl, aralkyl, heterocycloalkyl, alkylthioalkyl, arylcarbonyl, aralkylcarbonyl, and alkoxyalkyl; wherein the $R^7$ is optionally substituted alkyl, alkenyl, alkynyl, cycloalkyl, heterocycloalkyl, aryl and heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;
d)

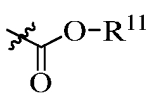

wherein $R^{11}$ is selected from group comprising of optionally substituted alkyl, alkenyl, alkynyl, cycloalkyl, heterocycloalkyl, aryl, and heteroaryl; wherein the $R^{11}$ is optionally substituted alkyl, alkenyl, alkynyl, cycloalkyl, heterocycloalkyl, aryl, and heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy,

acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;
e)

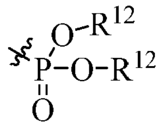

wherein each $R^{12}$ is selected independently from group comprising of hydrogen; optionally substituted alkyl, aryl, acyl and heteroaryl; wherein the $R^{12}$ is optionally substituted alkyl, aryl, acyl, and heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro;
with a proviso that when one of $R^{12}$ is selected from optionally substituted alkyl and aryl, then another $R^{12}$ substitution is hydrogen;
f)

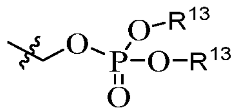

wherein each $R^{13}$ is selected independently from group comprising of hydrogen; optionally substituted alkyl, cycloalkyl, heterocycloalkyl, aryl, and heteroaryl; wherein the $R^{13}$ is optionally substituted alkyl, cycloalkyl, heterocycloalkyl, aryl, and heteroaryl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro; and
g)

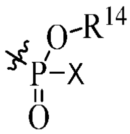

wherein $R^{14}$ is selected from group comprising of hydrogen; optionally substituted alkyl, aryl, acyl, heteroaryl; and X is optionally substituted heterocycloalkyl wherein the $R^{14}$ is optionally substituted alkyl, aryl, acyl, heteroaryl and X is optionally substituted heterocycloalkyl is substituted at a substitutable position with one or more radicals are independently selected at each occurrence from the group consisting of C1-C8-alkyl, C2-C8-alkenyl, C2-C8-alkynyl, cycloalkyl, heterocycloalkyl, aryl, heteroaryl, amino, alkylamino, acyl, acyloxy, acylamino, aminocarbonyl, alkoxycarbonyl, alkoxyalkyloxy, alkoxycarbonyloxy, carbamate, sulfinyl, sulfonyl, alkoxy, sulfanyl, halogen, carboxy, trihalomethyl, cyano, hydroxy, mercapto, nitro.

**16.** A composition for the use in the treatment of benign or malignant diseases of the breast or reproductive tract comprising at least one of the compound formula I-A defined in claim 14; and enantiomers, diastereomers, racemates, pharmaceutically acceptable salts or solvates thereof.

**17.** A composition for use in the treatment of benign or malignant diseases of the breast or reproductive tract comprising at least one of the compound formula II defined in claim 15; and enantiomers, diastereomers, racemates, pharmaceutically acceptable salts or solvates thereof.

**Patentansprüche**

1. Verbindung von Formel I-A

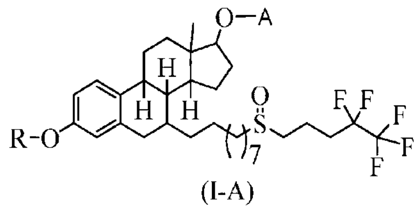

(I-A)

Enantiomere, Diastereomere, Racemate, pharmazeutisch verträgliche Salze oder Solvate davon, wobei A ausgewählt ist aus Wasserstoff,

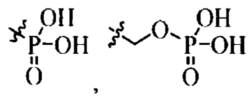

und
R ausgewählt ist aus einer Gruppe, umfassend:

a)

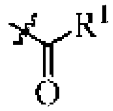

,

wobei $R^1$ ausgewählt ist aus einer Gruppe, umfassend substituiertes Aryl; optional substituiertes Alkyl, Alkenyl, Alkinyl, Cycloalkyl, Heteroaryl und $CR^2R^3$, wobei $R^2$ und $R^3$ zusammen mit dem Kohlenstoff genommen werden, an den sie gebunden sind, um einen drei- bis siebengliedrigen gesättigten, teilweise ungesättigten oder ungesättigten heterocyclischen Ring auszubilden, in dem bis zu 4 Kohlenstoffatome durch Heteroatome ersetzt sind, die gewählt sind aus der Gruppe, bestehend aus O, S oder N, und optional an einer substituierbaren Position mit einem oder mehreren $R^4$-Radikalen substituiert sein können, wobei die $R^4$-Radikale bei jedem Auftreten unabhängig ausgewählt sind aus der Gruppe, bestehend aus Alkyl, Cycloalkyl, Heterocycloalkyl, Aryl, Heteroaryl, Alkylcarbonyl, Formyl, Halogen, Alkylphosphat, Phosphat, Cyano, Nitro, Alkoxy, Alkoxycarbonyl, Alkenyl, Alkinyl, Alkylthio und Arylcarbonyl; wobei das $R^1$-substituierte Aryl, optional substituierte Alkyl, Alkenyl, Alkinyl, Cycloalkyl, Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalomethyl, Cyano, Hydroxy, Mercapto, Nitro;
b)

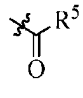

,

wobei $R^5$

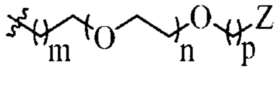

ist; wobei m und p unabhängig ausgewählt sind aus 0 bis 5, sich n auf einen Polymerisationsgrad bezieht und ausgewählt ist aus 1 bis 250 und Z optional substituiertes Alkyl oder Cycloalkyl ist; wobei das Z optional

substituiertes Alkyl oder Cycloalkyl ist, das an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalomethyl, Cyano, Hydroxy, Mercapto, Nitro;

c)

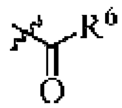
,

wobei $R^6$ ausgewählt ist aus $NH_2$, $NHR^7$ und $NR^8R^9$; wobei $R^7$ ausgewählt ist aus optional substituiertem Alkyl, Alkenyl, Alkinyl, Cycloalkyl, Heterocycloalkyl, Aryl und Heteroaryl; $R^8$ und $R^9$ zusammen mit dem Stickstoff genommen werden, an den sie gebunden sind, um einen drei- bis siebengliedrigen gesättigten, teilweise ungesättigten oder ungesättigten heterocyclischen Ring auszubilden und optional an einer substituierbaren Position mit einem oder mehreren $R^{10}$-Radikalen substituiert sein können, wobei die $R^{10}$-Radikale bei jedem Auftreten unabhängig ausgewählt sind aus der Gruppe, bestehend aus Alkyl, Alkylcarbonyl, Formyl, Halogen, Halogenalkyl, Alkylphosphat, Phosphat, Oxo, Cyano, Nitro, Amino, Alkoxy, Alkoxycarbonyl, Carboxyalkyl, Hydroxyalkyl, Alkenyl, Alkinyl, Alkylthio, Cycloalkyl, Aryl, Aralkyl, Heterocycloalkyl, Alkylthioalkyl, Arylcarbonyl, Aralkylcarbonyl und Alkoxyalkyl; wobei das $R^7$ optional substituiertes Alkyl, Alkenyl, Alkinyl, Cycloalkyl, Heterocycloalkyl, Aryl ist und Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalogenmethyl, Cyano, Hydroxy, Mercapto, Nitro;

d)

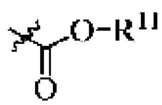
,

wobei $R^{11}$ ausgewählt ist aus einer Gruppe, umfassend optional substituiertes Alkyl, Alkenyl, Alkinyl, Cycloalkyl, Heterocycloalkyl, Aryl und Heteroaryl; wobei das $R^{11}$ optional substituiertes Alkyl, Alkenyl, Alkinyl, Cycloalkyl, Heterocycloalkyl, Aryl ist und Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalogenmethyl, Cyano, Hydroxy, Mercapto, Nitro;

e)

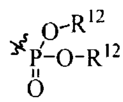
,

wobei jedes $R^{12}$ unabhängig ausgewählt ist aus einer Gruppe, umfassend Wasserstoff; optional substituiertes Alkyl, Aryl, Acyl und Heteroaryl; wobei das $R^{12}$ optional substituiertes Alkyl, Aryl, Acyl ist und Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalogenmethyl, Cyano, Hydroxy, Mercapto, Nitro;

mit einer Bedingung, dass, wenn A Wasserstoff ist und eine der $R^{12}$-Substitution ausgewählt ist aus optional substituiertem Alkyl und Aryl, dann eine andere $R^{12}$-Substitution Wasserstoff ist;

f)

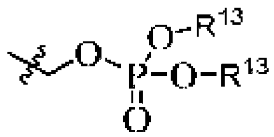

,

wobei jedes $R^{13}$ unabhängig ausgewählt ist aus einer Gruppe, umfassend Wasserstoff; optional substituiertes Alkyl, Cycloalkyl, Heterocycloalkyl, Aryl und Heteroaryl; wobei das $R^{13}$ optional substituiertes Alkyl, Cycloalkyl, Heterocycloalkyl, Aryl ist und Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalogenmethyl, Cyano, Hydroxy, Mercapto, Nitro;

g)

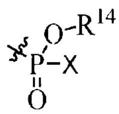

,

wobei $R^{14}$ ausgewählt ist aus einer Gruppe, umfassend Wasserstoff; optional substituiertes Alkyl, Aryl, Acyl, Heteroaryl; und X ausgewählt ist aus optional substituiertem Heterocycloalkyl; wobei das $R^{14}$ optional substituiertes Alkyl, Aryl, Acyl, Heteroaryl ist und X optional substituiertes Heterocycloalkyl ist, das an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalomethyl, Cyano, Hydroxy, Mercapto, Nitro; und

h) Wasserstoff mit der Bedingung, dass, wenn R Wasserstoff ist, A nicht Wasserstoff ist.

**2.** Verbindung von Formel II

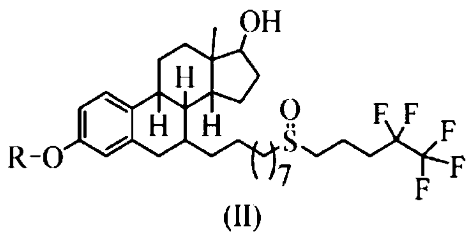

(II)

Enantiomere, Diastereomere, Racemate, pharmazeutisch verträgliche Salze oder Solvate davon, wobei R ausgewählt ist aus einer Gruppe, umfassend:

a)

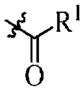

,

wobei $R^1$ ausgewählt ist aus einer Gruppe, umfassend substituiertes Aryl; optional substituiertes Alkyl, Alkenyl, Alkinyl, Cycloalkyl, Heteroaryl und $CR^2R^3$; wobei $R^2$ und $R^3$ zusammen mit dem Kohlenstoff genommen werden, an den sie gebunden sind, um einen drei- bis siebengliedrigen gesättigten, teilweise ungesättigten oder ungesättigten heterocyclischen Ring auszubilden, in dem bis zu 4 Kohlenstoffatome durch Heteroatome ersetzt sind, die gewählt sind aus der Gruppe, bestehend aus O, S oder N, und optional an einer substituierbaren Position mit

einem oder mehreren $R^4$-Radikalen substituiert sein können, wobei die $R^4$-Radikale bei jedem Auftreten unabhängig ausgewählt sind aus der Gruppe, bestehend aus Alkyl, Cycloalkyl, Heterocycloalkyl, Aryl, Heteroaryl, Alkylcarbonyl, Formyl, Halogen, Alkylphosphat, Phosphat, Cyano, Nitro, Alkoxy, Alkoxycarbonyl, Alkenyl, Alkinyl, Alkylthio und Arylcarbonyl; wobei das $R^1$-substituierte Aryl, optional substituierte Alkyl, Alkenyl, Alkinyl, Cycloalkyl, Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalomethyl, Cyano, Hydroxy, Mercapto, Nitro;

b)

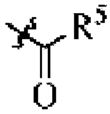

,

wobei $R^5$

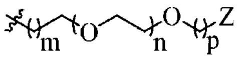

ist; wobei m und p unabhängig ausgewählt sind aus 0 bis 5, sich n auf einen Polymerisationsgrad bezieht und ausgewählt ist aus 1 bis 250 und Z optional substituiertes Alkyl oder Cycloalkyl ist; wobei das Z optional substituiertes Alkyl oder Cycloalkyl ist, das an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalomethyl, Cyano, Hydroxy, Mercapto, Nitro;

c)

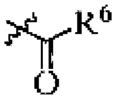

,

wobei $R^6$ ausgewählt ist aus $NH_2$, $NHR^7$ und $NR^8R^9$, wobei $R^7$ ausgewählt ist aus optional substituiertem Alkyl, Alkenyl, Alkinyl, Cycloalkyl, Heterocycloalkyl, Aryl und Heteroaryl; $R^8$ und $R^9$ zusammen mit dem Stickstoff genommen werden, an den sie gebunden sind, um einen drei- bis siebengliedrigen gesättigten, teilweise ungesättigten oder ungesättigten heterocyclischen Ring auszubilden und optional an einer substituierbaren Position mit einem oder mehreren $R^{10}$-Radikalen substituiert sein können, wobei die $R^{10}$-Radikale bei jedem Auftreten unabhängig ausgewählt sind aus der Gruppe, bestehend aus Alkyl, Alkylcarbonyl, Formyl, Halogen, Halogenalkyl, Alkylphosphat, Phosphat, Oxo, Cyano, Nitro, Amino, Alkoxy, Alkoxycarbonyl, Carboxyalkyl, Hydroxyalkyl, Alkenyl, Alkinyl, Alkylthio, Cycloalkyl, Aryl, Aralkyl, Heterocycloalkyl, Alkylthioalkyl, Arylcarbonyl, Aralkylcarbonyl und Alkoxyalkyl; wobei das $R^7$ optional substituiertes Alkyl, Alkenyl, Alkinyl, Cycloalkyl, Heterocycloalkyl, Aryl ist und Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalogenmethyl, Cyano, Hydroxy, Mercapto, Nitro;

d)

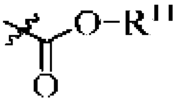

,

wobei $R^{11}$ ausgewählt ist aus einer Gruppe, umfassend optional substituiertes Alkyl, Alkenyl, Alkinyl, Cycloalkyl, Heterocycloalkyl, Aryl und Heteroaryl; wobei das $R^{11}$ optional substituiertes Alkyl, Alkenyl, Alkinyl, Cycloalkyl,

Heterocycloalkyl, Aryl ist und Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalogenmethyl, Cyano, Hydroxy, Mercapto, Nitro;

e)

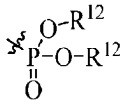

,

wobei jedes $R^{12}$ unabhängig ausgewählt ist aus einer Gruppe, umfassend Wasserstoff; optional substituiertes Alkyl, Aryl, Acyl und Heteroaryl; wobei das $R^{12}$ optional substituiertes Alkyl, Aryl, Acyl ist und Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalogenmethyl, Cyano, Hydroxy, Mercapto, Nitro;

mit einer Bedingung, dass, wenn eines von $R^{12}$ ausgewählt ist aus optional substituiertem Alkyl und Aryl, dann eine andere $R^{12}$-Substitution Wasserstoff ist;

f)

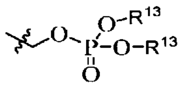

,

wobei $R^{13}$ ausgewählt ist aus einer Gruppe, umfassend Wasserstoff; optional substituiertes Alkyl, Cycloalkyl, Heterocycloalkyl, Aryl und Heteroaryl; wobei das $R^{13}$ optional substituiertes Alkyl, Cycloalkyl, Heterocycloalkyl, Aryl ist und Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalogenmethyl, Cyano, Hydroxy, Mercapto, Nitro; und

g)

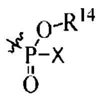

,

wobei $R^{14}$ ausgewählt ist aus einer Gruppe, umfassend Wasserstoff; optional substituiertes Alkyl, Aryl, Acyl, Heteroaryl; und X optional substituiertes Heterocycloalkyl ist; wobei das $R^{14}$ optional substituiertes Alkyl, Aryl, Acyl, Heteroaryl ist und X optional substituiertes Heterocycloalkyl ist, das an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalomethyl, Cyano, Hydroxy, Mercapto, Nitro.

**3.** Verbindung nach Anspruch 2, wobei ein oder mehrere $R^{10}$-Radikale Heterocycloalkyl sind, ausgewählt aus Pyrrolidin, Piperidin, Piperazin, Morpholin, Tetrahydrofuran und Tetrahydrothiophenyl.

**4.** Verbindung nach Anspruch 2, umfassend die Verbindung III, dargestellt durch folgende Formeln:

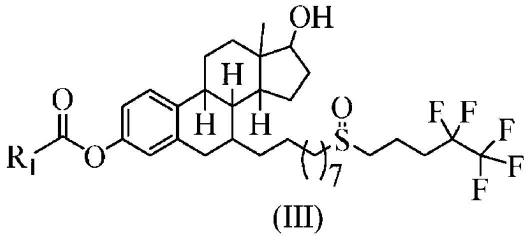

(III)

Enantiomere, Diastereomere, Racemate, pharmazeutisch verträgliche Salze oder Solvate davon, wobei $R^1$ ausgewählt ist aus

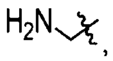, 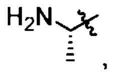, 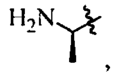, 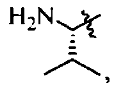,

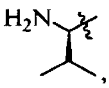, 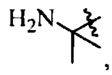, 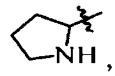, 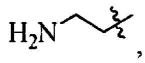,

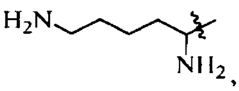, 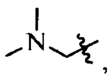, 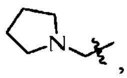, 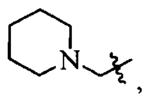,

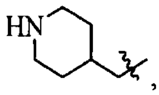, 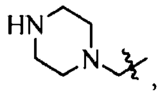, 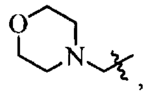, 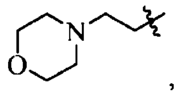,

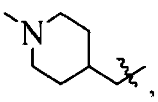, 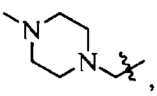, 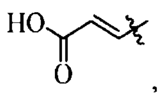, 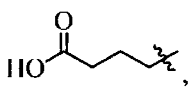,

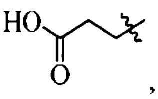, 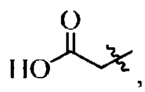, 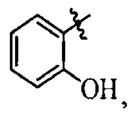, 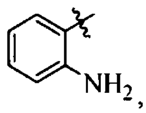,

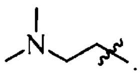.

5. Verbindung nach Anspruch 2, umfassend die Verbindung IV, dargestellt durch folgende Formeln:

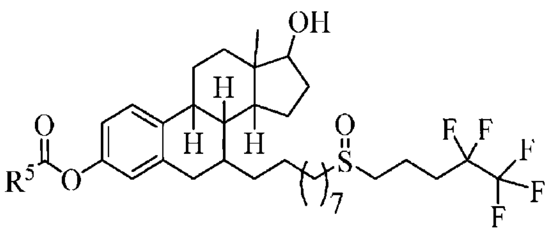

(IV)

Enantiomere, Diastereomere, Racemate, pharmazeutisch verträgliche Salze oder Solvate davon, wobei $R^5$ ausgewählt ist aus

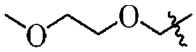, 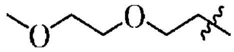,

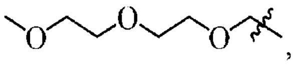, 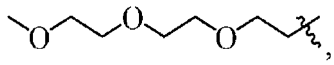,

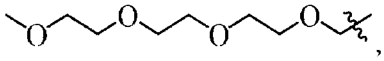, 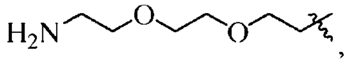,

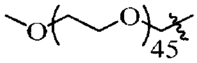, 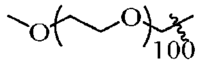,

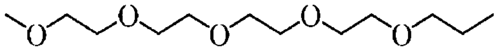, 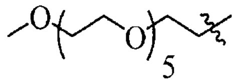,

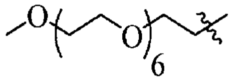.

**6.** Verbindung nach Anspruch 2, umfassend die Verbindung V, dargestellt durch folgende Formeln:

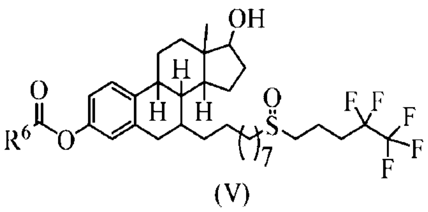

(V)

Enantiomere, Diastereomere, Racemate, pharmazeutisch verträgliche Salze oder Solvate davon, wobei $R^6$ ausgewählt ist aus

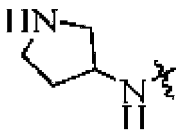, 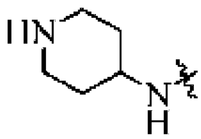, 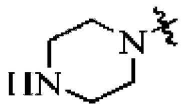,

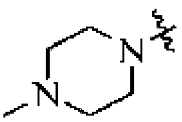, 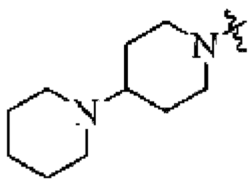, 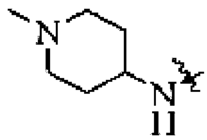,

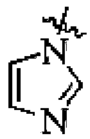, 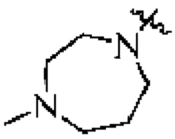.

**7.** Verbindung nach Anspruch 2, umfassend die Verbindung VI, dargestellt durch folgende Formeln:

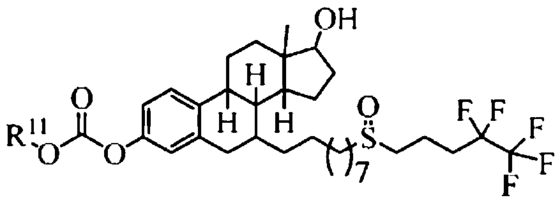

(VI)

Enantiomere, Diastereomere, Racemate, pharmazeutisch verträgliche Salze oder Solvate davon, wobei $R^{11}$ ausgewählt ist aus

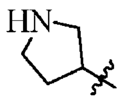,
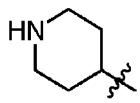,
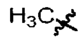,
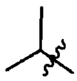,
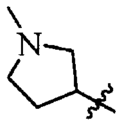,
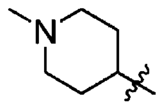.

**8.** Verbindung nach Anspruch 2, umfassend die Verbindung VII, dargestellt durch folgende Formeln:

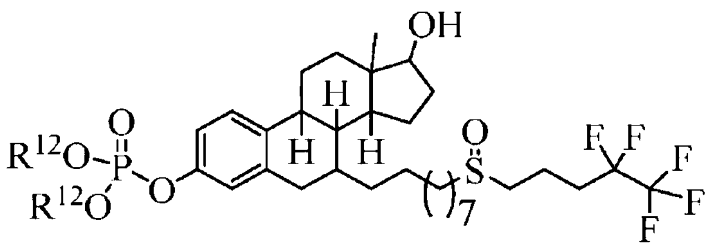

(VII)

Enantiomere, Diastereomere, Racemate, pharmazeutisch verträgliche Salze oder Solvate davon, wobei jedes $R^{12}$ unabhängig ausgewählt ist aus Wasserstoff, Methyl, Propyl, Isopropyl, n-Butyl,

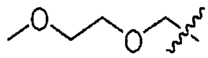,
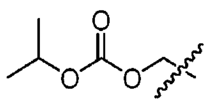;

mit einer Bedingung, dass, wenn eines von $R^{12}$ ausgewählt ist aus Alkyl und Aryl, dann eine andere $R^{12}$-Substitution Wasserstoff ist.

**9.** Verbindung nach Anspruch 2, umfassend die Verbindung VIII, dargestellt durch folgende Formeln:

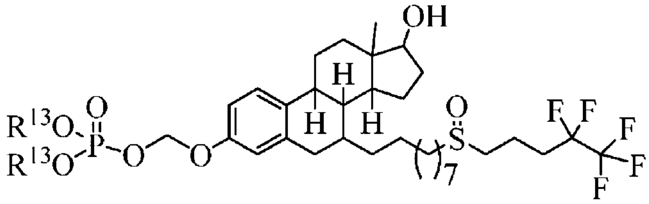

(VIII)

Enantiomere, Diastereomere, Racemate, pharmazeutisch verträgliche Salze oder Solvate davon, wobei jedes $R^{13}$ unabhängig ausgewählt ist aus Wasserstoff, Methyl, Ethyl, Propyl, Isopropyl, n-Butyl, t-Butyl, Cyclopropyl, Hexyl, Phenyl und 3-Pyridyl.

**10.** Verbindung nach Anspruch 2, umfassend die Verbindung XV, dargestellt durch folgende Formeln:

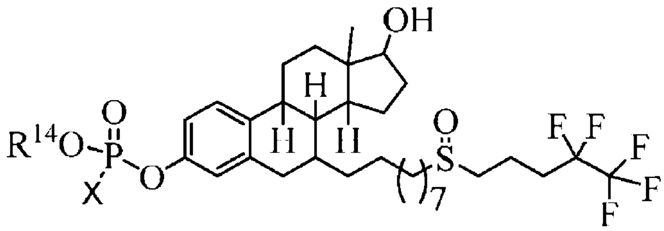

(XV)

Enantiomere, Diastereomere, Racemate, pharmazeutisch verträgliche Salze oder Solvate davon, wobei $R^{14}$ ausgewählt ist aus Wasserstoff, Methyl, Propyl, Isopropyl, n-Butyl; und X ausgewählt ist aus optional substituiertem Aziridin, Azetidin, Pyrrolidin, Piperidin, Azepan und Azokan, wobei das X optional substituiertes Aziridin, Azetidin, Pyrrolidin, Piperidin, Azepan ist und Azokan an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalogenmethyl, Cyano, Hydroxy, Mercapto, Nitro.

**11.** Verbindung nach Anspruch 1, ausgewählt aus der Gruppe, umfassend

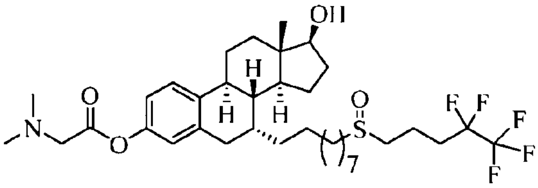

(I-a)

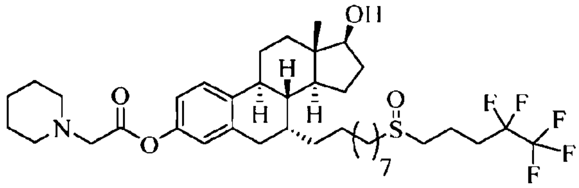

(I-b)

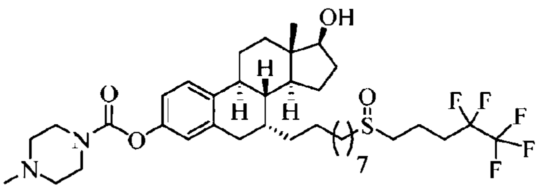

(I-c)

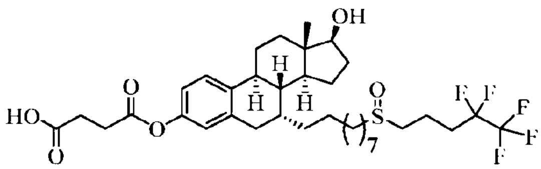

(I-d)

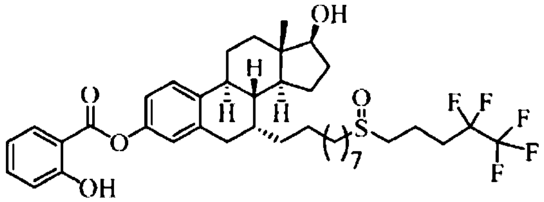

(I-e)

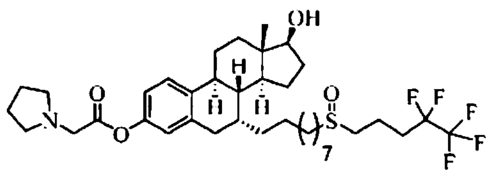

(I-g)

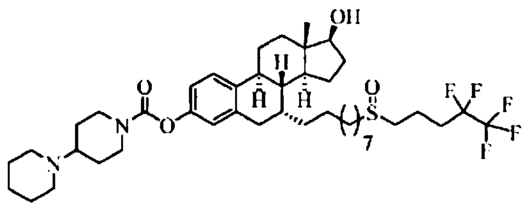

(I-h)

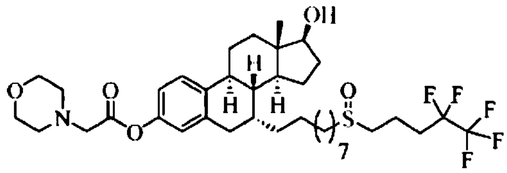

(I-i)

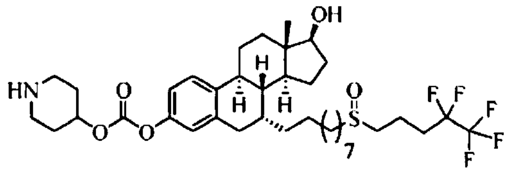

(I-j)

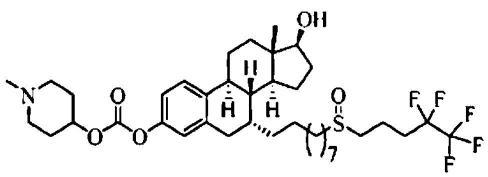

(I-k)

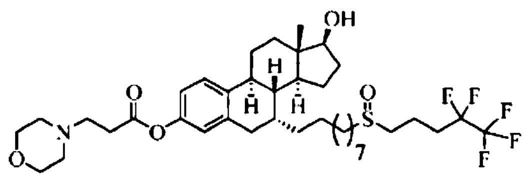

(I-l)

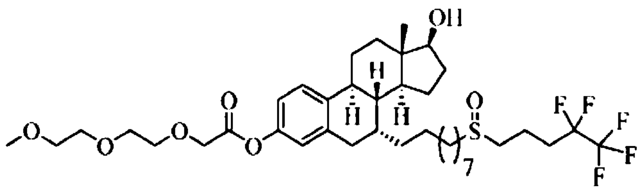

(I-m)

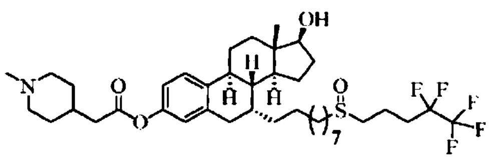

(I-n)

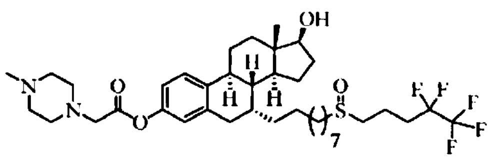

(I-o)

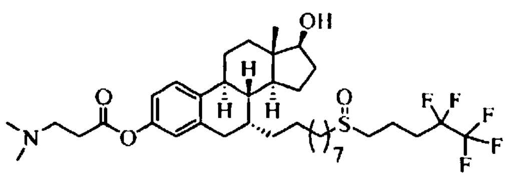

(I-p)

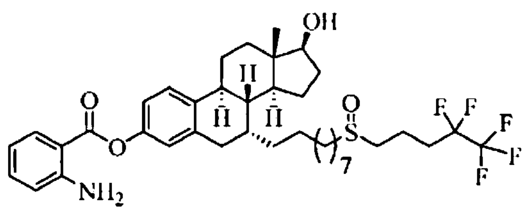

(I-q)

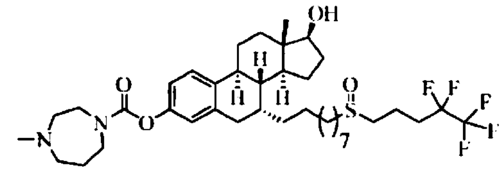

(I-r)

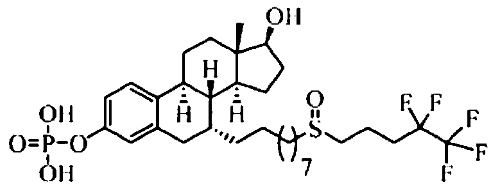

(I-s)

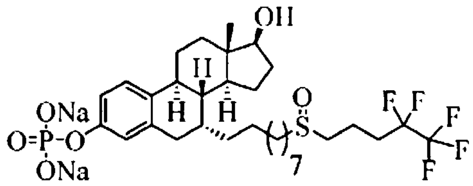

(I-t)

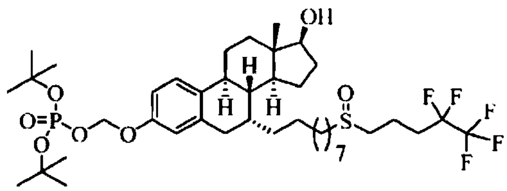

(I-u)

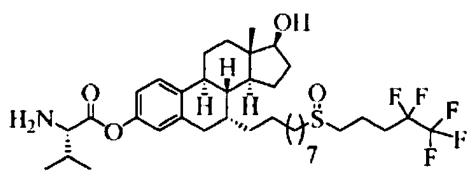

(I-v)

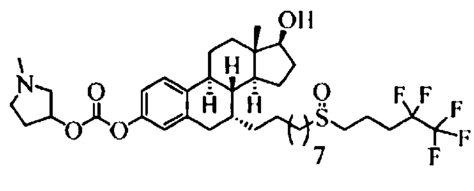

(I-x)

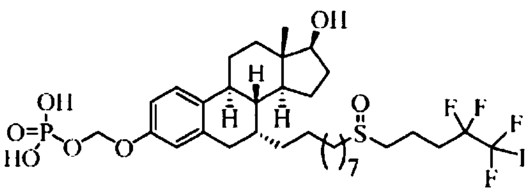

(I-y)

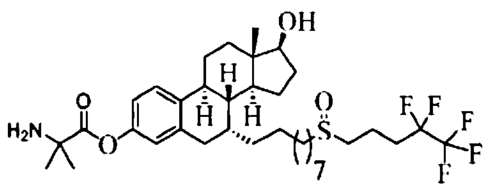

(I-z)

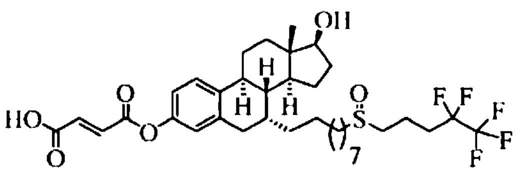

(I-aa)

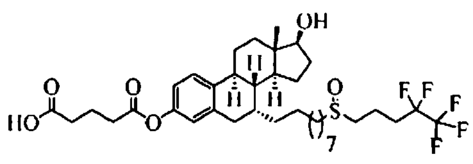

(I-ab)

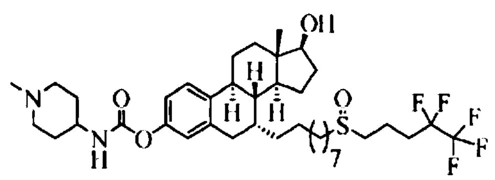

(I-ac)

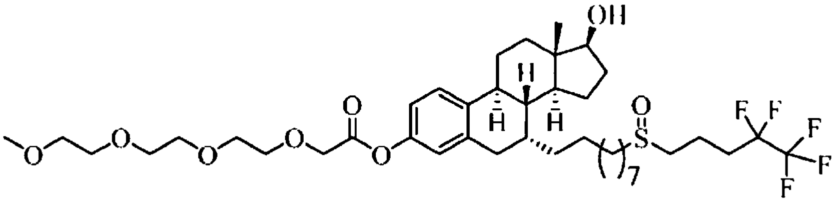

(I-ad)

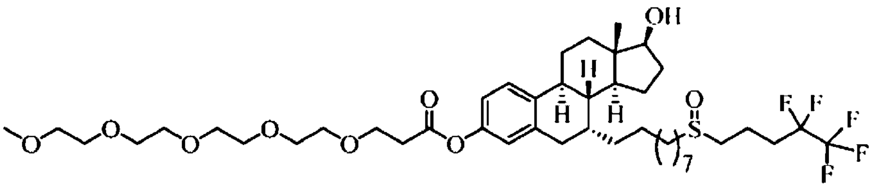

(I-ae)

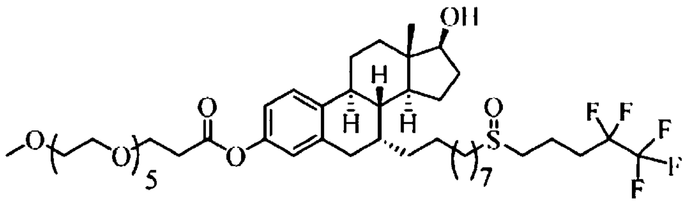

(I-af)

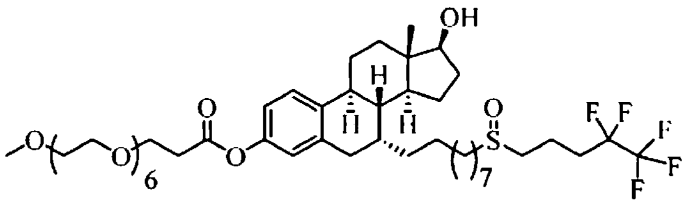

(I-ag)

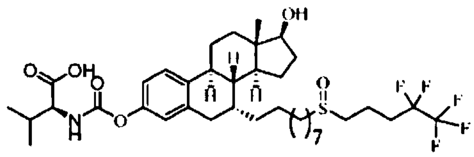

(I-ah)

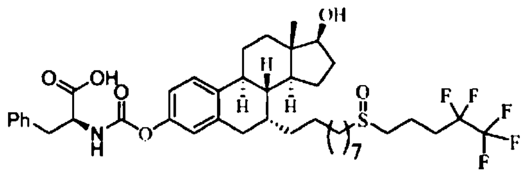

(I-ai)

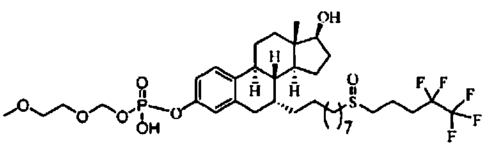

(I-aj)

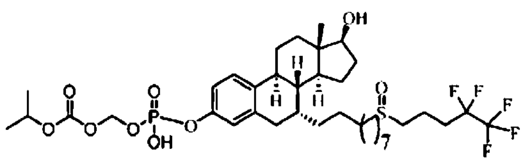

(I-ak)

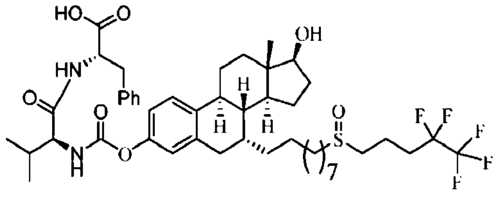

(I-al)

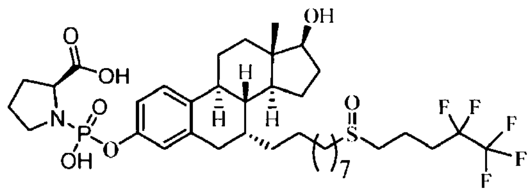

(I-am)

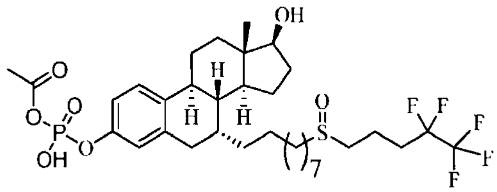

(I-an)

;

Enantiomere, Diastereomere, Racemate, pharmazeutisch verträgliche Salze oder Solvate davon.

**12.** Pharmazeutische Zusammensetzung, umfassend eine Verbindung von Formel I-A nach Anspruch 1, Enantiomere, Diastereomere, Racemate, pharmazeutisch verträgliche Salze oder Solvate davon; und pharmazeutisch verträgliche Hilfsstoffe.

**13.** Pharmazeutische Zusammensetzung, umfassend eine Verbindung von Formel II nach Anspruch 2, Enantiomere, Diastereomere, Racemate, pharmazeutisch verträgliche Salze oder Solvate davon; und pharmazeutisch verträgliche Hilfsstoffe.

**14.** Verbindung von Formel I-A

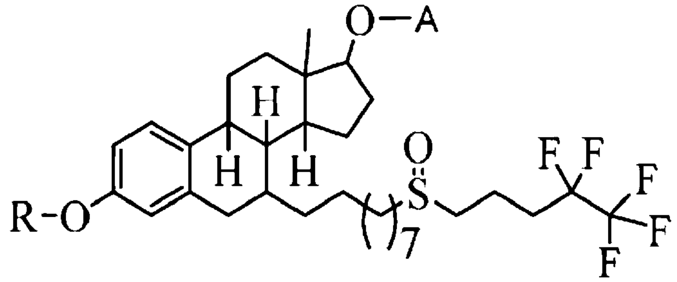

(I-A)

Enantiomere, Diastereomere, Racemate, pharmazeutisch verträgliche Salze oder Solvate davon zur Verwendung beim Behandeln gutartiger oder bösartiger Erkrankungen der Brust oder des Fortpflanzungstrakts, wobei
A ausgewählt ist aus Wasserstoff,

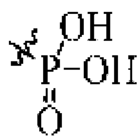 , 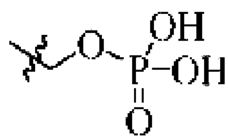 ;

und
R ausgewählt ist aus einer Gruppe, umfassend:

a)

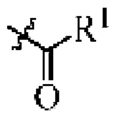

,

wobei $R^1$ ausgewählt ist aus einer Gruppe, umfassend substituiertes Aryl; optional substituiertes Alkyl, Alkenyl, Alkinyl, Cycloalkyl, Heteroaryl und $CR^2R^3$; wobei $R^2$ und $R^3$ zusammen mit dem Kohlenstoff genommen werden, an den sie gebunden sind, um einen drei- bis siebengliedrigen gesättigten, teilweise ungesättigten oder ungesättigten heterocyclischen Ring auszubilden, in dem bis zu 4 Kohlenstoffatome durch Heteroatome ersetzt sind, die gewählt sind aus der Gruppe, bestehend aus O, S oder N, und optional an einer substituierbaren Position mit einem oder mehreren $R^4$-Radikalen substituiert sein können, wobei die $R^4$-Radikale bei jedem Auftreten unabhängig ausgewählt sind aus der Gruppe, bestehend aus Alkyl, Cycloalkyl, Heterocycloalkyl, Aryl, Heteroaryl, Alkylcarbonyl, Formyl, Halogen, Alkylphosphat, Phosphat, Cyano, Nitro, Alkoxy, Alkoxycarbonyl, Alkenyl, Alkinyl, Alkylthio und Arylcarbonyl; wobei das $R^1$-substituierte Aryl, optional substituierte Alkyl, Alkenyl, Alkinyl, Cycloalkyl, Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalomethyl, Cyano, Hydroxy, Mercapto, Nitro;

b)

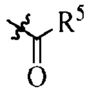

,

wobei $R^5$

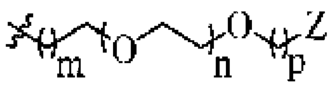

ist, wobei m und p unabhängig ausgewählt sind aus 0 bis 5, sich n auf einen Polymerisationsgrad bezieht und ausgewählt ist aus 1 bis 250 und Z optional substituiertes Alkyl oder Cycloalkyl ist; wobei das Z optional substituiertes Alkyl oder Cycloalkyl ist, das an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalomethyl, Cyano, Hydroxy, Mercapto, Nitro;

c)

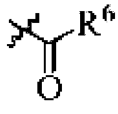

,

wobei $R^6$ ausgewählt ist aus $NH_2$, $NHR^7$ und $NR^8R^9$; wobei $R^7$ ausgewählt ist aus optional substituiertem Alkyl, Alkenyl, Alkinyl, Cycloalkyl, Heterocycloalkyl, Aryl und Heteroaryl; $R^8$ und $R^9$ zusammen mit dem Stickstoff genommen werden, an den sie gebunden sind, um einen drei- bis siebengliedrigen gesättigten, teilweise ungesättigten oder ungesättigten heterocyclischen Ring auszubilden und optional an einer substituierbaren Position mit einem oder mehreren $R^{10}$-Radikalen substituiert sein können, wobei die $R^{10}$-Radikale bei jedem Auftreten unabhängig ausgewählt sind aus der Gruppe, bestehend aus Alkyl, Alkylcarbonyl, Formyl, Halogen, Halogenalkyl, Alkylphosphat, Phosphat, Oxo, Cyano, Nitro, Amino, Alkoxy, Alkoxycarbonyl, Carboxyalkyl, Hydroxyalkyl, Alkenyl, Alkinyl, Alkylthio, Cycloalkyl, Aryl, Aralkyl, Heterocycloalkyl,

Alkylthioalkyl, Arylcarbonyl, Aralkylcarbonyl und Alkoxyalkyl; wobei das $R^7$ optional substituiertes Alkyl, Alkenyl, Alkinyl, Cycloalkyl, Heterocycloalkyl, Aryl ist und Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalogenmethyl, Cyano, Hydroxy, Mercapto, Nitro;

d)

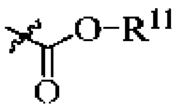

,

wobei $R^{11}$ ausgewählt ist aus einer Gruppe, umfassend optional substituiertes Alkyl, Alkenyl, Alkinyl, Cycloalkyl, Heterocycloalkyl, Aryl und Heteroaryl; wobei das $R^{11}$ optional substituiertes Alkyl, Alkenyl, Alkinyl, Cycloalkyl, Heterocycloalkyl, Aryl ist und Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalogenmethyl, Cyano, Hydroxy, Mercapto, Nitro;

e)

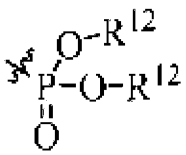

,

wobei jedes $R^{12}$ unabhängig ausgewählt ist aus einer Gruppe, umfassend Wasserstoff; optional substituiertes Alkyl, Aryl, Acyl und Heteroaryl; wobei das $R^{12}$ optional substituiertes Alkyl, Aryl, Acyl ist und Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalogenmethyl, Cyano, Hydroxy, Mercapto, Nitro;

mit einer Bedingung, dass, wenn A Wasserstoff ist und eine der $R^{12}$-Substitution ausgewählt ist aus optional substituiertem Alkyl und Aryl, dann eine andere $R^{12}$-Substitution Wasserstoff ist;

f)

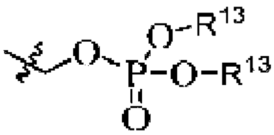

,

wobei jedes $R^{13}$ unabhängig ausgewählt ist aus einer Gruppe, umfassend Wasserstoff; optional substituiertes Alkyl, Cycloalkyl, Heterocycloalkyl, Aryl und Heteroaryl; wobei das $R^{13}$ optional substituiertes Alkyl, Cycloalkyl, Heterocycloalkyl, Aryl ist und Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalogenmethyl, Cyano, Hydroxy, Mercapto, Nitro;

g)

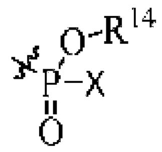

wobei $R^{14}$ ausgewählt ist aus einer Gruppe, umfassend Wasserstoff; optional substituiertes Alkyl, Aryl, Acyl, Heteroaryl; und X optional substituiertes Heterocycloalkyl ist; wobei das $R^{14}$ optional substituiertes Alkyl, Aryl, Acyl, Heteroaryl ist und X optional substituiertes Heterocycloalkyl ist, das an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalomethyl, Cyano, Hydroxy, Mercapto, Nitro; und

h) Wasserstoff mit einer Bedingung, dass, wenn R Wasserstoff ist, A nicht Wasserstoff ist.

**15.** Verbindung von Formel II

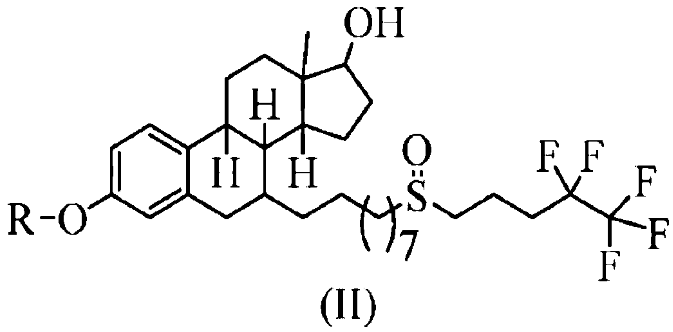

(II)

Enantiomere, Diastereomere, Racemate, pharmazeutisch verträgliche Salze oder Solvate davon zur Verwendung beim Behandeln gutartiger oder bösartiger Erkrankungen der Brust oder des Fortpflanzungstrakts, wobei R ausgewählt ist aus einer Gruppe, umfassend:

a)

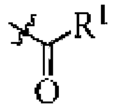

wobei $R^1$ ausgewählt ist aus einer Gruppe, umfassend substituiertes Aryl; optional substituiertes Alkyl, Alkenyl, Alkinyl, Cycloalkyl, Heteroaryl und $CR^2R^3$, wobei $R^2$ und $R^3$ zusammen mit dem Kohlenstoff genommen werden, an den sie gebunden sind, um einen drei- bis siebengliedrigen gesättigten, teilweise ungesättigten oder ungesättigten heterocyclischen Ring auszubilden, in dem bis zu 4 Kohlenstoffatome durch Heteroatome ersetzt sind, die gewählt sind aus der Gruppe, bestehend aus O, S oder N, und optional an einer substituierbaren Position mit einem oder mehreren $R^4$-Radikalen substituiert sein können, wobei die $R^4$-Radikale bei jedem Auftreten unabhängig ausgewählt sind aus der Gruppe, bestehend aus Alkyl, Cycloalkyl, Heterocycloalkyl, Aryl, Heteroaryl, Alkylcarbonyl, Formyl, Halogen, Alkylphosphat, Phosphat, Cyano, Nitro, Alkoxy, Alkoxycarbonyl, Alkenyl, Alkinyl, Alkylthio und Arylcarbonyl; wobei das $R^1$-substituierte Aryl, optional substituierte Alkyl, Alkenyl, Alkinyl, Cycloalkyl, Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalomethyl, Cyano, Hydroxy, Mercapto, Nitro;

b)

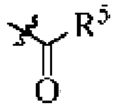
,

wobei $R^5$

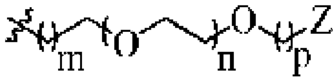

ist, wobei m und p unabhängig ausgewählt sind aus 0 bis 5, sich n auf einen Polymerisationsgrad bezieht und ausgewählt ist aus 1 bis 250 und Z optional substituiertes Alkyl oder Cycloalkyl ist; wobei das Z optional substituiertes Alkyl oder Cycloalkyl ist, das an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalomethyl, Cyano, Hydroxy, Mercapto, Nitro;

c)

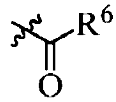
,

wobei $R^6$ ausgewählt ist aus $NH_2$, $NHR^7$ und $NR^8R^9$, wobei $R^7$ ausgewählt ist aus optional substituiertem Alkyl, Alkenyl, Alkinyl, Cycloalkyl, Heterocycloalkyl, Aryl und Heteroaryl; $R^8$ und $R^9$ zusammen mit dem Stickstoff genommen werden, an den sie gebunden sind, um einen drei- bis siebengliedrigen gesättigten, teilweise ungesättigten oder ungesättigten heterocyclischen Ring auszubilden und optional an einer substituierbaren Position mit einem oder mehreren $R^{10}$-Radikalen substituiert sein können, wobei die $R^{10}$-Radikale bei jedem Auftreten unabhängig ausgewählt sind aus der Gruppe, bestehend aus Alkyl, Alkylcarbonyl, Formyl, Halogen, Halogenalkyl, Alkylphosphat, Phosphat, Oxo, Cyano, Nitro, Amino, Alkoxy, Alkoxycarbonyl, Carboxyalkyl, Hydroxyalkyl, Alkenyl, Alkinyl, Alkylthio, Cycloalkyl, Aryl, Aralkyl, Heterocycloalkyl, Alkylthioalkyl, Arylcarbonyl, Aralkylcarbonyl und Alkoxyalkyl; wobei das $R^7$ optional substituiertes Alkyl, Alkenyl, Alkinyl, Cycloalkyl, Heterocycloalkyl, Aryl ist und Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalogenmethyl, Cyano, Hydroxy, Mercapto, Nitro;

d)

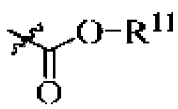
,

wobei $R^{11}$ ausgewählt ist aus einer Gruppe, umfassend optional substituiertes Alkyl, Alkenyl, Alkinyl, Cycloalkyl, Heterocycloalkyl, Aryl und Heteroaryl; wobei das $R^{11}$ optional substituiertes Alkyl, Alkenyl, Alkinyl, Cycloalkyl, Heterocycloalkyl, Aryl ist und Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalogenmethyl, Cyano, Hydroxy, Mercapto, Nitro;

e)

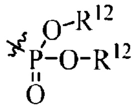

,

wobei jedes $R^{12}$ unabhängig ausgewählt ist aus einer Gruppe, umfassend Wasserstoff; optional substituiertes Alkyl, Aryl, Acyl und Heteroaryl; wobei das $R^{12}$ optional substituiertes Alkyl, Aryl, Acyl ist und Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalogenmethyl, Cyano, Hydroxy, Mercapto, Nitro;

mit einer Bedingung, dass, wenn eines von $R^{12}$ ausgewählt ist aus optional substituiertem Alkyl und Aryl, dann eine andere $R^{12}$-Substitution Wasserstoff ist;

f)

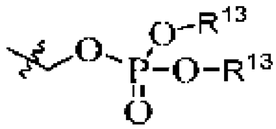

,

wobei jedes $R^{13}$ unabhängig ausgewählt ist aus einer Gruppe, umfassend Wasserstoff; optional substituiertes Alkyl, Cycloalkyl, Heterocycloalkyl, Aryl und Heteroaryl; wobei das $R^{13}$ optional substituiertes Alkyl, Cycloalkyl, Heterocycloalkyl, Aryl ist und Heteroaryl an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalogenmethyl, Cyano, Hydroxy, Mercapto, Nitro; und

g)

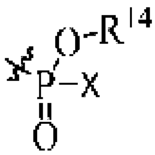

,

wobei $R^{14}$ ausgewählt ist aus einer Gruppe, umfassend Wasserstoff; optional substituiertes Alkyl, Aryl, Acyl, Heteroaryl; und X optional substituiertes Heterocycloalkyl ist; wobei das $R^{14}$ optional substituiertes Alkyl, Aryl, Acyl, Heteroaryl ist und X optional substituiertes Heterocycloalkyl ist, das an einer substituierbaren Position mit einem oder mehreren Radikalen substituiert ist, die bei jedem Auftreten unabhängig ausgewählt sind aus der Gruppe, bestehend aus C1-C8-Alkyl, C2-C8-Alkenyl, C2-C8-Alkinyl, Cycloalkyl, Heterocycloalkyl, Aryl, Heteroaryl, Amino, Alkylamino, Acyl, Acyloxy, Acylamino, Aminocarbonyl, Alkoxycarbonyl, Alkoxyalkyloxy, Alkoxycarbonyloxy, Carbamat, Sulfinyl, Sulfonyl, Alkoxy, Sulfanyl, Halogen, Carboxy, Trihalomethyl, Cyano, Hydroxy, Mercapto, Nitro.

**16.** Zusammensetzung zur Verwendung beim Behandeln gutartiger oder bösartiger Erkrankungen der Brust oder des Fortpflanzungstrakts, umfassend mindestens eine der Verbindungsformel I-A nach Anspruch 14; und Enantiomere, Diastereomere, Racemate, pharmazeutisch verträgliche Salze oder Solvate davon.

**17.** Zusammensetzung zur Verwendung beim Behandeln gutartiger oder bösartiger Erkrankungen der Brust oder des Fortpflanzungstrakts, umfassend mindestens eine der Verbindungsformel II nach Anspruch 15; und Enantiomere, Diastereomere, Racemate, pharmazeutisch verträgliche Salze oder Solvate davon.

**Revendications**

1. Composé de formule I-A

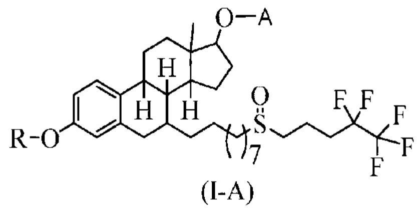

(I-A)

énantiomères, diastéréoisomères, racémates, sels pharmaceutiquement acceptables ou solvates de celui-ci, dans lequel
A est choisi parmi un hydrogène,

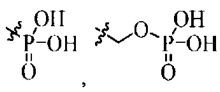

,

et
R est choisi dans le groupe constitué par :

a)

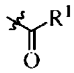

dans lequel $R^1$ est choisi dans le groupe constitué par un aryle substitué ; un alkyle éventuellement substitué, un alcényle, un alcynyle, un cycloalkyle, un hétéroaryle et $CR^2R^3$, dans lequel $R^2$ et $R^3$ forment, conjointement avec le carbone auquel ils sont liés, un noyau hétérocyclique saturé, partiellement insaturé ou insaturé à trois à sept chaînons dans lequel jusqu'à 4 atomes de carbone sont remplacés par des hétéroatomes choisis dans le groupe constitué par O, S ou N et peuvent éventuellement être substitués à une position substituable par un ou plusieurs radicaux $R^4$, dans lequel les radicaux $R^4$ sont indépendamment choisis à chaque occurrence dans le groupe constitué par un alkyle, un cycloalkyle, un hétérocycloalkyle, un aryle, un hétéroaryle, un alkylcarbonyle, un formyle, un halogéno, un alkylphosphate, un phosphate, un cyano, un nitro, un alcoxy, un alcoxycarbonyle, un alcényle, un alcynyle, un alkylthio et un arylcarbonyle ; dans lequel l'aryle substitué par $R^1$, l'alkyle éventuellement substitué, l'alcényle, l'alcynyle, le cycloalkyle, l'hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;
b)

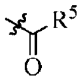

dans lequel $R^5$ représente

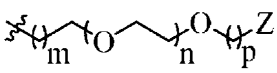

;

dans lequel m et p sont indépendamment choisis parmi de 0 à 5, n désigne le degré de polymérisation et est choisi parmi de 1 à 250 et Z représente un alkyle ou un cycloalkyle éventuellement substitué ; dans lequel Z

représente un alkyle ou un cycloalkyle éventuellement substitué qui est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;

c)

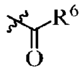

dans lequel $R^6$ est choisi parmi $NH_2$, $NHR^7$ et $NR^8R^9$ ; dans lequel $R^7$ est choisi parmi une alkyle éventuellement substitué, un alcényle, un alcynyle, un cycloalkyle, un hétérocycloalkyle, un aryle et un hétéroaryle ; $R^8$ et $R^9$ forment, conjointement avec l'azote auquel ils sont liés, un noyau hétérocyclique saturé, partiellement insaturé ou insaturé à trois à sept chaînons et peuvent éventuellement être substitués à une position substituable par un ou plusieurs radicaux $R^{10}$, dans lequel les radicaux $R^{10}$ sont indépendamment choisis à chaque occurrence dans le groupe constitué par un alkyle, un alkylcarbonyle, un formyle, un halogéno, un halogénoalkyle, un alkylphosphate, un phosphate, un oxo, un cyano, un nitro, un amino, un alcoxy, un alcoxycarbonyle, un carboxyalkyle, un hydroxyalkyle, un alcényle, un alcynyle, un alkylthio, un cycloalkyle, un aryle, un aralkyle, un hétérocycloalkyle, un alkylthioalkyle, un arylcarbonyle, un aralkylcarbonyle et un alcoxyalkyle ; dans lequel $R^7$ qui est un alkyle éventuellement substitué, un alcényle, un alcynyle, un cycloalkyle, un hétérocycloalkyle, un aryle et un hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;

d)

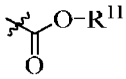

dans lequel $R^{11}$ est choisi dans le groupe constitué par un alkyle éventuellement substitué, un alcényle, un alcynyle, un cycloalkyle, un hétérocycloalkyle, un aryle et un hétéroaryle ; dans lequel $R^{11}$ qui est un alkyle éventuellement substitué, un alcényle, un alcynyle, un cycloalkyle, un hétérocycloalkyle, un aryle et un hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;

e)

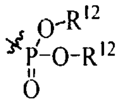

dans lequel chaque $R^{12}$ est indépendamment choisi dans le groupe constitué par un hydrogène ; un alkyle éventuellement substitué, un aryle, un acyle et un hétéroaryle ; dans lequel $R^{12}$ qui est un alkyle éventuellement substitué, un aryle, un acyle et un hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un

alcoxy, un sulfanyle, un halogène, un carboxy, un trihalogénométhyle, un cyano, un hydroxy, un mercapto, un nitro ;

à condition que lorsque A représente un hydrogène et que l'une des substitutions de $R^{12}$ est choisie parmi un alkyle éventuellement substitué et un aryle, alors l'autre substitution de $R^{12}$ est un hydrogène ;

f)

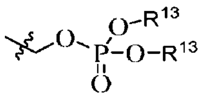

dans lequel chaque $R^{13}$ est indépendamment choisi dans le groupe constitué par un hydrogène ; un alkyle éventuellement substitué, un cycloalkyle, un hétérocycloalkyle, un aryle et un hétéroaryle ; dans lequel $R^{13}$ qui est un alkyle éventuellement substitué, un cycloalkyle, un hétérocycloalkyle, un aryle et un hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;

g)

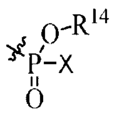

dans lequel $R^{14}$ est choisi dans le groupe constitué par un hydrogène ; un alkyle éventuellement substitué, un aryle, un acyle, un hétéroaryle ; et X est choisi parmi un hétérocycloalkyle éventuellement substitué ; dans lequel $R^{14}$ qui est un alkyle éventuellement substitué, un aryle, un acyle, un hétéroaryle et X représente un hétérocycloalkyle éventuellement substitué est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ; et

h) un hydrogène, à condition que lorsque R représente un hydrogène, A ne représente pas un hydrogène.

**2.** Composé de formule II

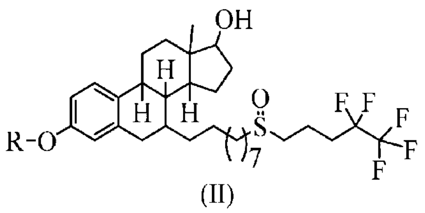

(II)

énantiomères, diastéréoisomères, racémates, sels pharmaceutiquement acceptables ou solvates de celui-ci, dans lequel R est choisi dans le groupe constitué par :

a)

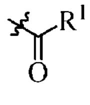

dans lequel $R^1$ est choisi dans le groupe constitué par un aryle substitué ; un alkyle éventuellement substitué, un alcényle, un alcynyle, un cycloalkyle, un hétéroaryle et $CR^2R^3$ ; dans lequel $R^2$ et $R^3$ forment, conjointement avec

le carbone auquel ils sont liés, un noyau hétérocyclique saturé, partiellement insaturé ou insaturé à trois à sept chaînons dans lequel jusqu'à 4 atomes de carbone sont remplacés par des hétéroatomes choisis dans le groupe constitué par O, S ou N et peuvent éventuellement être substitués à une position substituable par un ou plusieurs radicaux $R^4$, dans lequel les radicaux $R^4$ sont indépendamment choisis à chaque occurrence dans le groupe constitué par un alkyle, un cycloalkyle, un hétérocycloalkyle, un aryle, un hétéroaryle, un alkylcarbonyle, un formyle, un halogéno, un alkylphosphate, un phosphate, un cyano, un nitro, un alcoxy, un alcoxycarbonyle, un alcényle, un alcynyle, un alkylthio et un arylcarbonyle ; dans lequel l'aryle substitué par $R^1$, l'alkyle éventuellement substitué, l'alcényle, l'alcynyle, le cycloalkyle, l'hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;

b)

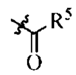

dans lequel $R^5$ représente

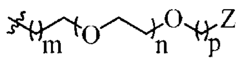 ;

dans lequel m et p sont indépendamment choisis parmi de 0 à 5, n désigne le degré de polymérisation et est choisi parmi de 1 à 250 et Z représente un alkyle ou un cycloalkyle éventuellement substitué ; dans lequel Z représente un alkyle ou un cycloalkyle éventuellement substitué qui est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;

c)

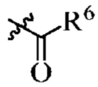

dans lequel $R^6$ est choisi parmi $NH_2$, $NHR^7$ et $NR^8R^9$ ; dans lequel $R^7$ est choisi parmi une alkyle éventuellement substitué, un alcényle, un alcynyle, un cycloalkyle, un hétérocycloalkyle, un aryle et un hétéroaryle ; $R^8$ et $R^9$ forment, conjointement avec l'azote auquel ils sont liés, un noyau hétérocyclique saturé, partiellement insaturé ou insaturé à trois à sept chaînons et peuvent éventuellement être substitués à une position substituable par un ou plusieurs radicaux $R^{10}$, dans lequel les radicaux $R^{10}$ sont indépendamment choisis à chaque occurrence dans le groupe constitué par un alkyle, un alkylcarbonyle, un formyle, un halogéno, un halogénoalkyle, un alkylphosphate, un phosphate, un oxo, un cyano, un nitro, un amino, un alcoxy, un alcoxycarbonyle, un carboxyalkyle, un hydroxyalkyle, un alcényle, un alcynyle, un alkylthio, un cycloalkyle, un aryle, un aralkyle, un hétérocycloalkyle, un alkylthioalkyle, un arylcarbonyle, un aralkylcarbonyle et un alcoxyalkyle ; dans lequel $R^7$ qui est un alkyle éventuellement substitué, un alcényle, un alcynyle, un cycloalkyle, un hétérocycloalkyle, un aryle et un hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;

d)

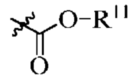

dans lequel $R^{11}$ est choisi dans le groupe constitué par un alkyle éventuellement substitué, un alcényle, un alcynyle, un cycloalkyle, un hétérocycloalkyle, un aryle et un hétéroaryle ; dans lequel $R^{11}$ qui est un alkyle éventuellement substitué, un alcényle, un alcynyle, un cycloalkyle, un hétérocycloalkyle, un aryle et un hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;

e)

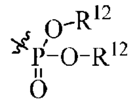

dans lequel chaque $R^{12}$ est indépendamment choisi dans le groupe constitué par un hydrogène ; un alkyle éventuellement substitué, un aryle, un acyle et un hétéroaryle ; dans lequel $R^{12}$ qui est un alkyle éventuellement substitué, un aryle, un acyle et un hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;

à condition que lorsque l'une des $R^{12}$ est choisie parmi un alkyle éventuellement substitué et un aryle, alors l'autre substitution de $R^{12}$ est un hydrogène ;

f)

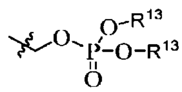

dans lequel $R^{13}$ est choisi dans le groupe constitué par un hydrogène ; un alkyle éventuellement substitué, un cycloalkyle, un hétérocycloalkyle, un aryle et un hétéroaryle ; dans lequel $R^{13}$ qui est un alkyle éventuellement substitué, un cycloalkyle, un hétérocycloalkyle, un aryle et un hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ; et

g)

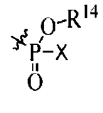

dans lequel $R^{14}$ est choisi dans le groupe constitué par un hydrogène ; un alkyle éventuellement substitué, un aryle, un acyle, un hétéroaryle ; et X représente un hétérocycloalkyle éventuellement substitué ; dans lequel $R^{14}$ qui est un alkyle éventuellement substitué, un aryle, un acyle, un hétéroaryle et X représente un hétérocycloalkyle éventuellement substitué est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un

trihalogénométhyle, un cyano, un hydroxy, un mercapto, un nitro.

3. Composé selon la revendication 2, dans lequel un ou plusieurs radicaux $R^{10}$ représentent des hétérocycloalkyle choisis parmi la pyrrolidine, la pipéridine, la pipérazine, la morpholine, le tétrahydrofurane et le tétrahydrothiophényle.

4. Composé selon la revendication 2, comprenant le composé III représenté par les formules suivantes :

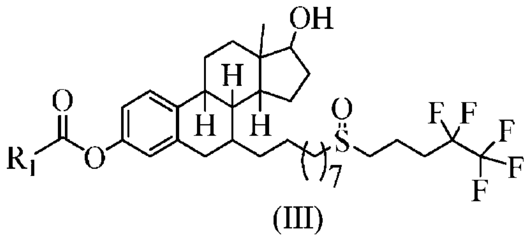

(III)

énantiomères, diastéréoisomères, racémates, sels pharmaceutiquement acceptables ou solvates de celui-ci, dans lequel $R^1$ est choisi parmi

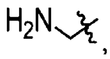, 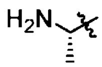, 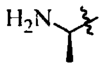, 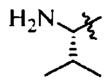,

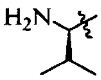, 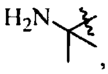, 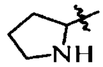, 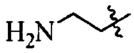,

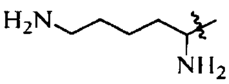, 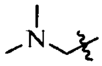, 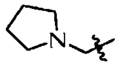, 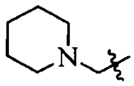,

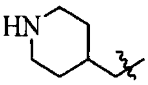, 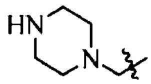, 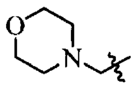, 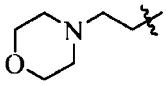,

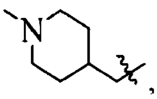, 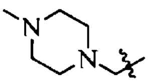, 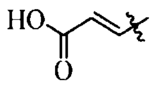, 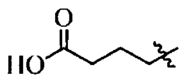,

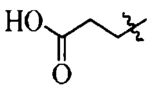, 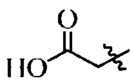, 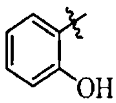, 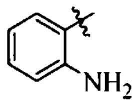,

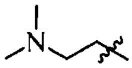.

5. Composé selon la revendication 2, comprenant le composé IV représenté par les formules suivantes :

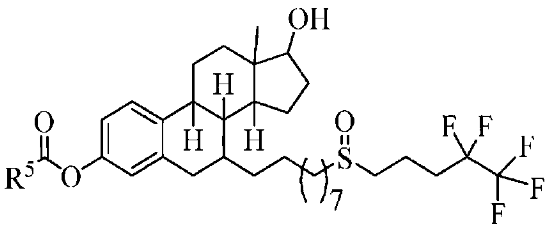

(IV)

énantiomères, diastéréoisomères, racémates, sels pharmaceutiquement acceptables ou solvates de celui-ci, dans lequel $R^5$ est choisi parmi

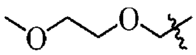,
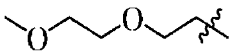,
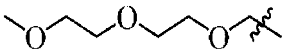,
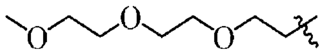,
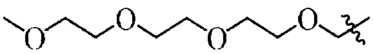,
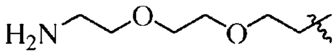,
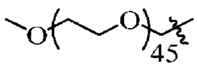,
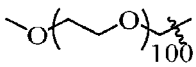,
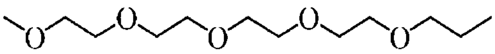,
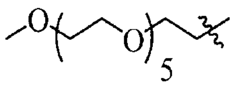,
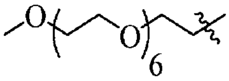.

**6.** Composé selon la revendication 2, comprenant le composé V représenté par les formules suivantes :

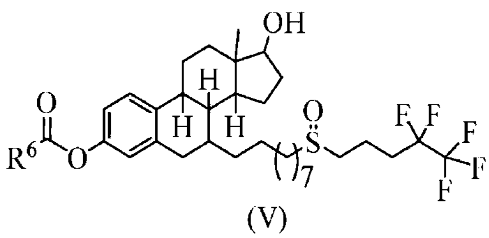

(V)

énantiomères, diastéréoisomères, racémates, sels pharmaceutiquement acceptables ou solvates de celui-ci, dans lequel $R^6$ est choisi parmi

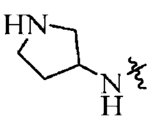,
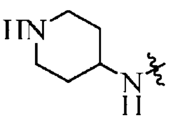,
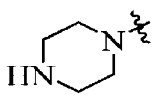,

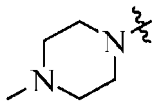, 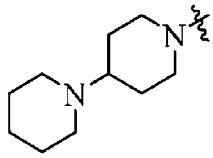, 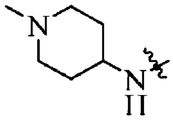,

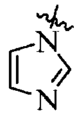, 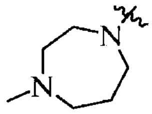.

**7.** Composé selon la revendication 2, comprenant le composé VI représenté par les formules suivantes :

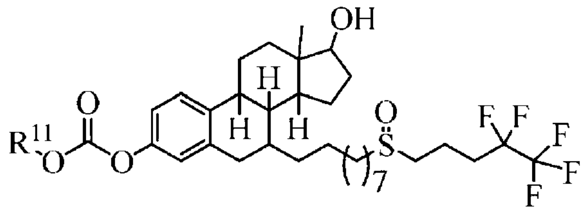

(VI)

énantiomères, diastéréoisomères, racémates, sels pharmaceutiquement acceptables ou solvates de celui-ci, dans lequel $R^{11}$ est choisi parmi

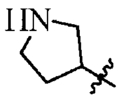, 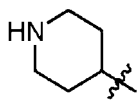, 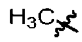,

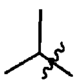, 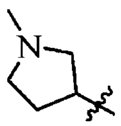, 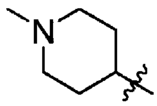.

**8.** Composé selon la revendication 2, comprenant le composé VII représenté par les formules suivantes :

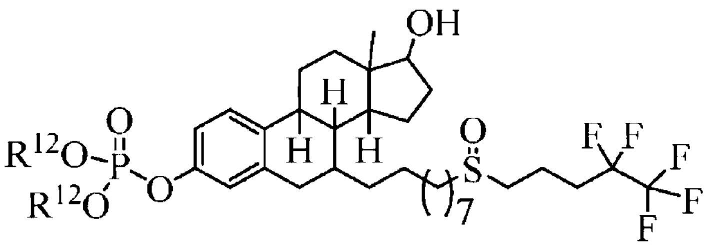

(VII)

énantiomères, diastéréoisomères, racémates, sels pharmaceutiquement acceptables ou solvates de celui-ci, dans lequel chaque $R^{12}$ est indépendamment choisi parmi un hydrogène, un méthyle, un propyle, un isopropyle, un n-butyle,

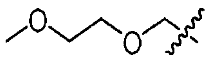, 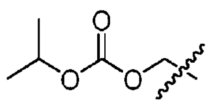 ;

à condition que lorsque l'une des $R^{12}$ est choisie parmi un alkyle et un aryle, alors l'autre substitution de $R^{12}$ est un hydrogène.

**9.** Composé selon la revendication 2, comprenant le composé VIII représenté par les formules suivantes :

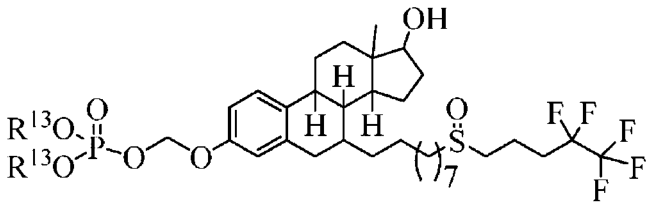

(VIII)

énantiomères, diastéréoisomères, racémates, sels pharmaceutiquement acceptables ou solvates de celui-ci, dans lequel chaque $R^{13}$ est indépendamment choisi parmi un hydrogène, un méthyle, un éthyle, un propyle, un isopropyle, un n-butyle, un t-butyle, un cyclopropyle, un hexyle, un phényle et un 3-pyridyle.

**10.** Composé selon la revendication 2, comprenant le composé XV représenté par les formules suivantes :

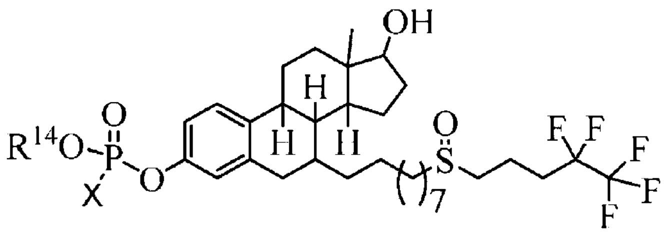

(XV)

énantiomères, diastéréoisomères, racémates, sels pharmaceutiquement acceptables ou solvates de celui-ci, dans lequel $R^{14}$ est choisi parmi un hydrogène, un méthyle, un propyle, un isopropyle, un n-butyle ; et X est choisi parmi une aziridine éventuellement substituée, une azétidine, une pyrrolidine, une pipéridine, un azépane et un azocane, dans lequel X est une aziridine éventuellement substituée, une azétidine, une pyrrolidine, une pipéridine, un azépane et un azocane qui est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro.

**11.** Composé selon la revendication 1 choisi dans le groupe constitué par

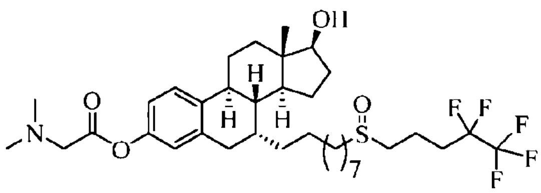

(I-a)

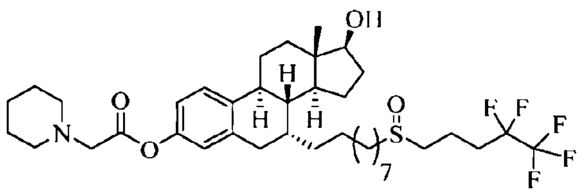

(I-b)

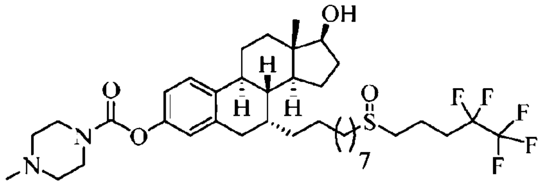

(I-c)

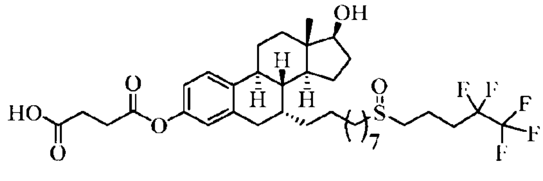

(I-d)

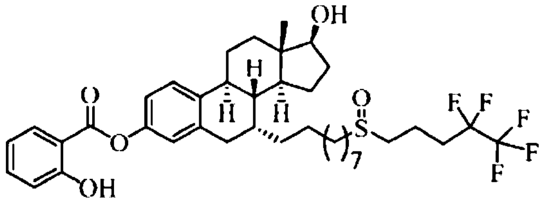

(I-e)

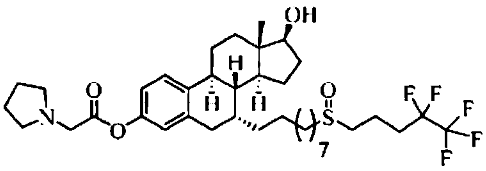

(I-g)

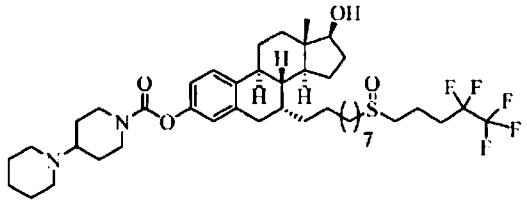

(I-h)

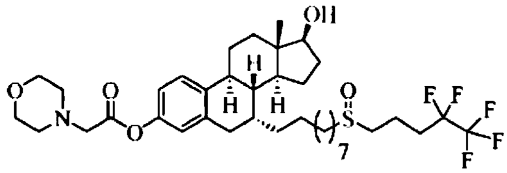

(I-i)

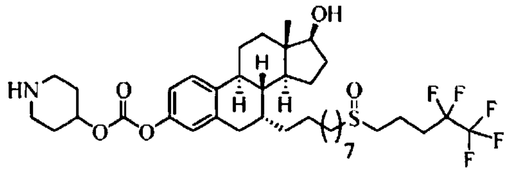

(I-j)

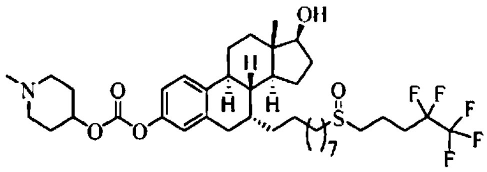

(I-k)

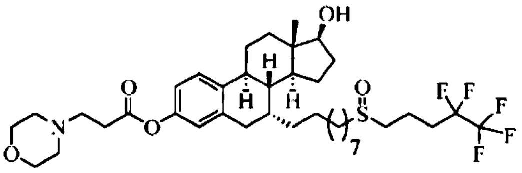

(I-l)

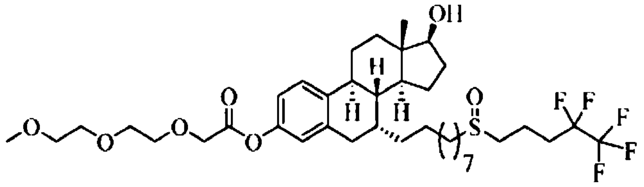

(I-m)

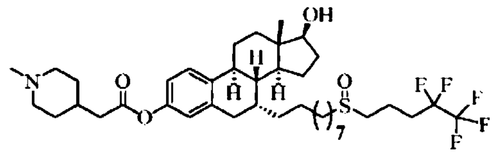

(I-n)

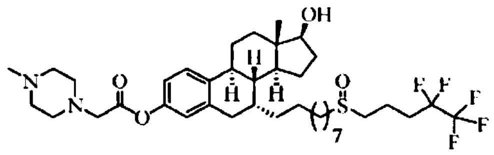

(I-o)

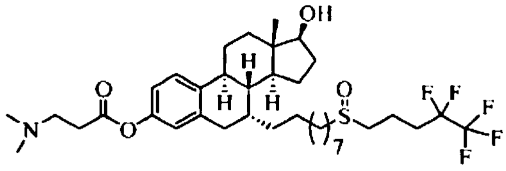

(I-p)

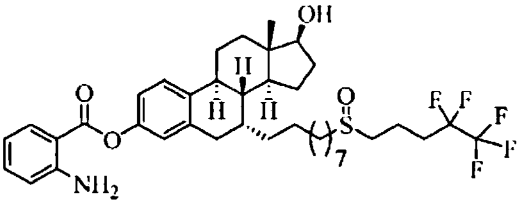

(I-q)

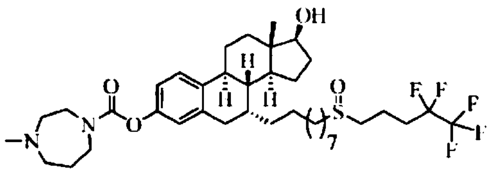

(I-r)

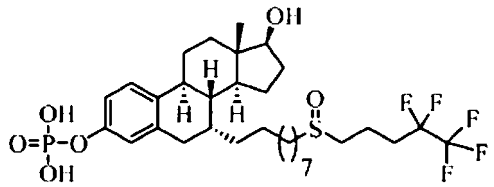

(I-s)

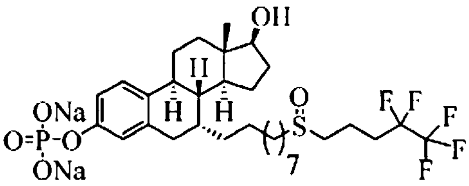

(I-t)

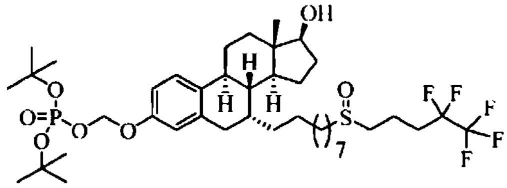

(I-u)

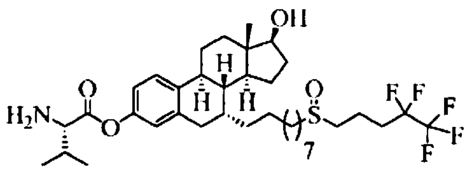

(I-v)

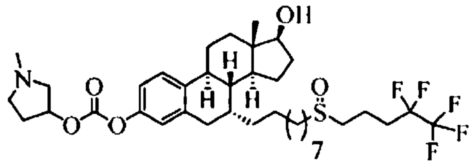

(I-x)

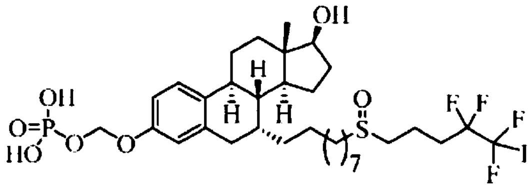

(I-y)

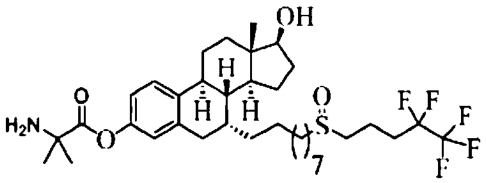

(I-z)

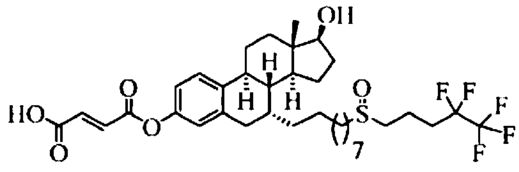

(I-aa)

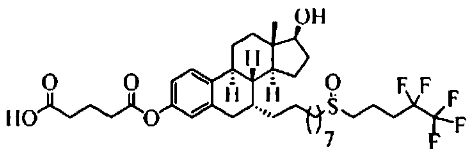

(I-ab)

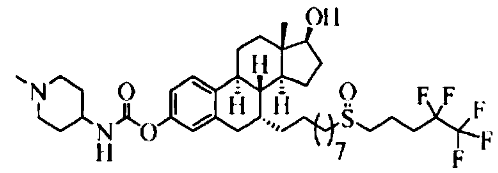

(I-ac)

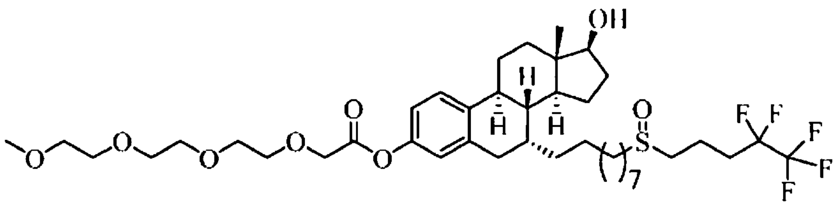

(I-ad)

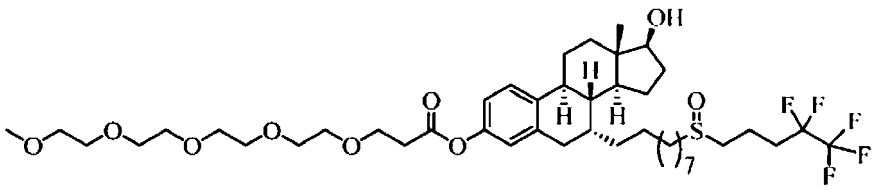

(I-ae)

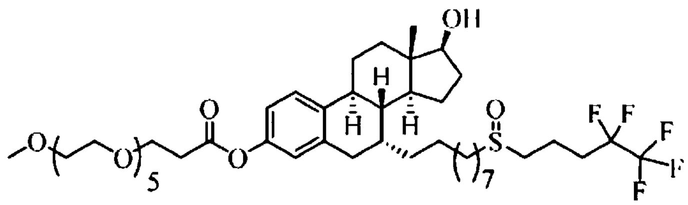

(I-af)

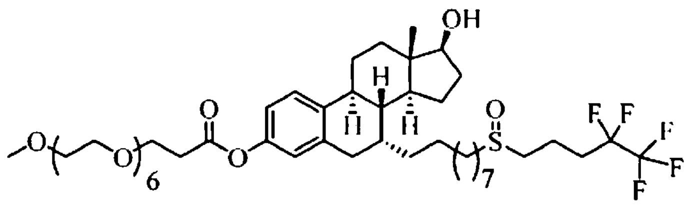

(I-ag)

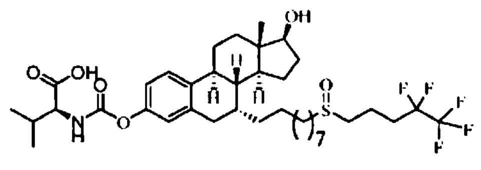

(I-ah)

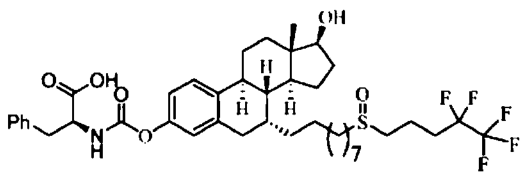

(I-ai)

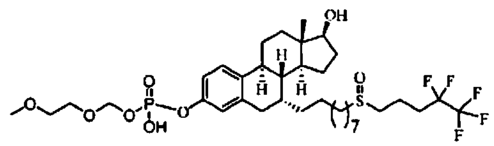

(I-aj)

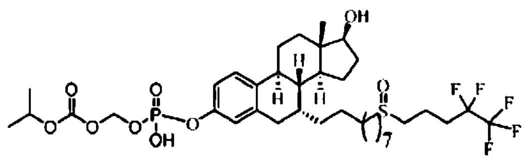

(I-ak)

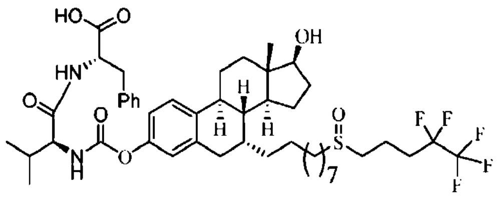

(I-al)

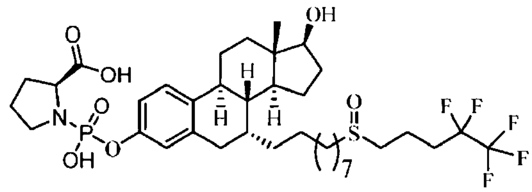

(I-am)

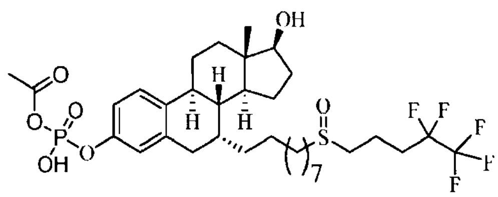

(I-an)

;

des énantiomères, des diastéréoisomères, des racémates, des sels pharmaceutiquement acceptables ou des solvates de celui-ci.

**12.** Composition pharmaceutique comprenant un composé de formule I-A selon la revendication 1, des énantiomères, des diastéréoisomères, des racémates, des sels pharmaceutiquement acceptables ou des solvates de celui-ci ; et des excipients pharmaceutiquement acceptables.

**13.** Composition pharmaceutique comprenant un composé de formule II selon la revendication 2, des énantiomères, des diastéréoisomères, des racémates, des sels pharmaceutiquement acceptables ou des solvates de celui-ci ; et des excipients pharmaceutiquement acceptables.

**14.** Composé de formule I-A

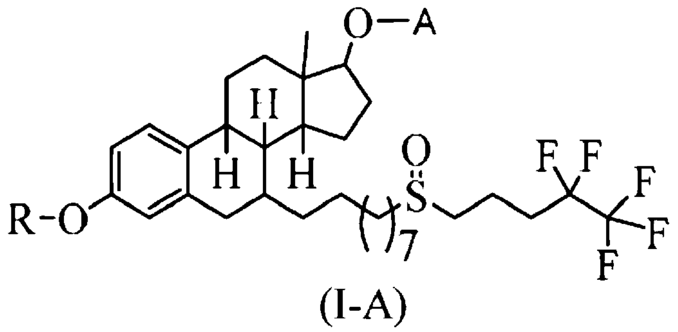

(I-A)

énantiomères, diastéréoisomères, racémates, sels pharmaceutiquement acceptables ou solvates de celui-ci, destinés à être utilisés dans le traitement de maladies bénines ou malines du sein ou de l'appareil génital, dans lequel

A est choisi parmi un hydrogène,

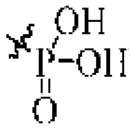

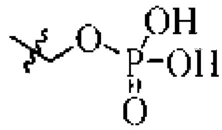

et

R est choisi dans le groupe constitué par :

a)

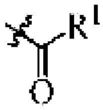

dans lequel $R^1$ est choisi dans le groupe constitué par un aryle substitué ; un alkyle éventuellement substitué, un alcényle, un alcynyle, un cycloalkyle, un hétéroaryle et $CR^2R^3$ ; dans lequel $R^2$ et $R^3$ forment, conjointement avec le carbone auquel ils sont liés, un noyau hétérocyclique saturé, partiellement insaturé ou insaturé à trois à sept chaînons dans lequel jusqu'à 4 atomes de carbone sont remplacés par des hétéroatomes choisis dans le groupe constitué par O, S ou N et peuvent éventuellement être substitués à une position substituable par un ou plusieurs radicaux $R^4$, dans lequel les radicaux $R^4$ sont indépendamment choisis à chaque occurrence dans le groupe constitué par un alkyle, un cycloalkyle, un hétérocycloalkyle, un aryle, un hétéroaryle, un alkylcarbonyle, un formyle, un halogéno, un alkylphosphate, un phosphate, un cyano, un nitro, un alcoxy, un alcoxycarbonyle, un alcényle, un alcynyle, un alkylthio et un arylcarbonyle ; dans lequel l'aryle substitué par $R^1$, l'alkyle éventuellement substitué, l'alcényle, l'alcynyle, le cycloalkyle, l'hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;

b)

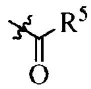

dans lequel $R^5$ représente

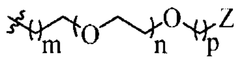
;

dans lequel m et p sont indépendamment choisis parmi de 0 à 5, n désigne le degré de polymérisation et est choisi parmi de 1 à 250 et Z représente un alkyle ou un cycloalkyle éventuellement substitué ; dans lequel Z représente un alkyle ou un cycloalkyle éventuellement substitué qui est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;

c)

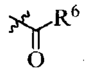

dans lequel $R^6$ est choisi parmi $NH_2$, $NHR^7$ et $NR^8R^9$ ; dans lequel $R^7$ est choisi parmi une alkyle éventuellement substitué, un alcényle, un alcynyle, un cycloalkyle, un hétérocycloalkyle, un aryle et un hétéroaryle ; $R^8$ et $R^9$ forment, conjointement avec l'azote auquel ils sont liés, un noyau hétérocyclique saturé, partiellement insaturé ou insaturé à trois à sept chaînons et peuvent éventuellement être substitués à une position substituable par un ou plusieurs radicaux $R^{10}$, dans lequel les radicaux $R^{10}$ sont indépendamment choisis à chaque occurrence dans le groupe constitué par un alkyle, un alkylcarbonyle, un formyle, un halogéno, un halogénoalkyle, un alkylphosphate, un phosphate, un oxo, un cyano, un nitro, un amino, un alcoxy, un alcoxycarbonyle, un carboxyalkyle, un hydroxyalkyle, un alcényle, un alcynyle, un alkylthio, un cycloalkyle, un aryle, un aralkyle, un hétérocycloalkyle, un alkylthioalkyle, un arylcarbonyle, un aralkylcarbonyle et un alcoxyalkyle ; dans lequel $R^7$ qui est un alkyle éventuellement substitué, un alcényle, un alcynyle, un cycloalkyle, un hétérocycloalkyle, un aryle et un hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;

d)

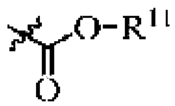

dans lequel $R^{11}$ est choisi dans le groupe constitué par un alkyle éventuellement substitué, un alcényle, un alcynyle, un cycloalkyle, un hétérocycloalkyle, un aryle et un hétéroaryle ; dans lequel $R^{11}$ qui est un alkyle éventuellement substitué, un alcényle, un alcynyle, un cycloalkyle, un hétérocycloalkyle, un aryle et un hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;

e)

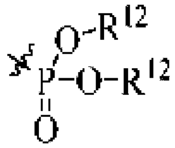

e) dans lequel chaque $R^{12}$ est indépendamment choisi dans le groupe constitué par un hydrogène ; un alkyle éventuellement substitué, un aryle, un acyle et un hétéroaryle ; dans lequel $R^{12}$ qui est un alkyle éventuellement substitué, un aryle, un acyle et un hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;
à condition que lorsque A représente un hydrogène et que l'une des substitutions de $R^{12}$ est choisie parmi un alkyle éventuellement substitué et un aryle, alors l'autre substitution de $R^{12}$ est un hydrogène ;
f)

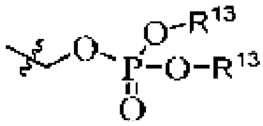

dans lequel chaque $R^{13}$ est indépendamment choisi dans le groupe constitué par un hydrogène ; un alkyle éventuellement substitué, un cycloalkyle, un hétérocycloalkyle, un aryle et un hétéroaryle ; dans lequel $R^{13}$ qui est un alkyle éventuellement substitué, un cycloalkyle, un hétérocycloalkyle, un aryle et un hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;
g)

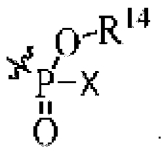

dans lequel $R^{14}$ est choisi dans le groupe constitué par un hydrogène ; un alkyle éventuellement substitué, un aryle, un acyle, un hétéroaryle ; et X représente un hétérocycloalkyle éventuellement substitué ; dans lequel $R^{14}$ qui est un alkyle éventuellement substitué, un aryle, un acyle, un hétéroaryle et X représente un hétérocycloalkyle éventuellement substitué est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ; et
h) un hydrogène, à condition que lorsque R représente un hydrogène, A ne représente pas un hydrogène.

**15.** Composé de formule II

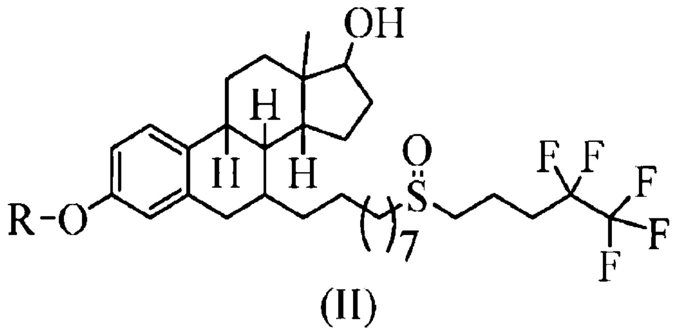

(II)

énantiomères, diastéréoisomères, racémates, sels pharmaceutiquement acceptables ou solvates de celui-ci, destinés à être utilisés dans le traitement de maladies bénines ou malines du sein ou de l'appareil génital, dans lequel R est choisi dans le groupe constitué par :

a)

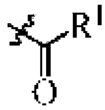

dans lequel $R^1$ est choisi dans le groupe constitué par un aryle substitué ; un alkyle éventuellement substitué, un alcényle, un alcynyle, un cycloalkyle, un hétéroaryle et $CR^2R^3$, dans lequel $R^2$ et $R^3$ forment, conjointement avec le carbone auquel ils sont liés, un noyau hétérocyclique saturé, partiellement insaturé ou insaturé à trois à sept chaînons dans lequel jusqu'à 4 atomes de carbone sont remplacés par des hétéroatomes choisis dans le groupe constitué par O, S ou N et peuvent éventuellement être substitués à une position substituable par un ou plusieurs radicaux $R^4$, dans lequel les radicaux $R^4$ sont indépendamment choisis à chaque occurrence dans le groupe constitué par un alkyle, un cycloalkyle, un hétérocycloalkyle, un aryle, un hétéroaryle, un alkylcarbonyle, un formyle, un halogéno, un alkylphosphate, un phosphate, un cyano, un nitro, un alcoxy, un alcoxycarbonyle, un alcényle, un alcynyle, un alkylthio et un arylcarbonyle ; dans lequel l'aryle substitué par $R^1$, l'alkyle éventuellement substitué, l'alcényle, l'alcynyle, le cycloalkyle, l'hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;

b)

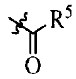

dans lequel $R^5$ représente

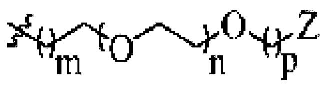

,

dans lequel m et p sont indépendamment choisis parmi de 0 à 5, n désigne le degré de polymérisation et est choisi parmi de 1 à 250 et Z représente un alkyle ou un cycloalkyle éventuellement substitué ; dans lequel Z représente un alkyle ou un cycloalkyle éventuellement substitué qui est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un

sulfinyle, un sulfonyle, un alcoxy, un sulfanyle, un halogène, un carboxy, un trihalogénométhyle, un cyano, un hydroxy, un mercapto, un nitro ;

c)

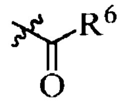

dans lequel $R^6$ est choisi parmi $NH_2$, $NHR^7$ et $NR^8R^9$ ; dans lequel $R^7$ est choisi parmi une alkyle éventuellement substitué, un alcényle, un alcynyle, un cycloalkyle, un hétérocycloalkyle, un aryle et un hétéroaryle ; $R^8$ et $R^9$ forment, conjointement avec l'azote auquel ils sont liés, un noyau hétérocyclique saturé, partiellement insaturé ou insaturé à trois à sept chaînons et peuvent éventuellement être substitués à une position substituable par un ou plusieurs radicaux $R^{10}$, dans lequel les radicaux $R^{10}$ sont indépendamment choisis à chaque occurrence dans le groupe constitué par un alkyle, un alkylcarbonyle, un formyle, un halogéno, un halogénoalkyle, un alkylphosphate, un phosphate, un oxo, un cyano, un nitro, un amino, un alcoxy, un alcoxycarbonyle, un carboxyalkyle, un hydroxyalkyle, un alcényle, un alcynyle, un alkylthio, un cycloalkyle, un aryle, un aralkyle, un hétérocycloalkyle, un alkylthioalkyle, un arylcarbonyle, un aralkylcarbonyle et un alcoxyalkyle ; dans lequel $R^7$ qui est un alkyle éventuellement substitué, un alcényle, un alcynyle, un cycloalkyle, un hétérocycloalkyle, un aryle et un hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;

d)

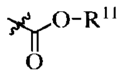

dans lequel $R^{11}$ est choisi dans le groupe constitué par un alkyle éventuellement substitué, un alcényle, un alcynyle, un cycloalkyle, un hétérocycloalkyle, un aryle et un hétéroaryle ; dans lequel $R^{11}$ qui est un alkyle éventuellement substitué, un alcényle, un alcynyle, un cycloalkyle, un hétérocycloalkyle, un aryle et un hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;

e)

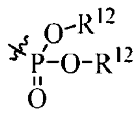

dans lequel chaque $R^{12}$ est indépendamment choisi dans le groupe constitué par un hydrogène ; un alkyle éventuellement substitué, un aryle, un acyle et un hétéroaryle ; dans lequel $R^{12}$ qui est un alkyle éventuellement substitué, un aryle, un acyle et un hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ;

à condition que lorsque l'une des $R^{12}$ est choisie parmi un alkyle éventuellement substitué et un aryle, alors l'autre substitution de $R^{12}$ est un hydrogène ;

f)

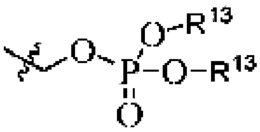

dans lequel chaque $R^{13}$ est indépendamment choisi dans le groupe constitué par un hydrogène ; un alkyle éventuellement substitué, un cycloalkyle, un hétérocycloalkyle, un aryle et un hétéroaryle ; dans lequel $R^{13}$ qui est un alkyle éventuellement substitué, un cycloalkyle, un hétérocycloalkyle, un aryle et un hétéroaryle est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro ; et

g)

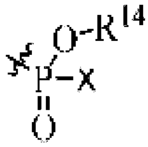

dans lequel $R^{14}$ est choisi dans le groupe constitué par un hydrogène ; un alkyle éventuellement substitué, un aryle, un acyle, un hétéroaryle ; et X représente un hétérocycloalkyle éventuellement substitué, dans lequel $R^{14}$ qui est un alkyle éventuellement substitué, un aryle, un acyle, un hétéroaryle et X représente un hétérocycloalkyle éventuellement substitué est substitué à une position substituable par un ou plusieurs radicaux qui sont indépendamment choisis à chaque occurrence dans le groupe constitué par un alkyle en C1-C8, un alcényle en C2-C8, un alcynyle en C2-C8, un cycloalkyle, un hétérocycloalkyle, un aryle, un hétéroaryle, un amino, un alkylamino, un acyle, un acyloxy, un acylamino, un aminocarbonyle, un alcoxycarbonyle, un alcoxyalkyloxy, un alcoxycarbonyloxy, un carbamate, un sulfinyle, un sulfonyle, un alcoxy, un sulfanyle, un halogène, un carboxy, un trihalogénométhyle, un cyano, un hydroxy, un mercapto, un nitro.

**16.** Composition destinée à être utilisée dans le traitement de maladies bénines ou malines du sein ou de l'appareil génital comprenant au moins l'un parmi le composé de formule I-A selon la revendication 14 ; et des énantiomères, des diastéréoisomères, des racémates, des sels pharmaceutiquement acceptables ou des solvates de celui-ci.

**17.** Composition destinée à être utilisée dans le traitement de maladies bénines ou malines du sein ou de l'appareil génital comprenant au moins l'un parmi le composé de formule II selon la revendication 15 ; et des énantiomères, des diastéréoisomères, des racémates, des sels pharmaceutiquement acceptables ou des solvates de celui-ci.

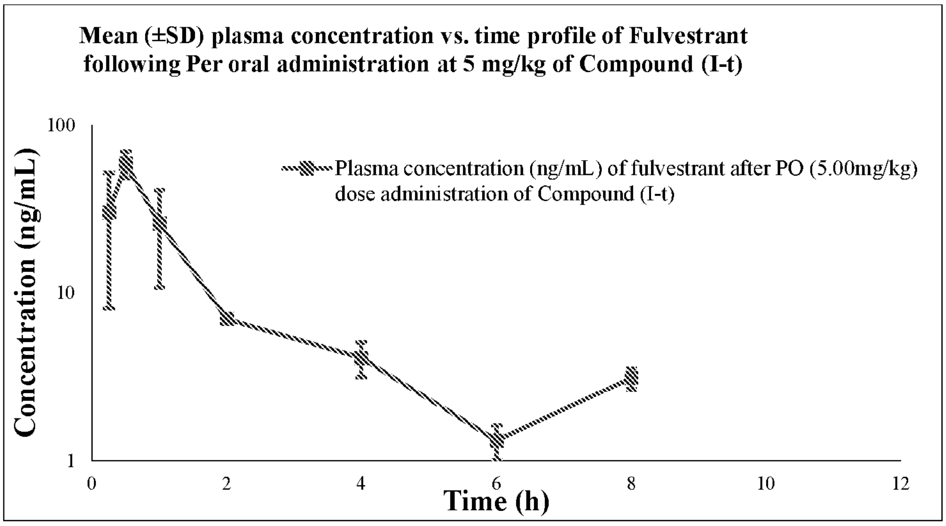


**Figure 1.** Time-concentration profile in mice (compound (I-t) and fulvestrant blood levels)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6774122 B2
- WO 2016004166 A1
- CN 103421069 A


### Non-patent literature cited in the description


- *J. Med. Chem.*, 2016, vol. 59 (17), 8134-8140